# EUROPEAN PATENT SPECIFICATION

(11) **EP 3 809 838 B1**
(45) Date of publication and mention of the grant of the patent: **01.04.2026**
(21) Application number: 19821744.0
(22) Date of filing: 21.06.2019
(51) Int. Cl.: A01K 67/0275, C07K 14/47, C07K 14/705, C07K 14/775, C12N 9/06, C12N 9/16

(54) **GENETICALLY MODIFIED MOUSE MODELS OF ALZHEIMER'S DISEASE**
GENETISCH MODIFIZIERTE MAUSMODELLE DER ALZHEIMER-KRANKHEIT
MODÈLES DE SOURIS GÉNÉTIQUEMENT MODIFIÉE DE LA MALADIE D'ALZHEIMER

(30) Priority: 21.06.2018 US 201862687952 P; 18.07.2018 US 201862700004 P
(43) Date of publication of application: 28.04.2021
(73) Proprietor: The Jackson Laboratory, Bar Harbor, ME 04609 (US); Indiana University Research & Technology Corporation, Indianapolis, IN 46202 (US); Sage Bionetworks, Seattle WA 98121 (US)
(72) Inventor: HOWELL, Gareth, Bar Harbor, ME 04609 (US); CARTER, Gregory, Bar Harbor, ME 04609 (US); SASNER, Michael, Bar Harbor, ME 04609 (US); RIZZO, Stacey, Bar Harbor, ME 04609 (US); WILLIAMS, Harriet, Bar Harbor, ME 04609 (US); LAMB, Bruce, Carmel, IN 46032 (US); TERRITO, Paul, Fishers, IN 46037 (US); OBLAK, Adrian, Fishers, IN 46037 (US); LOGSDON, Ben, Seattle, WA 98121 (US); MANGRAVITE, Lara, Seattle, WA 98121 (US); GRAHAM, Leah, Bar Harbor, Maine 04609 (US)
(74) Representative: Potter Clarkson
(86) International application number: PCT/US2019/038401
(87) International publication number: WO 2019/246483

(56) References cited:
- US-A1- 2006 160 079
- US-A1- 2006 272 038
- PAUL J. CHENG-HATHAWAY ET AL: "The Trem2 R47H variant confers loss-of-function-like phenotypes in Alzheimer's disease", MOLECULAR NEURODEGENERATION, vol. 13, no. 29, 1 June 2018 (2018-06-01), pages 1 - 12, XP055664658, DOI: 10.1186/s13024-018-0262-8
- MURRAY C. E. ET AL: "APOE [epsilon] 4 is also required in TREM2 R47H variant carriers for Alzheimer's disease to develop", vol. 45, no. 2, 7 February 2018 (2018-02-07), GB, pages 183 - 186, XP055890399, ISSN: 0305-1846, Retrieved from the Internet <URL:https://api.wiley.com/onlinelibrary/tdm/v1/articles/10.1111%2Fnan.12474> DOI: 10.1111/nan.12474
- CHAN ELIZABETH S. ET AL: "ApoE4 expression accelerates hippocampus-dependent cognitive deficits by enhancing A[beta] impairment of insulin signaling in an Alzheimer's disease mouse model", vol. 6, no. 1, September 2016 (2016-09-01), XP055890337, Retrieved from the Internet <URL:https://www.nature.com/articles/srep26119.pdf> DOI: 10.1038/srep26119
- ONOS KRISTEN D ET AL: "Toward more predictive genetic mouse models of Alzheimer's disease", BRAIN RESEARCH BULLETIN, ELSEVIER SCIENCE LTD, OXFORD, GB, vol. 122, 17 December 2015 (2015-12-17), pages 1 - 11, XP029483616, ISSN: 0361-9230, DOI: 10.1016/J.BRAINRESBULL.2015.12.003
- SHAO WEN ET AL: "Genetics of Alzheimer's disease: From pathogenesis to clinical usage", JOURNAL OF CLINICAL NEUROSCIENCE, vol. 45, 2017, pages 1 - 8, XP085232882, ISSN: 0967-5868, DOI: 10.1016/J.JOCN.2017.06.074
- HOLTZMAN ET AL.: "Apolipoprotein E isoform-dependent amyloid deposition and neuritic degeneration in a mouse model of Alzheimer's disease", PNAS, vol. 97, no. 6, 14 March 2000 (2000-03-14), pages 2892 - 2897, XP055664650, DOI: 10.1073/pnas.050004797
- CASTILLO ET AL.: "Comparative profiling of cortical gene expression in Alzheimer's disease patients and mouse models demonstrates a link between amyloidosis and neuroinflammation", SCIENTIFIC REPORTS, vol. 7, no. 17762, 19 December 2017 (2017-12-19), pages 1 - 16, XP055664654, DOI: 10.1038/s41598-017-17999-3
- CHENG-HATHAWAY ET AL.: "The Trem2 R47H variant confers loss-of-function-like phenotypes in Alzheimer's disease", MOL NEURODEGENER, vol. 13, no. 29, 1 June 2018 (2018-06-01), pages 1 - 12, XP055664658, DOI: 10.1186/s13024-018-0262-8
- ATAGI ET AL.: "Apolipoprotein E Is a Ligand for Triggering Receptor Expressed on Myeloid Cells 2 (TREM2)", J BIOL CHEM., vol. 290, no. 43, 23 October 2015 (2015-10-23), pages 26043 - 26050, XP055664659, ISSN: 0021-9258, DOI: 10.1074/jbc.M115.679043

## Description

### RELATED APPLICATIONS

This application claims the benefit under 35 U.S.C. § 119(e) of U.S. provisional application number 62/687,952, filed June 21, 2018, and U.S. provisional application number 62/700,004, filed July 18, 2018.

### GOVERNMENT SUPPORT

This invention was made with government support under AG054345 awarded by the National Institutes of Health. The government has certain rights in the invention.

### BACKGROUND

One of the major obstacles to developing therapies for Alzheimer's disease (AD) is the lack of animal models for use in preclinical trials. One reason for this may be that existing models are based on familial mutations, while the vast majority of the clinical population has non-familial late-onset AD.

Familial or early-onset Alzheimer's disease is caused by mutations in, or overexpression of, the amyloid precursor protein (*APP*) gene or mutations in presenilin genes (*PSEN1* or *PSEN2*). All of these lead to increased production of the ABETA42 peptide, which is thought to be neurotoxic. Dozens if not hundreds of mouse models that mimic aspects of familial Alzheimer's disease have been created. Many treatments have been shown to be effective in these familial Alzheimer's disease mouse models, but none have been effective when tested in clinical trials.

In contrast, late-onset Alzheimer's disease, which accounts for 95-98% of the human Alzheimer's disease patient population, does not have a simple and defined genetic etiology. Late-onset Alzheimer's disease is thought to be a multifactorial syndrome caused by a variety of genetic and environmental causes interacting with the aging process. There is a continuing need for mouse models of late-onset Alzheimer's disease in humans.

### SUMMARY

The invention is as defined in the claims. The present invention provides a genetically modified mouse model of late-onset Alzheimer's disease comprising a genomic nucleic acid encoding human APOE4, a genomic nucleic acid encoding mouse TREM2 modified to include a R47H substitution, and a genomic nucleic acid encoding mouse APP modified to include G601R, F606Y, and R609H substitutions (humanized APP).

In some embodiments, the genomic nucleic acid encoding human APOE4 comprises a nucleotide sequence having at least 95% identity to the nucleotide sequence of SEQ ID NO:2, and/or the human APOE4 comprises an amino acid sequence having at least 95% identity to the amino acid sequence of SEQ ID NO: 1. In some embodiments, the genomic nucleic acid encoding human APOE4 comprises the nucleotide sequence of SEQ ID NO:2, and/or the human APOE4 comprises the amino acid sequence of SEQ ID NO: 1. In some embodiments, the genetically modified mouse expresses the human APOE4 and/or does not express a detectable level of mouse APOE4.

In some embodiments, the genomic nucleic acid encoding mouse TREM2 modified to include a R47H substitution comprises a nucleotide sequence having at least 95% identity to the nucleotide sequence of SEQ ID NO:4, and/or the mouse TREM2 modified to include a R47H substitution comprises an amino acid sequence having at least 95% identity to the amino acid sequence of SEQ ID NO:3. In some embodiments, the genomic nucleic acid encoding mouse TREM2 modified to include a R47H substitution comprises the nucleotide sequence of SEQ ID NO:4, and/or the mouse TREM2 modified to include a R47H substitution comprises the amino acid sequence of SEQ ID NO:3. In some embodiments, the genetically modified mouse expresses the mouse TREM2 modified to include a R47H substitution.

In some embodiments, the genomic nucleic acid encoding mouse APP modified to include G601R, F606Y, and R609H substitutions comprises a nucleotide sequence that shares at least 95% identity with the nucleotide sequence of SEQ ID NO:23, and/or the mouse APP modified to include G601R, F606Y, and R609H substitutions comprises an amino acid sequence that shares at least 95% identity with the amino acid sequence of SEQ ID NO:24. In some embodiments, the genomic nucleic acid encoding mouse APP modified to include G601R, F606Y, and R609H substitutions comprises the nucleotide sequence of SEQ ID NO:23, and/or the mouse APP modified to include G601R, F606Y, and R609H substitutions comprises the amino acid sequence of SEQ ID NO:24. In some embodiments, the genetically modified mouse expresses the mouse APP modified to include G601R, F606Y, and R609H substitutions.

In some embodiments, total cholesterol level, low density lipoprotein (LDL) level, and/or high density lipoprotein (HDL) level is reduced in the genetically modified mouse, relative to a control mouse, optionally wherein the control mouse is a wild-type C57BL/6J mouse.

In some embodiments, the total cholesterol level in the genetically modified mouse is reduced by at least 10% or at least 20%, relative to the control mouse.

In some embodiments, the LDL level in the genetically modified mouse is reduced by at least 30%, at least 40%, or at least 50%, relative to the control mouse.

In some embodiments, the HDL level in the genetically modified mouse is reduced by at least 10%, relative to the control mouse.

In some embodiments, the genetically modified mouse exhibits signs of cerebrovascular leakage.

In some embodiments, at least one of the following genes is differentially expressed in the genetically modified mouse: *Pcsk2, Mapk10, Mapk9, Prkcq, Slc18a2, Plcb2, Slc6a4, Gng2, Akt3, Gnao1, Plcb3, Arrb2, Il6, Myh10, Cnrl, Stx1a, Unc13a, Cdk5, Calbl, Gria4, Thy1, Hcn1, Chl1, Uchll, Amph, Chmp2b,* and *Casp7.*

Also provided herein, in some aspects, is a method of producing a genetically modified mouse model of late-onset Alzheimer's disease, the method comprising (i) providing a genetically modified mouse comprising a genomic nucleic acid encoding human APOE4 and a genomic nucleic acid encoding mouse TREM2 modified to include a R47H substitution; and (ii) introducing into the genetically modified mouse of (i) a genomic nucleic acid encoding mouse APP modified to include G601R, F606Y, and R609H substitutions, thereby producing a genetically modified mouse.

Further provided herein, in some aspects, is a method of producing a genetically modified mouse model of late-onset Alzheimer's disease, the method comprising introducing into a mouse a genomic nucleic acid encoding human APOE4, a genomic nucleic acid encoding mouse TREM2 modified to include a R47H substitution, and a genomic nucleic acid encoding mouse APP modified to include G601R, F606Y, and R609H substitutions, thereby producing a genetically modified mouse.

Other aspects of the invention include a method of screening for a compound for use in the treatment of Alzheimer's disease, comprising administering a compound to a genetically modified mouse of the present invention, and assessing the mouse for an effect of the compound on a symptom associated with Alzheimer's disease.

### BRIEF DESCRIPTION OF THE DRAWINGS

Figure 1 is a schematic diagram of a humanized *APOE4* expression construct described in detail in examples herein.
Figure 2 is an image of a Western blot of brain tissue from B6(SJL)-*Apoe^{tm1.1(APOE*4)Adiuj} Trem2^{em1Adiuj}*/J (common name: B6J.APOE4/Trem2) mice (lanes 4-6) and control (wild-type (WT) C57BL/6J (abbreviated B6J) mice (lanes 1-3) brain tissue.
Figure 3A is a graph showing results of an assay for high density lipoprotein (HDL) in blood samples obtained from 12-month-old B6J.APOE4/Trem2 mice and 12-month-old C57BL/6J control mice.
Figure 3B is a graph showing results of an assay for low density lipoprotein (LDL) in blood samples obtained from 12-month-old B6J.APOE4/Trem2 mice and 12-month-old C57BL/6J control mice.
Figure 3C is a graph showing results of an assay for total cholesterol in blood samples obtained from 12-month-old B6J.APOE4/Trem2 mice and 12-month-old C57BL/6J control mice.
Figure 4 shows representative images of B6J.APOE4/Trem2 tissue at 7-8 months of age immunostained to show Collagen IV (Col IV) and Fibrin(ogen) along with representative images from similar sagittal sections of brain from control C57BL/6J (abbreviated B6J) mice which were similarly immunostained.
Figure 5 is a gene expression heatmap of differentially-expressed genes in B6J.APOE4/Trem2 mice compared to wild-type control B6J mice.

### DETAILED DESCRIPTION

The present disclosure provides, in some aspects, mutant mouse strains that carry a humanized *ApoE* knock-in mutation (sequence coding for isoform E4), a CRISPR/cas9-generated R47H point mutation of the *Trem2* gene, and at least one additional genomic modification that renders the mice suitable, for example, for use in studies related to Alzheimer's disease. In some embodiments, the mutant mouse strains are also suitable for use in studies related to lipoproteins, arteriosclerosis, and/or coronary heart disease.

In some embodiments, the present disclosure relates generally to a genetically modified mouse that is a model of non-familial late-onset Alzheimer's disease. In the present invention, the genetically modified mouse encodes at least three exogenously introduced risk factors for non-familial late-onset AD in its genome, such that the genetically modified mouse expresses human APOE4, mouse TREM2 p.R47H, and a humanized mutant of mouse amyloid precursor protein (APP) including G601R, F606Y, and R609H substitutions. Also disclosed herein but not forming part of the claimed invention is a genetically modified mouse expressing human APOE4, mouse TREM2 p.R47H, and one or more of (at least one of) the following genomic modifications: (1) a humanized mutant of mouse ATP-binding cassette, sub-family A, member 7 (ABCA7); (2) a humanized mutant of mouse phospholipase C γ2 gene (PLCG2); (3) a mutant of mouse methylenetetrahydrofolate reductase (MTHFR); (4) a knockout (KO) of mouse carcinoembryonic antigen-related cell adhesion molecule 1 (CEACAM1) (e.g., an inactivated *Ceacam1* allele); and (5) a KO of mouse interleukin 1 receptor accessory protein (IL1RAP) (e.g., an inactivated *IL1RAP* allele).

Herein, the human APOE protein and the mouse APOE protein may be abbreviated as APOE p (protein), and the human *APOE* gene and the mouse *Apoe* gene may be abbreviated as *APOE* g (gene) and *Apoe* g, respectively; the mouse TREM2 protein and the mouse *Trem2* gene may be abbreviated as TREM2 p and *Trem2* g, respectively; the human ABCA7 protein and the mouse ABCA7 protein may be abbreviated as ABCA7 p (protein), and the human *ABCA7* gene and the mouse *Abca7* gene may be abbreviated as *ABCA7* g and *Abca7* g, respectively; the human APP protein and the mouse APP protein may be abbreviated as APP p, and the human *APP* gene and the mouse *App* gene may be abbreviated as *APP* g and *App* g, respectively; the human PLCG2 protein and the mouse PLCG2 protein may be abbreviated as PLCG2 p, and the human *PLCG2* gene and the mouse *Plcg2* gene may be abbreviated as *PLCG2* g and *Plcg2* g, respectively; and the human MTHFR protein and the mouse MTHFR protein may be abbreviated as MTHFR p, and the human *MTHFR* gene and the mouse *Mthfr* gene may be abbreviated as *MTHFR* g and *Mthfr* g, respectively.

Herein, mouse TREM2 modified to include a R47H substitution may be abbreviated as TREM2*R47H p; ABCA7 modified to include an A1541G substitution may be abbreviated as ABCA7*A1541G p; APP modified to include G601R, F606Y, and R609H substitutions may be abbreviated as APP* p; PLCG2 modified to include a M28L substitution may be abbreviated as PLCG2*M28L p; and MTHFR modified to include a A262V substitution may be abbreviated as MTHFR*A262V p.

Human apolipoprotein is a polymorphic protein with three isoforms designated APOE2, APOE3 and APOE4. These three isoforms differ from each other with respect to the identity of amino acids at positions 130 and 176 in the amino acid sequence of the proteins (corresponding to positions 112 and 158 in the mature APOE protein without the 18 amino acid signal peptide). APOE2 is characterized by cysteine at both 130 and 176, APOE3 is characterized by cysteine at 130 and arginine at 176 and APOE4 is characterized by arginine at both 130 and 176. Individuals having one or more *APOE4* alleles are at greater risk for developing non-familial late-onset AD and a number of mechanisms relating to pathology have been proposed, see for example, DiBattista et al., 2016, Exp. Neurol. 280:97-105; Bu et al., Nature Reviews Neuroscience, 2009, 10:333-344; Huang et al., Cell, 2017, 168:1-15; and Tambini et al., EMBO Reports, 2016, 17:27-36.

TREM2 (triggering receptor expressed on myeloid cells 2) is an immune phagocytic receptor expressed by brain microglia. TREM2 triggers phagocytosis of cell debris and regulates aspects of the inflammatory response. A rare variant in *TREM2,* p.R47H, is significantly associated with Alzheimer's disease in humans.

The targeted apolipoprotein E gene is important in lipoprotein metabolism and cardiovascular disease as well as Alzheimer's disease, immunoregulation and cognition. The targeted Trem2 gene encodes a protein that is part of a receptor signaling complex with TYRO protein tyrosine kinase binding protein, and that activates macrophages and dendritic cells during immune responses. The TREM2 R47H mutation is a missense mutation in exon 2 that is one of the strongest genetic risk factors for late-onset Alzheimer's disease.

### APOE4/Trem2*R47H

In some embodiments, the genetically modified mice provided herein are derived from the B6(SJL)-*Apoe^{tm1.1(APOE*4)Adiuj} Trem2^{em1Adiuj}*/J mouse strain (also known as APOE4/Trem2*R47H or APOE*4/Trem2*R47H). The B6(SJL)-*Apoe^{tm1.1(APOE*4)Adiuj} Trem2^{em1Adiuj}*/J mouse strain is a double mutant strain that carries a humanized ApoE knock-in allele, in which exons 2, 3 and most of exon 4 of the mouse *Apoe* gene were replaced by human *APOE4* gene sequence including exons 2, 3 and 4 (and some 3' UTR sequence), and a knock-in point mutation in the mouse *Trem2* gene that includes an R47H point mutation, along with two other silent mutations. Mice that are homozygous for both alleles are viable and fertile.

This double mutant line was generated by crossing two single mutant lines.

For the *Apoe^{tm1.1(APOE*4)Adiuj}* allele: A targeting vector containing a 3' FRT flanked neo cassette was used to replace exons 2, 3 and most of exon 4 with 1.5 kb of human *APOE* gene sequence including exons 2, 3 and 4 (and some 3' UTR sequence). The human ApoE sequence also contains a two nucleotide A to T substitution in intron 3 for identification of the targeted allele by Southern analysis. The construct was electroporated into C57BL/6J derived embryonic stem (ES) cells. Correctly targeted ES cells were injected into blastocysts. The resulting chimeric animals were tested for germline transmission by crossing to C57BL/6J. Heterozygous animals were crossed subsequently to FLP recombinase expressing mice to remove the FRT site flanked PGK-neo cassette. Mice that no longer contained the FRT flanked PGK-neo cassette were then backcrossed to C57BL/6J at least once to remove the FLP recombinase transgene.

For the *Trem2^{em1Adiuj}* allele: Plasmids encoding a single guide RNA designed to introduce a R47H point mutation, with two silent mutations, into the *Trem2* gene and the *cas9* nuclease were introduced into the cytoplasm C57BL/6J-derived fertilized eggs with well recognized pronuclei. Correctly targeted embryos were transferred to pseudopregnant females. Correctly targeted pups were identified by sequencing and PCR and further bred to C57BL/6J to develop the colony.

### Abca7*A1527G/APOE4/Trem2*R47H

In some embodiments, the genetically modified mice provided herein are derived from the B6.Cg*-Apoe^{tm1.1(APOE*4)Adiuj} Abca7^{em2Adiuj} Trem2^{em1Adiuj}*/J mouse strain (also known as Abca7*A1527G/APOE4/Trem2*R47H). The B6.Cg*-Apoe^{tm1.1(APOE*4)Adiuj} Abca7^{em2Adiuj} Trem2^{em1Adiuj}*/J mouse strain is a triple mutant strain that carries a humanized ApoE knock-in allele, in which exons 2, 3 and most of exon 4 of the mouse *Apoe* gene were replaced by human *APOE4* gene sequence including exons 2, 3 and 4 (and some 3' UTR sequence); a rs3752246 SNP knock-in mutation into the *Abca7* gene (alanine to glycine at position 1541 in exon 33); and a knock-in of a point mutation into mouse the *Trem2* gene containing a R47H point mutation, with two silent mutations. This *Abca7* point mutation models the human ABCA7 A1527G polymorphism.

This triple mutant line was generated by utilizing CRISPR/cas9 endonuclease mediated genome editing of the *Abca7* gene in B6(SJL)-*Apoe^{tm1.1(APOE*4)Adiuj} Trem2^{em1Adiuj}*/J mice.

The double mutant B6(SJL)-*Apoe^{tm1.1(APOE*4)Adiuj} Trem2^{em1Adiuj}*/J was generated by crossing two single mutant lines, as described above.

For the *Abca7^{em2Adiuj}* allele: plasmids encoding a single guide RNA designed to introduce rs3752246 SNP knock-in mutation into the *Abca7* gene (alanine to glycine at position 1527 in exon 33) and the *cas9* nuclease were introduced into the cytoplasm of B6(SJL)-*Apoe^{tm1.1(APOE*4)Adiuj} Trem2^{em1Adiuj}*/J-derived fertilized eggs with well recognized pronuclei. Correctly targeted embryos were transferred to pseudopregnant females. Correctly targeted pups were identified by sequencing and PCR and further bred to B6(SJL)-*Apoe^{tm1.1(APOE*4)Adiuj} Trem2^{em1Adiuj}*/J to develop the colony.

### Abca7 KO/APOE4/Trem2*R47H

In some embodiments, the genetically modified mice provided herein are derived from the B6.Cg*-Apoe^{tm1.1(APOE*4)Adiuj} Abca7^{em1Adiuj} Trem2^{em1Adiuj}*/J mouse strain (also known as Abca7 KO/APOE4/Trem2*R47H). The B6.Cg*-Apoe^{tm1.1(APOE*4)Adiuj} Abca7^{em1Adiuj} Trem2^{em1Adiuj}*/J mouse strain is a triple mutant strain that carries a humanized ApoE knock-in allele, in which exons 2, 3 and most of exon 4 of the mouse *Apoe* gene were replaced by human *APOE4* gene sequence including exons 2, 3 and 4 (and some 3' UTR sequence); a mutant knock-out (33bp deletion exon 32) allele of the *Abca7* gene; and a knock-in of a point mutation into mouse the *Trem2* gene containing a R47H point mutation, with two silent mutations.

This triple mutant line was generated by utilizing CRISPR/cas9 endonuclease mediated genome editing of the *Abca7* gene in B6(SJL)-*Apoe^{tm1.1(APOE*4)Adiuj} Trem2^{em1Adiuj}*/J mice.

The double mutant B6(SJL)-*Apoe^{tm1.1(APOE*4)Adiuj} Trem2^{emAdiuj}*/J was generated by crossing the single mutant lines, as described above.

For the *Abca7^{em1Adiuj}* allele: plasmids encoding a single guide RNA designed to introduce knock-out (33bp deletion exon 32) mutation into the *Abca7* gene and the *cas9* nuclease were introduced into the cytoplasm of B6(SJL)-*Apoe^{tm1.1(APOE*4)Adiuj} Trem2^{em1Adiuj}*/J-derived fertilized eggs with well recognized pronuclei. Correctly targeted embryos were transferred to pseudopregnant females. Correctly targeted pups were identified by sequencing and PCR and further bred to B6(SJL)-*Apoe^{tm1.1(APOE*4)Adiuj} Trem2^{em1Adiuj}*/J to develop the colony.

### hAPP/APOE4/Trem2*R47H

In some embodiments, the genetically modified mice provided herein are derived from the B6.Cg*-Apoe^{tm1.1(APOE*4)Adiuj} App^{em1Adiuj} Trem2^{em1Adiuj}*/J mouse strain (also known as hAPP/APOE4/Trem2*R47H). The B6.Cg*-Apoe^{tm1.1(APOE*4)Adiuj} App^{em1Adiuj} Trem2^{em1Adiuj}*/J mouse strain that carries a humanized ApoE knock-in allele in which exons 2, 3 and most of exon 4 of the mouse *Apoe* gene were replaced by human *APOE4* gene sequence including exons 2, 3 and 4 (and some 3' UTR sequence), a mutant allele of the *App* gene containing G601R, F606Y, and R609H point mutations, and a knock-in of a point mutation into mouse *Trem2* gene containing an R47H point mutation with two silent mutations.

This triple mutant line was generated by utilizing CRISPR/cas9 endonuclease-mediated genome editing of the *App* gene in B6(SJL)-*Apoe^{tm1.1(APOE*4)Adiuj} Trem2^{em1Adiuj}*/J mice.

The double mutant B6(SJL)-*Apoe^{tm1.1(APOE*4)Adiuj} Trem2^{em1Adiuj}*/J was generated by crossing two single mutant lines, as described above.

For the *App^{em1Adiuj}* allele: plasmids encoding a single guide RNA designed to introduce the G601R, F606Y, and R609H point mutations into the *App* gene and the *cas9* nuclease were introduced into the cytoplasm of B6(SJL)-*Apoe^{tm1.1(APOE*4)Adiuj} Trem2^{em1Adiuj}*/J-derived fertilized eggs with well recognized pronuclei. Correctly targeted embryos were transferred to pseudopregnant females. Correctly targeted pups were identified by sequencing and PCR and further bred to B6(SJL)-*Apoe^{tm1.1(APOE*4)Adiuj} Trem2^{em1Adiuj}*/J to develop the colony.

### App KO/APOE4/Trem2*R47H

In some embodiments, the genetically modified mice provided herein are derived from the B6.Cg*-Apoe^{tm1.1(APOE*4)Adiuj} App^{em2Adiuj} Trem2^{em1Adiuj}*/J mouse strain (also known as App KO/APOE4/Trem2*R47H). The B6.Cg*-Apoe^{tm1.1(APOE*4)Adiuj} App^{em2Adiuj} Trem2^{em1Adiuj}*/J mouse strain is a triple mutant strain that carries a humanized ApoE knock-in allele, in which exons 2, 3 and most of exon 4 of the mouse *Apoe* gene were replaced by human *APOE4* gene sequence including exons 2, 3 and 4 (and some 3' UTR sequence); a mutant allele of the *App* gene containing a 94bp deletion in exon 14; and a knock-in of a point mutation into the mouse *Trem2* gene containing a R47H point mutation, with two silent mutations.

This triple mutant line was generated by utilizing CRISPR/cas9 endonuclease mediated genome editing of the *App* gene in B6(SJL)-Apoe*^{tm1.1(APOE*4)Adiuj} Trem2^{em1Adiuj}*/J mice.

The double mutant B6(SJL)-*Apoe^{tm1.1(APOE*4)Adiuj} Trem2^{em1Adiuj}*/J was generated by crossing two single mutant lines, as described above.

For the *App^{em2Adiuj}* allele: plasmids encoding a single guide RNA designed to introduce a 94bp deletion in exon 14 into the *App* gene and the *cas9* nuclease were introduced into the cytoplasm of B6(SJL)-*Apoe^{tm1.1(APOE*4)Adiuj} Trem2^{em1Adiuj}*/J-derived fertilized eggs with well recognized pronuclei. Correctly targeted embryos were transferred to pseudopregnant females. Correctly targeted pups were identified by sequencing and PCR and further bred to B6(SJL)-*Apoe^{tm1.1(APOE*4)Adiuj} Trem2^{em1Adiuj}*/J to develop the colony.

### Plcg2*M28L/APOE4/Trem2*R47H

In some embodiments, the genetically modified mice provided herein are derived from the B6.Cg*-Apoe^{tm1.1(APOE*4)Adiuj} Plcg2^{em3Adiuj} Trem2^{em1Adiuj}*/J mouse strain (also known as Plcg2*M28L/APOE4/Trem2*R47H). The B6.Cg*-Apoe^{tm1.1(APOE*4)Adiuj} Plcg2^{em3Adiuj} Trem2^{em1Adiuj}*/J mouse strain is a triple mutant strain carries a humanized ApoE knock-in allele, in which exons 2, 3 and most of exon 4 of the mouse *Apoe* gene were replaced by human *APOE4* gene sequence including exons 2, 3 and 4 (and some 3' UTR sequence); a CRISPR/Cas9 generated mutant of the *Plcg2,* phospholipase C, gamma 2, gene with the M28L mutation; and a knock-in of a point mutation into the mouse *Trem2* gene containing a R47H point mutation, with two silent mutations.

This triple mutant line was generated by was generated by utilizing CRISPR/cas9 endonuclease mediated genome editing of the *Plcg2* (phospholipase C, gamma 2) gene in B6(SJL)*-Apoe^{tm1.1(APOE*4)Adiuj} Trem2^{em1Adiuj}*/J mice.

For the *Plcg2^{em2Adiuj}* allele: plasmids encoding a single guide RNA designed to introduce the M28L mutation into the *Plcg2gene* and the *cas9* nuclease were introduced into the cytoplasm of B6(SJL)-*Apoe^{tm1.1(APOE*4)Adiuj} Trem2^{em1Adiuj}*/J derived fertilized eggs with well recognized pronuclei. Correctly targeted embryos were transferred to pseudopregnant females. Correctly targeted pups were identified by sequencing and PCR and further bred to B6(SJL)-*Apoe^{tm1.1(APOE*4)Adpmc} Trem2^{em1Adpmc}*/J to develop the colony.

### Mthfr*C677T/APOE4/Trem2*R47H

In some embodiments, the genetically modified mice provided herein are derived from the B6.Cg-*Mthfr^{em1Adiuj} Apoe^{tm1.1(APOE*4)Adiuj} Trem2^{em10Adiuj}*/*J* mouse strain (also known as Mthfr*C677T/APOE4/Trem2*R47H). The B6.Cg-*Mthfr^{em1Adiuj} Apoe^{tm1.1(APOE*4)Adiuj} Trem2^{em10Adiuj}*/J mouse strain is a triple mutant strain that carries a humanized ApoE knock-in allele, in which exons 2, 3 and most of exon 4 of the mouse *Apoe* gene were replaced by human *APOE4* gene sequence including exons 2, 3 and 4 (and some 3' UTR sequence); a knock-in of a point mutation into mouse *Trem2* gene containing a R47H point mutation, with two silent mutations; and a CRISPR/Cas9 generated mutant of the *Mthfr* gene that carries a point mutation resulting in the A262V amino acid substitution that models the human C677T polymorphism. This *Mthfr* point mutation models the human MTHFR C677T polymorphism, which is associated with an increased risk for Alzheimer's Disease.

This triple mutant line was generated by utilizing CRISPR/cas9 endonuclease mediated genome editing of the *Mthfr* gene in B6(SJL)-*Apoe^{tm1.1(APOE*4)Adiuj} Trem2^{em1Adiuj}*/J mice.

For the *Mthfr^{em1Adiuj}* allele: plasmids encoding a single guide RNA designed to introduce a A262V point mutation into the *Mthfr* gene and the *cas9* nuclease were introduced into the cytoplasm B6(SJL)-*Apoe^{tm1.1(APOE*4)Adiuj} Trem2^{em1Adiuj}*/J-derived fertilized eggs with well recognized pronuclei. Correctly targeted embryos were transferred to pseudopregnant females. Correctly targeted pups were identified by sequencing and PCR and further bred to B6(SJL)-Apoe*^{tm1.1(APOE*4)Adiuj}Trem2^{em1Adiuj}*/J for two generations to develop the colony. Sperm was cryopreserved. To establish the live colony, an aliquot of frozen sperm was used to fertilize oocytes from B6(SJL)-*Apoe^{tm1.1(APOE*4)Adiuj} Trem2^{em1Adiuj}*/J females.

### Ceacam1 KO/APOE4/Trem2*R47H

In some embodiments, the genetically modified mice provided herein are derived from the B6.Cg*-Apoe^{tm1.1(APOE*4)Adiuj} Ceacam1^{em1Adiuj} Trem2^{em1Adiuj}*/J mouse strain (also known as *Ceacam1* KO/APOE4/Trem2*R47H). The B6.Cg*-Apoe^{tm1.1(APOE*4)Adiuj} Ceacam1^{em1Adiuj} Trem2^{em1Adiuj}*/J mouse strain is a triple mutant strain carries a humanized ApoE knock-in allele, in which exons 2, 3 and most of exon 4 of the mouse *Apoe* gene were replaced by human *APOE4* gene sequence including exons 2, 3 and 4 (and some 3' UTR sequence); a knock-out mutant allele, deletion of exon 1, of the *Ceacam1* gene; and a knock-in of a point mutation into the mouse *Trem2* gene containing a R47H point mutation, with two silent mutations.

This triple mutant line was generated by utilizing CRISPR/cas9 endonuclease mediated genome editing of the *Ceacam1* gene in B6(SJL)-*Apoe^{tm1.1(APOE*4)Adiuj} Trem2^{em1Adiuj}*/J mice.

The double mutant B6(SJL)-*Apoe^{tm1.1(APOE*4)Adiuj} Trem2^{em1Adiuj}*/J was generated by crossing two single mutant lines, as described above.

For the *Ceacam1^{em1Adiuj}* allele: plasmids encoding a single guide RNA designed to introduce a knock-out mutation, deletion of exon 1, into the *Ceacam1* gene and the *cas9* nuclease were introduced into the cytoplasm of B6(SJL)-*Apoe^{tm1.1(APOE*4)Adiuj} Trem2^{em1Adiuj}*/J-derived fertilized eggs with well recognized pronuclei. Correctly targeted embryos were transferred to pseudopregnant females. Correctly targeted pups were identified by sequencing and PCR and further bred to B6(SJL)-*Apoe^{tm1.1(APOE*4)Adiuj} Trem2^{em1Adiuj}*/J to develop the colony.

### Il1rap KO/APOE4/Trem2*R47H, exon 2 KO

In some embodiments, the genetically modified mice provided herein are derived from the B6.Cg*-Apoe^{tm1.1(APOE*4)Adiuj} Il1rap^{em1Adiuj} Trem2^{em1Adiuj}*/J mouse strain (also known as Il1rap KO/APOE4/Trem2*R47H, exon 2 KO). The B6.Cg*-Apoe^{tm1.1(APOE*4)Adiuj} Il1rap^{em1Adiuj} Trem2^{em1Adiuj}*/J mouse strain is a triple mutant strain carries a humanized ApoE knock-in allele, in which exons 2, 3 and most of exon 4 of the mouse *Apoe* gene were replaced by human *APOE4* gene sequence including exons 2, 3 and 4 (and some 3' UTR sequence); a mutant knock-out allele, 387bp deletion in the first coding exon, of the *Illrap* gene; and a knock-in of a point mutation into mouse the *Trem2* gene containing a R47H point mutation, with two silent mutations.

This triple mutant line was generated by utilizing CRISPR/cas9 endonuclease mediated genome editing of the *Illrap* gene in B6(SJL)-*Apoe^{tm1.1(APOE*4)Adiuj} Trem2^{em1Adiuj}*/J mice.

The double mutant B6(SJL)-*Apoe^{tm1.1(APOE*4)Adiuj} Trem2^{em1Adiuj}*/J was generated by crossing the single mutant lines, as described above.

For the *Il1rap^{em1Adiuj}* allele: plasmids encoding a single guide RNA designed to introduce a knock-out mutation, 387bp deletion in the first coding exon, into the *Illrap* gene and the *cas9* nuclease were introduced into the cytoplasm of B6(SJL)-*Apoe^{tm1.1(APOE*4)Adiuj} Trem2^{em1Adiuj}*/J-derived fertilized eggs with well recognized pronuclei. Correctly targeted embryos were transferred to pseudopregnant females. Correctly targeted pups were identified by sequencing and PCR and further bred to B6(SJL)-*Apoe^{tm1.1(APOE*4)Adiuj}Trem2^{em1Adiuj}*/J to develop the colony.

Some aspects of the present invention provide a genetically modified mouse comprising a genomic nucleic acid encoding human APOE4, a genomic nucleic acid encoding mouse TREM2 modified to include a R47H substitution, and a genomic nucleic acid encoding mouse APP modified to include G601R, F606Y, and R609H substitutions (a humanized *App* gene). Also disclosed herein but not forming part of the claimed invention is a genetically modified mouse comprising a genomic nucleic acid encoding human APOE4, a genomic nucleic acid encoding mouse TREM2 modified to include a R47H substitution, and at least one genomic modification selected from the group consisting of: (a) a genomic nucleic acid encoding mouse ABCA7 modified to include an A1541G substitution (a humanized *Abca7* gene); (b) a genomic nucleic acid encoding mouse PLCG2 modified to include a M28L substitution (a humanized *Plcg2* gene); (c) a genomic nucleic acid encoding mouse MTHFR modified to include a A262V substitution; (d) an inactivated *Ceacam1* allele; and (e) an inactivated *Illrap* allele.

In some examples, a genetically modified mouse comprises a genomic nucleic acid encoding human APOE4, a genomic nucleic acid encoding mouse TREM2 modified to include a R47H substitution, and a genomic nucleic acid encoding mouse ABCA7 modified to include an A1541G substitution.

As described in the present invention, a genetically modified mouse comprises a genomic nucleic acid encoding human APOE4, a genomic nucleic acid encoding mouse TREM2 modified to include a R47H substitution, and a genomic nucleic acid encoding mouse APP modified to include G601R, F606Y, and R609H substitutions.

In some examples, a genetically modified mouse comprises a genomic nucleic acid encoding human APOE4, a genomic nucleic acid encoding mouse TREM2 modified to include a R47H substitution, and a genomic nucleic acid encoding mouse PLCG2 modified to include a M28L substitution.

In some examples, a genetically modified mouse comprises a genomic nucleic acid encoding human APOE4, a genomic nucleic acid encoding mouse TREM2 modified to include a R47H substitution, and a genomic nucleic acid encoding mouse MTFHR modified to include a A262V substitution.

In some examples, a genetically modified mouse comprises a genomic nucleic acid encoding human APOE4, a genomic nucleic acid encoding mouse TREM2 modified to include a R47H substitution, and an inactivated *Ceacam1* allele.

In some examples, a genetically modified mouse comprises a genomic nucleic acid encoding human APOE4, a genomic nucleic acid encoding mouse TREM2 modified to include a R47H substitution, and an inactivated *Illrap* allele.

### Methylenetetrahydrofolate reductase (MTHFR)

MTHFR is the rate-limiting enzyme in the methyl cycle. It is involved in the conversion of 5,10-methylenetetrahydrofolate to 5-methyltetrahydrofolate, which is a cosubstrate for homocysteine remethylation to methionine. Homozygous knockout (KO) of MTHFR causes an abnormally high level of homocysteine in blood and urine, called hyperhomocysteinemia. Patients with this disorder exhibit developmental delay, thrombosis, motor and gait dysfunction, seizures, and other neurological symptoms. Mild MTHFR deficiency due to polymorphic variation causes a more modest elevation of plasma homocysteine (mild hyperhomocysteinemia), which is associated with increased risk for some common multifactorial disorders. Homozygous *Mthfr* KO mice exhibit motor and behavioral anomalies, memory impairment, and liver steatosis. Homozygous KO males have normal fertility and relatively normal viability (~80%). These mice on a C57BL/6 background have a milder phenotype than on a BALB/cJ background, although mice on both backgrounds exhibit a 10-fold increase in plasma homocysteine levels.

### ATP-binding cassette, sub-family A, member 7 (ABCA7)

*ABCA7* is a member of the ATP-binding cassette genes family. The ABCA7 gene product is involved in lipid transport. ABCA7 is highly expressed in hippocampal CA1 neurons and microglia. Gene expression levels of *ABCA7* increase with AD pathology. Genome-wide association studies (GWAS) have reported association of *ABCA7* with Alzheimer's disease. Mutations in the *ABCA7* locus have been associated with genetic risk of late-onset Alzheimer's disease. In particular, rs3752246a is single nucleotide polymorphism in the human *ABCA7* gene, which encodes a mutation of alanine to glycine at position 1527 in exon 33 and which is associated with genetic risk of late-onset Alzheimer's disease. Thus, genetically modified mice according to some examples of the present disclosure include the corresponding mutation in the homologous region of exon 32 of the mouse *Abca7* gene, which results in a A1541G substitution in the AMCA7 protein.

### Amyloid Precursor Protein (APP)

One of the hallmarks of Alzheimer's disease is amyloid plaques, which include aggregates of a 42 amino acid fragment. This peptide is created by cleaving the amyloid precursor protein at the beta- and gamma-cleavage sites, and is called "Abeta42".

The mouse and human amyloid precursor proteins are highly conserved, and the beta- and gamma-cleavage sites are identical between human and mouse proteins. However, there are 3 amino acid differences between human and mouse within the ABETA42 sequence. There is some evidence that the human form of the amyloid precursor protein is more prone to aggregation and therefore may be more likely to lead to Alzheimer's disease than the mouse protein, see Li, L., et al., DNA Seq, 1999. 10(4-5): p. 219-28; Podlisny, M.B. et al., Am J Pathol, 1991. 138(6): p. 1423-35; Rosen, R.F., et al., Neurobiol Aging, 2016. 44: p. 185-196; and Roychaudhuri, R., et al., ACS Chem Neurosci, 2015. 6(12): p. 1941-55. Thus, genetically modified mice according to aspects of the present disclosure include three corresponding mutations, G601R, F606Y, and R609H, in the homologous region in exon 14 of the mouse *App* gene so that the mouse ABETA42 sequence is identical to the human ABETA42 sequence.

### Phospholipase C y2 (PLCG2)

The *PLCG2* locus has been identified by genome-wide association studies to be related to the risk of Alzheimer's disease. Specifically, the PLCG2 M28L point mutation in the human *PLCG2*locus is associated with increased AD risk. Thus, genetically modified mice according to examples of the present disclosure include a M28L mutation in the homologous region of the mouse gene.

### Carcinoembryonic antigen-related cell adhesion molecule 1

Carcinoembryonic antigen-related cell adhesion molecule 1 (CEACAM1) is a cell adhesion protein that mediates hemophilic cell adhesion in a calcium-independent manner. The *CEACAM1* gene has been related to the genetic risk of Alzheimer's disease by genome-wide association studies. Genetically modified mice according to examples of the present disclosure include a knockout of expression of CEACAM1 (e.g., an inactivated *Ceacam1* allele) such that functional CEACAM1 is not produced in the genetically modified mice.

### Interleukin 1 receptor accessory protein

The interleukin 1 receptor accessory protein (*IL1RAP*) gene has been related to the genetic risk of Alzheimer's disease by genome-wide association studies. This includes amyloid accumulation, temporal cortical atrophy and risk to progress from mild cognitive impairment to Alzheimer's disease. However, individual coding variants do not associate with disease risk, and even the most frequent missense mutations are very rare in the general population. The strongest risk variant is in an intron, suggesting that variations in expression levels, rather than structural variations, may increase Alzheimer's disease risk. Thus, genetically modified mice according to examples of the present disclosure include a knockout of expression of IL1RAP (e.g., an inactivated *Illrap* allele) such that functional IL1RAP is not produced in the genetically modified mice.

### Additional Examples of Genetically Modified Mice of the Present Disclosure

### Genetically modified mouse expressing human APOE4 and mouse TREM2

A human *APOE4* DNA sequence (hereinafter APOE4g or human *APOE4*) encoding human APOE4 protein is shown herein as SEQ ID NO:2. An encoded human APOE4 p is shown herein as SEQ ID NO: 1.

A mouse mutant *Trem2* DNA sequence encoding mouse TREM2 protein having the R47H point mutation is shown herein as SEQ ID NO:4. An encoded mouse TREM2*R47H p is shown herein as SEQ ID NO:3.

### Genetically modified mouse expressing human APOE4, mouse TREM2, and humanized ABCA7

As also disclosed herein, the present disclosure provides a genetically modified mouse with a genome that includes a DNA sequence encoding human APOE4 protein, a DNA sequence encoding mouse TREM2 protein having the R47H point mutation, and a DNA sequence encoding a humanized mouse ABCA7 protein having the A1541G point mutation (modification in exon 32 of the mouse *Abca7* gene).

A mouse mutant *Abca7* DNA sequence encoding mouse ABCA7 protein having the A1541G point mutation is shown herein as SEQ ID NO: 19. An encoded humanized ABCA7* A1541G p is shown herein as SEQ ID NO:20.

### Genetically modified mouse expressing human APOE4, mouse TREM2, and humanized APP

The present invention provides a genetically modified mouse with a genome that includes a DNA sequence encoding human APOE4 protein, a DNA sequence encoding mouse TREM2 protein having the R47H point mutation, and a DNA sequence encoding a humanized mutant mouse APP having G601R, F606Y, and R609H point mutations (modifications in exon 14 of the mouse *App* gene).

A mouse mutant *App* DNA sequence encoding mouse APP protein having G601R, F606Y, and R609H point mutations is shown herein as SEQ ID NO:23. An encoded humanized APP* p is shown herein as SEQ ID NO:24.

### Genetically modified mouse expressing human APOE4, mouse TREM2, and humanized PLCG2

As also disclosed herein, the present disclosure provides a genetically modified mouse with a genome that includes a DNA sequence encoding human APOE4 protein, a DNA sequence encoding mouse TREM2 protein having the R47H point mutation, and a DNA sequence encoding a humanized mutant mouse PLCG2 having an M28L point mutation.

A mouse mutant *Plcg2* DNA sequence encoding the first 50 amino acids mouse PLCG2 protein having an M28L point mutation is shown herein as SEQ ID NO:29. An encoded PLCG2 (first 50 amino acids) is shown herein as SEQ ID NO:30. A mouse mutant *Plcg2* DNA sequence encoding mouse PLCG2 protein having an M28L point mutation is shown herein as SEQ ID NO:31. An encoded humanized PLCG2*M28L p is shown herein as SEQ ID NO:32.

### Genetically modified mouse expressing human APOE4, mouse TREM2, and humanized MTHFR

As also disclosed herein, the present disclosure provides a genetically modified mouse with a genome that includes a DNA sequence encoding human APOE4 protein, a DNA sequence encoding mouse TREM2 protein having the R47H point mutation, and a DNA sequence encoding a mutant mouse MTHFR having an A262V point mutation.

A mouse mutant *Mthfr* DNA sequence encoding the mouse MTHFR protein having an A262V point mutation is shown herein as SEQ ID NO:49. An encoded MTHFR (is shown herein as SEQ ID NO:50.

### Genetically modified mouse expressing human APOE4, mouse TREM2, and having a knockout of mouse CEACAM1 expression

As also disclosed herein, the present disclosure provides a genetically modified mouse with a genome that includes a DNA sequence encoding human APOE4 protein, a DNA sequence encoding mouse TREM2 protein having the R47H point mutation, and a disruption of the DNA sequence encoding a mouse CEACAM1 (e.g., an inactivated mouse *Ceacam1* allele) such that no functional CEACAM1 is expressed.

### Genetically modified mouse expressing human APOE4, mouse TREM2, and knockout of mouse interleukin 1 receptor accessory protein (IL1RAP)

As also disclosed herein, the present disclosure provides a genetically modified mouse with a genome that includes a DNA sequence encoding human APOE4 protein, a DNA sequence encoding mouse TREM2 protein having the R47H point mutation, and a disruption of the DNA sequence encoding a mouse IL1RAP (e.g., an inactivated mouse *Illrap* allele) such that no functional IL1RAP is expressed.

### Variants

One or more genetic modifications can be introduced into a mouse genome to encode a variant of one or more of: APOE4 p, TREM2*R47H p, ABCA7*A1541G p, APP* p, PLCG2*M28L p, and MTHFR*A262V p in a genetically modified mouse according to embodiments of methods of the present disclosure.

As used herein, the term "variant" refers to any one or more of APOE4 p, TREM2*R47H p, ABCA7* A1541G p, APP* p, PLCG2*M28L p, and MTHFR*A262V p containing one or more mutations in its amino acid sequence compared to the corresponding protein of SEQ ID NO: 1, SEQ ID NO:3, SEQ ID NO:20, SEQ ID NO:24, SEQ ID NO:30 and SEQ ID NO:32. For example, such mutations can be one or more amino acid substitutions, additions, and/or deletions, so long as the variant of APOE4 p or TREM2*R47H p retains the functional characteristics APOE4 p, TREM2*R47H p, ABCA7*A1541G p, APP* p, PLCG2*M28L p, and MTHFR*A262V p, of SEQ ID NO:1, SEQ ID NO:3, SEQ ID NO:20, SEQ ID NO:24, SEQ ID NO:30, SEQ ID NO:32, and SEQ ID NO:49, respectively.

In particular embodiments, a variant APOE4 p according to embodiments of the present disclosure has at least 90%, 91%, 92%, 93%, 94%, 95%, 96%, 97%, 98%, 99% or greater identity to SEQ ID NO:1 over its entire length, and has R (Arginine) and R (Arginine) as amino acids 130 and 176 of the APOE4 protein including an 18 amino acid signal peptide, shown herein as SEQ ID NO:1, as well as retains the functional characteristics of APOE4 p of SEQ ID NO:1.

In particular embodiments, a variant APOE4 p according to embodiments of the present disclosure has at least 90%, 91%, 92%, 93%, 94%, 95%, 96%, 97%, 98%, 99% or greater identity to SEQ ID NO:15 over its entire length, and has R (Arginine) and R (Arginine) as amino acids 112 and 158 of the APOE4 protein not including the 18 amino acid signal peptide, shown herein as SEQ ID NO:15, as well as retains the functional characteristics of APOE4 p of SEQ ID NO:15.

In particular embodiments, a variant TREM2*R47H p according to embodiments of the present disclosure has at least 90%, 91%, 92%, 93%, 94%, 95%, 96%, 97%, 98%, 99% or greater identity to SEQ ID NO:3 over its entire length and retains the functional characteristics of TREM2*R47H p of SEQ ID NO:3.

In particular embodiments, a variant ABCA7*A1541G p according to embodiments of the present disclosure has at least 90%, 91%, 92%, 93%, 94%, 95%, 96%, 97%, 98%, 99% or greater identity to SEQ ID NO:20 over its entire length and retains the functional characteristics of ABCA7*A1541G p of SEQ ID NO:20.

In particular embodiments, a variant APP* p according to embodiments of the present disclosure has at least 90%, 91%, 92%, 93%, 94%, 95%, 96%, 97%, 98%, 99% or greater identity to SEQ ID NO:24 over its entire length and retains the functional characteristics of APP* p of SEQ ID NO:24.

In particular embodiments, a variant PLCG2*M28L p according to embodiments of the present disclosure has at least 90%, 91%, 92%, 93%, 94%, 95%, 96%, 97%, 98%, 99% or greater identity to SEQ ID NO:30 over its entire length and retains the functional characteristics of PLCG2*M28L p of SEQ ID NO:30.

In particular embodiments, a variant PLCG2*M28L p according to embodiments of the present disclosure has at least 90%, 91%, 92%, 93%, 94%, 95%, 96%, 97%, 98%, 99% or greater identity to SEQ ID NO:32 over its entire length and retains the functional characteristics of PLCG2*M28L p of SEQ ID NO:32.

Mutations can be introduced using standard molecular biology techniques, such as CRISPR technology. Alternative techniques include site-directed mutagenesis and PCR-mediated mutagenesis and the like. One of skill in the art will recognize that one or more amino acid mutations can be introduced without altering the functional properties of APOE4 p and mouse TREM2*R47H p proteins.

Assays for assessment of functional properties of APOE4 p, TREM2*R47H p, ABCA7* A1541G p, APP* p, PLCG2*M28L p, and MTHFR*A262V p and variants are known in the art.

Conservative amino acid substitutions can be made in any of APOE4 p, TREM2*R47H p, ABCA7* A1541G p, APP* p, PLCG2*M28L p, and MTHFR*A262V p proteins to produce a corresponding APOE4 p, TREM2*R47H p, ABCA7* A1541G p, APP* p, PLCG2*M28L p, and MTHFR*A262V p variant. Conservative amino acid substitutions are art recognized substitutions of one amino acid for another amino acid having similar characteristics. For example, each amino acid may be described as having one or more of the following characteristics: electropositive, electronegative, aliphatic, aromatic, polar, hydrophobic and hydrophilic. A conservative substitution is a substitution of one amino acid having a specified structural or functional characteristic for another amino acid having the same characteristic. Acidic amino acids include aspartate, glutamate; basic amino acids include histidine, lysine, arginine; aliphatic amino acids include isoleucine, leucine and valine; aromatic amino acids include phenylalanine, glycine, tyrosine and tryptophan; polar amino acids include aspartate, glutamate, histidine, lysine, asparagine, glutamine, arginine, serine, threonine and tyrosine; and hydrophobic amino acids include alanine, cysteine, phenylalanine, glycine, isoleucine, leucine, methionine, proline, valine and tryptophan; and conservative substitutions include substitution among amino acids within each group. Amino acids may also be described in terms of relative size, alanine, cysteine, aspartate, glycine, asparagine, proline, threonine, serine, valine, all typically considered to be small.

APOE4 p, TREM2*R47H p, ABCA7*A1541G p, APP* p, PLCG2*M28L p, and MTHFR*A262V p variants can include synthetic amino acid analogs, amino acid derivatives and/or non-standard amino acids, illustratively including, without limitation, alpha-aminobutyric acid, citrulline, canavanine, cyanoalanine, diaminobutyric acid, diaminopimelic acid, dihydroxy-phenylalanine, djenkolic acid, homoarginine, hydroxyproline, norleucine, norvaline, 3-phosphoserine, homoserine, 5-hydroxytryptophan, 1-methylhistidine, 3-methylhistidine, and ornithine.

It will be appreciated by those of ordinary skill in the art that, due to the degenerate nature of the genetic code, nucleic acid sequences other than SEQ ID NO:2 and SEQ ID NO:4 encode APOE4 p and TREM2*R47H p, respectively, and that such alternate nucleic acids may be introduced into a mouse genome to produce a genetically modified mouse expressing APOE4 p and TREM2*R47H p of the disclosure.

It will be appreciated by those of ordinary skill in the art that, due to the degenerate nature of the genetic code, nucleic acid sequences other than SEQ ID NO:19 encode ABCA7* A1541G p, and that such alternate nucleic acids may be introduced into a mouse genome to produce a genetically modified mouse expressing ABCA7* A1541G p of the disclosure.

It will be appreciated by those of ordinary skill in the art that, due to the degenerate nature of the genetic code, nucleic acid sequences other than SEQ ID NO:23 encode APP* p, and that such alternate nucleic acids may be introduced into a mouse genome to produce a genetically modified mouse expressing APP* p of the disclosure.

It will be appreciated by those of ordinary skill in the art that, due to the degenerate nature of the genetic code, nucleic acid sequences other than SEQ ID NO:29 encode PLCG2*M28L p fragment of SEQ ID NO:30, and that such alternate nucleic acids may be introduced into a mouse genome to produce a genetically modified mouse expressing PLCG2*M28L p of the disclosure.

It will be appreciated by those of ordinary skill in the art that, due to the degenerate nature of the genetic code, nucleic acid sequences other than SEQ ID NO:31 encode PLCG2*M28L p of SEQ ID NO:32, and that such alternate nucleic acids may be introduced into a mouse genome to produce a genetically modified mouse expressing PLCG2*M28L p of the disclosure.

The singular terms "a," "an," and "the" are not intended to be limiting and include plural referents unless explicitly stated otherwise or the context clearly indicates otherwise.

The terms "expressing" and "expresses" refer to transcription of a gene to produce a corresponding mRNA and/or translation of the mRNA to produce the corresponding protein.

The complement of a nucleic acid encoding an APOE4 p variant specifically hybridizes with SEQ ID NO:2 encoding APOE4 p under high stringency conditions. The complement of a nucleic acid encoding a TREM2*R47H p variant specifically hybridizes with SEQ ID NO:4 encoding TREM2*R47H p under high stringency conditions. The complement of a nucleic acid encoding a ABCA7*A1541G p variant specifically hybridizes with SEQ ID NO:19 encoding ABCA7*A1541G p under high stringency conditions. The complement of a nucleic acid encoding a APP* p variant specifically hybridizes with SEQ ID NO:23 encoding APP* p under high stringency conditions. The complement of a nucleic acid encoding a PLCG2*M28L p fragment variant specifically hybridizes with SEQ ID NO:29 encoding a PLCG2*M28L p fragment under high stringency conditions. The complement of a nucleic acid encoding a PLCG2*M28L p variant specifically hybridizes with SEQ ID NO:31 encoding PLCG2*M28L p under high stringency conditions.

The term "nucleic acid" refers to RNA or DNA molecules having more than one nucleotide in any form including single-stranded, double-stranded, oligonucleotide or polynucleotide. The term "nucleotide sequence" refers to the ordering of nucleotides in an oligonucleotide or polynucleotide in a single-stranded form of nucleic acid.

The terms "hybridization" and "hybridizes" refer to pairing and binding of complementary nucleic acids. Hybridization occurs to varying extents between two nucleic acids depending on factors such as the degree of complementarity of the nucleic acids, the melting temperature, Tm, of the nucleic acids and the stringency of hybridization conditions, as is well known in the art.

The term "stringency of hybridization conditions" refers to conditions of temperature, ionic strength, and composition of a hybridization medium with respect to particular common additives such as formamide and Denhardt's solution. Determination of particular hybridization conditions relating to a specified nucleic acid is routine and is well known in the art, for instance, as described in J. Sambrook and D.W. Russell, Molecular Cloning: A Laboratory Manual, Cold Spring Harbor Laboratory Press; 3rd Ed., 2001; and F.M. Ausubel, Ed., Short Protocols in Molecular Biology, Current Protocols; 5th Ed., 2002. High stringency hybridization conditions are those which only allow hybridization of substantially complementary nucleic acids. Typically, nucleic acids having about 85-100% complementarity are considered highly complementary and hybridize under high stringency conditions. Intermediate stringency conditions are exemplified by conditions under which nucleic acids having intermediate complementarity, about 50-84% complementarity, as well as those having a high degree of complementarity, hybridize. In contrast, low stringency hybridization conditions are those in which nucleic acids having a low degree of complementarity hybridize.

The terms "specific hybridization" and "specifically hybridizes" refer to hybridization of a particular nucleic acid to a target nucleic acid without substantial hybridization to nucleic acids other than the target nucleic acid in a sample.

Stringency of hybridization and washing conditions depends on several factors, including the Tm of the probe and target and ionic strength of the hybridization and wash conditions, as is well-known to the skilled artisan. Hybridization and conditions to achieve a desired hybridization stringency are described, for example, in Sambrook et al., Molecular Cloning: A Laboratory Manual, Cold Spring Harbor Laboratory Press, 2001; and Ausubel, F. et al., (Eds.), Short Protocols in Molecular Biology, Wiley, 2002.

An example of high stringency hybridization conditions is hybridization of nucleic acids over about 100 nucleotides in length in a solution containing 6X SSC, 5X Denhardt's solution, 30% formamide, and 100 micrograms/ml denatured salmon sperm at 37°C overnight followed by washing in a solution of 0.1X SSC and 0.1% SDS at 60°C for 15 minutes. SSC is 0.15M NaCl/0.015M Na citrate. Denhardt's solution is 0.02% bovine serum albumin/0.02% FICOLL/0.02% polyvinylpyrrolidone.

The term "complementary" refers to Watson-Crick base pairing between nucleotides and specifically refers to nucleotides hydrogen bonded to one another with thymine or uracil residues linked to adenine residues by two hydrogen bonds and cytosine and guanine residues linked by three hydrogen bonds. In general, a nucleic acid includes a nucleotide sequence described as having a "percent complementarity" to a specified second nucleotide sequence. For example, a nucleotide sequence may have 80%, 90%, or 100% complementarity to a specified second nucleotide sequence, indicating that 8 of 10, 9 of 10 or 10 of 10 nucleotides of a sequence are complementary to the specified second nucleotide sequence. For instance, the nucleotide sequence 3'-TCGA-5' is 100% complementary to the nucleotide sequence 5'-AGCT-3'. Further, the nucleotide sequence 3'-TCGA- is 100% complementary to a region of the nucleotide sequence 5'-TTAGCTGG-3'.

To determine the percent identity of two amino acid sequences or of two nucleic acid sequences, the sequences are aligned for optimal comparison purposes (e.g., gaps can be introduced in the sequence of a first amino acid or nucleic acid sequence for optimal alignment with a second amino acid or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (i.e., % identity=number of identical overlapping positions/total number of positions x 100%). In one embodiment, the two sequences are the same length or differ in length by no more than 1%, 2%, 3%, 4%, 5%, 6%, 7%, 8%, 9% or 10% of the total length of the reference sequence.

The determination of percent identity between two sequences can also be accomplished using a mathematical algorithm. A preferred, non-limiting example of a mathematical algorithm utilized for the comparison of two sequences is the algorithm of Karlin and Altschul, 1990, PNAS 87:2264 2268, modified as in Karlin and Altschul, 1993, PNAS. 90:5873 5877. Such an algorithm is incorporated into the NBLAST and XBLAST programs of Altschul et al., 1990, J. Mol. Biol. 215:403. BLAST nucleotide searches are performed with the NBLAST nucleotide program parameters set, e.g., for score=100, wordlength=12 to obtain nucleotide sequences homologous to a nucleic acid molecules of the present disclosure. BLAST protein searches are performed with the XBLAST program parameters set, e.g., to score 50, wordlength=3 to obtain amino acid sequences homologous to a protein molecule of the present disclosure. To obtain gapped alignments for comparison purposes, Gapped BLAST is utilized as described in Altschul et al., 1997, Nucleic Acids Res. 25:3389 3402. Alternatively, PSI BLAST is used to perform an iterated search which detects distant relationships between molecules (Id.). When utilizing BLAST, Gapped BLAST, and PSI Blast programs, the default parameters of the respective programs (e.g., of XBLAST and NBLAST) are used (see, e.g., the NCBI website). Another preferred, non-limiting example of a mathematical algorithm utilized for the comparison of sequences is the algorithm of Myers and Miller, 1988, CABIOS 4:11 17. Such an algorithm is incorporated in the ALIGN program (version 2.0) which is part of the GCG sequence alignment software package. When utilizing the ALIGN program for comparing amino acid sequences, a PAM120 weight residue table, a gap length penalty of 12, and a gap penalty of 4 is used.

The percent identity between two sequences is determined using techniques similar to those described above, with or without allowing gaps. In calculating percent identity, typically only exact matches are counted.

Nucleic acids encoding APOE4 p, TREM2*R47H p, ABCA7*A1541G p, APP* p, PLCG2*M28L p, or MTHFR*A262V p, or a variant of any thereof can be isolated from natural sources, generated recombinantly or made by chemical synthetic techniques using well-known methodology.

### Genetic Modification

A genetically modified mouse is provided according to embodiments of the present invention whose genome includes a nucleic acid encoding APOE4 p operably linked to a promoter, wherein the animal expresses the encoded APOE4 p, whose genome includes a nucleic acid encoding TREM2*R47H p operably linked to a promoter, wherein the animal expresses the encoded TREM2*R47H p, and whose genome includes a nucleic acid encoding: APP* p operably linked to a promoter, wherein the animal expresses the encoded APP* p. Also disclosed herein but not forming part of the claimed invention is a genetically modified mouse whose genome includes a nucleic acid encoding APOE4 p operably linked to a promoter, wherein the animal expresses the encoded APOE4 p, whose genome includes a nucleic acid encoding TREM2*R47H p operably linked to a promoter, wherein the animal expresses the encoded TREM2*R47H p, and whose genome includes one or more of the following: (1) a nucleic acid encoding: ABCA7*A1541G p operably linked to a promoter, wherein the animal expresses the encoded ABCA7* A1541G p; (2) a nucleic acid encoding: PLCG2*M28L p operably linked to a promoter, wherein the animal expresses the encoded PLCG2*M28L p; (3) a nucleic acid encoding: MTFHR*A262V p operably linked to a promoter, wherein the animal expresses the encoded MTFHR*A262V p; (4) a disrupted *Ceacam1* gene such that functional CEACAM1 is not produced in the genetically modified mouse; and (5) a disrupted Il1rap gene such that functional IL1RAP is not produced in the genetically modified mouse.

A genetically modified mouse is provided according to embodiments of the present invention whose genome includes a nucleic acid encoding APOE4 p operably linked to the endogenous mouse Apoe promoter, wherein the animal expresses the encoded APOE4 p, whose genome includes a nucleic acid encoding TREM2*R47H p operably linked to the endogenous mouse TREM2*R47H p promoter, wherein the animal expresses the encoded TREM2*R47H p, and whose genome includes a nucleic acid encoding: APP* p operably linked to a promoter, wherein the animal expresses the encoded APP* p. Also disclosed herein but not forming part of the claimed invention is a genetically modified mouse whose genome includes a nucleic acid encoding APOE4 p operably linked to the endogenous mouse Apoe promoter, wherein the animal expresses the encoded APOE4 p, whose genome includes a nucleic acid encoding TREM2*R47H p operably linked to the endogenous mouse TREM2*R47H p promoter, wherein the animal expresses the encoded TREM2*R47H p, and whose genome includes one or more of the following: (1) a nucleic acid encoding: ABCA7* A1541G p operably linked to a promoter, wherein the animal expresses the encoded ABCA7* A1541G p; (2) a nucleic acid encoding: PLCG2*M28L p operably linked to a promoter, wherein the animal expresses the encoded PLCG2*M28L p; (3) a nucleic acid encoding: MTFHR*A262V p operably linked to a promoter, wherein the animal expresses the encoded MTFHR*A262V p; (4) a disrupted *Ceacam1* gene such that functional CEACAM1 is not produced in the genetically modified mouse; and (5) a disrupted Il1rap gene such that functional IL1RAP is not produced in the genetically modified mouse.

Any of various methods can be used to introduce a genetic modification into a mouse genome to produce a genetically modified mouse expressing APOE4 p, TREM2*R47H p, and one or more of: ABCA7*A1541G p, APP* p, and PLCG2*M28L p, or a variant of any thereof.

Genome editing methods for generating a genetically modified mouse according to embodiments of the present invention whose genome includes a nucleic acid encoding APOE4 p operably linked to a promoter, wherein the animal expresses the encoded APOE4 p, whose genome includes a nucleic acid encoding TREM2*R47H p operably linked to a promoter, wherein the animal expresses the encoded TREM2*R47H p, and whose genome includes a nucleic acid encoding: APP* p operably linked to a promoter, wherein the animal expresses the encoded APP* p. Also disclosed herein herein but not forming part of the claimed invention are genome editing methods for generating a genetically modified mouse according to embodiments of the present disclosure whose genome includes a nucleic acid encoding APOE4 p operably linked to a promoter, wherein the animal expresses the encoded APOE4 p, whose genome includes a nucleic acid encoding TREM2*R47H p operably linked to a promoter, wherein the animal expresses the encoded TREM2*R47H p, and whose genome includes one or more of the following: (1) a nucleic acid encoding: ABCA7* A1541G p operably linked to a promoter, wherein the animal expresses the encoded ABCA7* A1541G p; (2) a nucleic acid encoding: PLCG2*M28L p operably linked to a promoter, wherein the animal expresses the encoded PLCG2*M28L p; (3) a nucleic acid encoding: MTFHR*A262V p operably linked to a promoter, wherein the animal expresses the encoded MTFHR*A262V p; (4) a disrupted *Ceacam1* gene such that functional CEACAM1 is not produced in the genetically modified mouse; and (5) a disrupted Il1rap gene such that functional IL1RAP is not produced in the genetically modified mouse, include, but are not limited to, site directed mutagenesis, recombination-based methods and nuclease genome editing techniques.

Genome editing techniques can be used to modify a genomic sequence by introduction of a discrete mutation at a predetermined target site in the genome.

For example, one or more nucleotides in a genomic sequence can be replaced with one or more different nucleotides using a genome editing technique so that the genomic sequence encodes a protein with a single amino acid difference, or multiple amino acid differences, compared to the unmodified genomic sequence.

Genome editing techniques can also be used to modify a genomic sequence by insertion of a coding sequence into the genome at a predetermined target site, a "knock-in" technique.

Genome editing techniques can also be used to modify a genomic sequence by insertion or deletion of a coding sequence or non-coding into the genome at a predetermined target site to disrupt or knockout a gene such that the gene is no longer capable of expressing the functional protein originally encoded.

As used herein, the terms "target site" and "target sequence" in the general context of a genetic editing technique refer to a nucleic acid sequence that defines a portion of a chromosomal sequence to be edited.

For example, a nucleic acid sequence encoding a protein can be inserted at a predetermined target site in the genome so that the genome includes a nucleic acid encoding the protein and the protein is expressed. The nucleic acid sequence can also contain a promoter to drive expression of the encoded protein or expression of the encoded protein can be driven by an endogenous promoter when the nucleic acid is inserted in a position so that it is operably linked to the endogenous promoter.

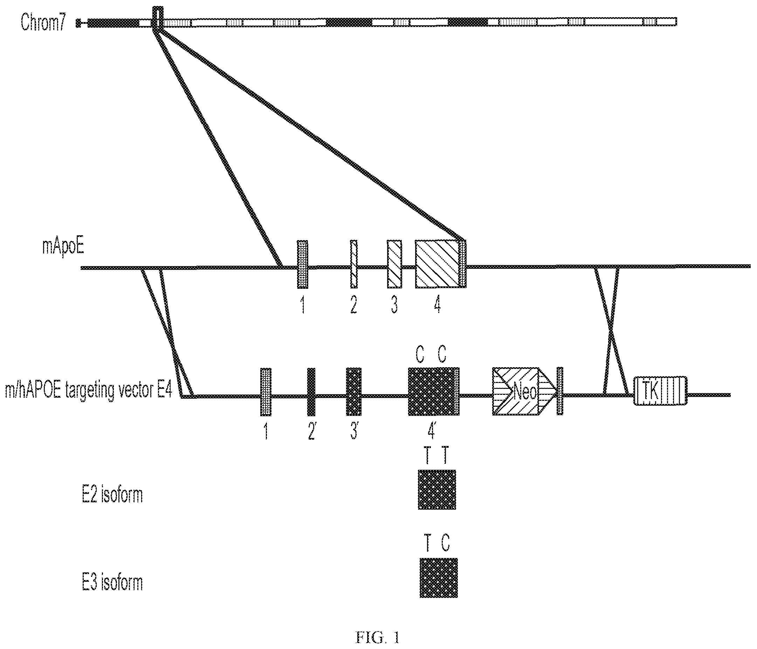

According to particular aspects of the present disclosure, a point mutation is introduced into the genome of a first mouse using a genome editing technique so that the mouse encodes TREM2*R47H p, and a nucleic acid encoding human APOE4 is inserted into the Apoe4 gene in the genome of a second mouse by a knock-in genome editing technique so that the genome of the second mouse contains a humanized APOE4 gene as shown in Figure 1, containing exon 1 of the mouse Apoe4 gene and exons 2, 3 and 4 of the human APOE4 gene. The first and second mice are then bred, naturally or by artificial methods, to obtain a genetically modified mouse whose genome includes a DNA sequence encoding APOE4 p and a DNA sequence encoding TREM2*R47H p.

According to particular aspects of the present disclosure, a point mutation is introduced into the genome of a first mouse using CRISPR genome editing technique so that the mouse encodes TREM2*R47H p, and a nucleic acid encoding human APOE4 is introduced into the Apoe4 gene in the genome of a second mouse by a knock-in CRISPR genome editing technique so that the genome of the second mouse contains a "humanized" APOE4 gene as shown in Figure 1, containing exon 1 of the mouse Apoe4 gene and exons 2, 3 and 4 of the human APOE4 gene. The first and second mice are then bred naturally or by artificial methods to produce a genetically modified mouse whose genome includes a DNA sequence encoding APOE4 p and a DNA sequence encoding TREM2*R47H p.

According to particular examples of the present disclosure, one or more point mutations are introduced into the genome of a first mouse which encodes both TREM2*R47H p and human APOE4 using a genome editing technique so that the mouse encodes ABCA7* A1541G p. Alternatively, one or more point mutations are introduced into the genome of a first mouse using a genome editing technique so that the mouse encodes ABCA7*A1541G p and a second mouse is provided which encodes both TREM2*R47H p and human APOE4. The first and second mice are then bred, naturally or by artificial methods, to obtain a genetically modified mouse whose genome includes a DNA sequence encoding APOE4 p, a DNA sequence encoding TREM2*R47H p and a DNA sequence encoding ABCA7*A1541G p, wherein all of APOE4 p, TREM2*R47H p and ABCA7*A1541G p are expressed in the genetically modified mouse. Further alternatives are possible such as where a first mouse is produced which has a genome which includes a DNA sequence encoding TREM2*R47H p and a DNA sequence encoding ABCA7*A1541G p and a second mouse is produced which has a genome which includes a DNA sequence encoding human APOE4 or where a first mouse is produced which has a genome which includes a DNA sequence encoding human APOE4 and a DNA sequence encoding ABCA7*A1541G p and a second mouse is produced which has a genome which includes a DNA sequence encoding TREM2*R47H p. The first and second mice are then bred, naturally or by artificial methods, to obtain a genetically modified mouse whose genome includes a DNA sequence encoding APOE4 p, a DNA sequence encoding TREM2*R47H p and a DNA sequence encoding ABCA7*A1541G p, wherein all of APOE4 p, TREM2*R47H p and ABCA7* A1541G p are expressed in the genetically modified mouse.

According to particular examples of the present disclosure, one or more point mutations are introduced into the genome of a first mouse which encodes both TREM2*R47H p and human APOE4 using a CRISPR genome editing technique so that the mouse encodes ABCA7* A1541G p. Alternatively, one or more point mutations are introduced into the genome of a first mouse using a CRISPR genome editing technique so that the mouse encodes ABCA7*A1541G p and a second mouse is provided which encodes both TREM2*R47H p and human APOE4. The first and second mice are then bred, naturally or by artificial methods, to obtain a genetically modified mouse whose genome includes a DNA sequence encoding APOE4 p, a DNA sequence encoding TREM2*R47H p and a DNA sequence encoding ABCA7*A1541G p, wherein all of APOE4 p, TREM2*R47H p and ABCA7* A1541G p are expressed in the genetically modified mouse.

According to particular aspects of the present disclosure, point mutations are introduced into the genome of a first mouse which encodes both TREM2*R47H p and human APOE4 using a genome editing technique so that the mouse encodes APP* p. Alternatively, point mutations are introduced into the genome of a first mouse using a genome editing technique so that the mouse encodes APP* p and a second mouse is provided which encodes both TREM2*R47H p and human APOE4. The first and second mice are then bred, naturally or by artificial methods, to obtain a genetically modified mouse whose genome includes a DNA sequence encoding APOE4 p, a DNA sequence encoding TREM2*R47H p and a DNA sequence encoding APP* p, wherein all of APOE4 p, TREM2*R47H p and APP* p are expressed in the genetically modified mouse. Further alternatives are possible such as where a first mouse is produced which has a genome which includes a DNA sequence encoding TREM2*R47H p and a DNA sequence encoding APP* p and a second mouse is produced which has a genome which includes a DNA sequence encoding human APOE4 or where a first mouse is produced which has a genome which includes a DNA sequence encoding human APOE4 and a DNA sequence encoding APP* p and a second mouse is produced which has a genome which includes a DNA sequence encoding TREM2*R47H p. The first and second mice are then bred, naturally or by artificial methods, to obtain a genetically modified mouse whose genome includes a DNA sequence encoding APOE4 p, a DNA sequence encoding TREM2*R47H p and a DNA sequence encoding APP* p, wherein all of APOE4 p, TREM2*R47H p and APP* p are expressed in the genetically modified mouse.

According to particular aspects of the present disclosure, point mutations are introduced into the genome of a first mouse which encodes both TREM2*R47H p and human APOE4 using a CRISPR genome editing technique so that the mouse encodes APP* p. Alternatively, point mutations are introduced into the genome of a first mouse using a CRISPR genome editing technique so that the mouse encodes APP* p and a second mouse is provided which encodes both TREM2*R47H p and human APOE4. The first and second mice are then bred, naturally or by artificial methods, to obtain a genetically modified mouse whose genome includes a DNA sequence encoding APOE4 p, a DNA sequence encoding TREM2*R47H p and a DNA sequence encoding APP* p, wherein all of APOE4 p, TREM2*R47H p and APP* p are expressed in the genetically modified mouse.

According to particular examples of the present disclosure, one or more point mutations are introduced into the genome of a first mouse which encodes both TREM2*R47H p and human APOE4 using a genome editing technique so that the mouse encodes PLCG2*M28L p. Alternatively, one or more point mutations are introduced into the genome of a first mouse using a genome editing technique so that the mouse encodes PLCG2*M28L p and a second mouse is provided which encodes both TREM2*R47H p and human APOE4. The first and second mice are then bred, naturally or by artificial methods, to obtain a genetically modified mouse whose genome includes a DNA sequence encoding APOE4 p, a DNA sequence encoding TREM2*R47H p and a DNA sequence encoding PLCG2*M28L p, wherein all of APOE4 p, TREM2*R47H p and PLCG2*M28L p are expressed in the genetically modified mouse. Further alternatives are possible such as where a first mouse is produced which has a genome which includes a DNA sequence encoding TREM2*R47H p and a DNA sequence encoding PLCG2*M28L p and a second mouse is produced which has a genome which includes a DNA sequence encoding human APOE4 or where a first mouse is produced which has a genome which includes a DNA sequence encoding human APOE4 and a DNA sequence encoding PLCG2*M28L p and a second mouse is produced which has a genome which includes a DNA sequence encoding TREM2*R47H p. The first and second mice are then bred, naturally or by artificial methods, to obtain a genetically modified mouse whose genome includes a DNA sequence encoding APOE4 p, a DNA sequence encoding TREM2*R47H p and a DNA sequence encoding PLCG2*M28L p, wherein all of APOE4 p, TREM2*R47H p and PLCG2*M28L p are expressed in the genetically modified mouse.

According to particular examples of the present disclosure, one or more point mutations are introduced into the genome of a first mouse which encodes both TREM2*R47H p and human APOE4 using a CRISPR genome editing technique so that the mouse encodes PLCG2*M28L p. Alternatively, one or more point mutations are introduced into the genome of a first mouse using a CRISPR genome editing technique so that the mouse encodes PLCG2*M28L p and a second mouse is provided which encodes both TREM2*R47H p and human APOE4. The first and second mice are then bred, naturally or by artificial methods, to obtain a genetically modified mouse whose genome includes a DNA sequence encoding APOE4 p, a DNA sequence encoding TREM2*R47H p and a DNA sequence encoding PLCG2*M28L p, wherein all of APOE4 p, TREM2*R47H p and PLCG2*M28L p are expressed in the genetically modified mouse.

According to particular examples of the present disclosure, one or more point mutations are introduced into the genome of a first mouse which encodes both TREM2*R47H p and human APOE4 using a genome editing technique so that the mouse encodes MTHFR*A262V p. Alternatively, one or more point mutations are introduced into the genome of a first mouse using a genome editing technique so that the mouse encodes MTHFR*A262V p and a second mouse is provided which encodes both TREM2*R47H p and human APOE4. The first and second mice are then bred, naturally or by artificial methods, to obtain a genetically modified mouse whose genome includes a DNA sequence encoding APOE4 p, a DNA sequence encoding TREM2*R47H p and a DNA sequence encoding MTHFR*A262V p, wherein all of APOE4 p, TREM2*R47H p and MTHFR*A262V p are expressed in the genetically modified mouse. Further alternatives are possible such as where a first mouse is produced which has a genome which includes a DNA sequence encoding TREM2*R47H p and a DNA sequence encoding MTHFR*A262V p and a second mouse is produced which has a genome which includes a DNA sequence encoding human APOE4 or where a first mouse is produced which has a genome which includes a DNA sequence encoding human APOE4 and a DNA sequence encoding MTHFR*A262V p and a second mouse is produced which has a genome which includes a DNA sequence encoding TREM2*R47H p. The first and second mice are then bred, naturally or by artificial methods, to obtain a genetically modified mouse whose genome includes a DNA sequence encoding APOE4 p, a DNA sequence encoding TREM2*R47H p and a DNA sequence encoding MTHFR*A262V p, wherein all of APOE4 p, TREM2*R47H p and MTHFR*A262V p are expressed in the genetically modified mouse.

According to particular examples of the present disclosure, one or more point mutations are introduced into the genome of a first mouse which encodes both TREM2*R47H p and human APOE4 using a CRISPR genome editing technique so that the mouse encodes MTHFR*A262V p. Alternatively, one or more point mutations are introduced into the genome of a first mouse using a CRISPR genome editing technique so that the mouse encodes MTHFR*A262V p and a second mouse is provided which encodes both TREM2*R47H p and human APOE4. The first and second mice are then bred, naturally or by artificial methods, to obtain a genetically modified mouse whose genome includes a DNA sequence encoding APOE4 p, a DNA sequence encoding TREM2*R47H p and a DNA sequence encoding MTHFR*A262V p, wherein all of APOE4 p, TREM2*R47H p and MTHFR*A262V p are expressed in the genetically modified mouse.

According to particular examples of the present disclosure, one or more mutations are introduced into the genome of a first mouse which encodes both TREM2*R47H p and human APOE4 using a genome editing technique to knockout the *Ceacam1* gene so that the mouse does not encode functional CEACAM1. Alternatively, one or more mutations are introduced into the genome of a first mouse using a genome editing technique so that the mouse does not encode functional CEACAM1 and a second mouse is provided which encodes both TREM2*R47H p and human APOE4. The first and second mice are then bred, naturally or by artificial methods, to obtain a genetically modified mouse whose genome includes a DNA sequence encoding APOE4 p, a DNA sequence encoding TREM2*R47H p and does not include a DNA sequence encoding functional CEACAM1, wherein all of APOE4 p, TREM2*R47H p are expressed in the genetically modified mouse and functional CEACAM1 is not expressed in the genetically modified mouse. Further alternatives are possible such as where a first mouse is produced which has a genome which includes a DNA sequence encoding TREM2*R47H p and the genome does not encode functional CEACAM1 and a second mouse is produced which has a genome which includes a DNA sequence encoding human APOE4 or where a first mouse is produced which has a genome which includes a DNA sequence encoding human APOE4 and the genome does not encode functional CEACAM1 and a second mouse is produced which has a genome which includes a DNA sequence encoding TREM2*R47H p. The first and second mice are then bred, naturally or by artificial methods, to obtain a genetically modified mouse whose genome includes a DNA sequence encoding APOE4 p, a DNA sequence encoding TREM2*R47H p and does not include a DNA sequence encoding functional CEACAM1, wherein both of APOE4 p, TREM2*R47H p are expressed in the genetically modified mouse and wherein functional CEACAM1 is not expressed in the genetically modified mouse.

According to particular examples of the present disclosure, one or more mutations are introduced into the genome of a first mouse which encodes both TREM2*R47H p and human APOE4 using a CRISPR genome editing technique so that the mouse does not encode functional CEACAM1. Alternatively, one or more mutations are introduced into the genome of a first mouse using a CRISPR genome editing technique so that the mouse does not encode functional CEACAM1 and a second mouse is provided which encodes both TREM2*R47H p and human APOE4. The first and second mice are then bred, naturally or by artificial methods, to obtain a genetically modified mouse whose genome includes a DNA sequence encoding APOE4 p and a DNA sequence encoding TREM2*R47H p and does not include a DNA sequence encoding functional CEACAM1, wherein all of APOE4 p and TREM2*R47H p are expressed in the genetically modified mouse and wherein functional CEACAM1 is not expressed in the genetically modified mouse.

According to particular examples of the present disclosure, one or more mutations are introduced into the genome of a first mouse which encodes both TREM2*R47H p and human APOE4 using a genome editing technique to knockout the Il1rap gene so that the mouse does not encode functional IL1RAP. Alternatively, one or more mutations are introduced into the genome of a first mouse using a genome editing technique so that the mouse does not encode functional IL1RAP and a second mouse is provided which encodes both TREM2*R47H p and human APOE4. The first and second mice are then bred, naturally or by artificial methods, to obtain a genetically modified mouse whose genome includes a DNA sequence encoding APOE4 p, a DNA sequence encoding TREM2*R47H p and does not include a DNA sequence encoding functional IL1RAP, wherein all of APOE4 p, TREM2*R47H p are expressed in the genetically modified mouse and functional IL1RAP is not expressed in the genetically modified mouse. Further alternatives are possible such as where a first mouse is produced which has a genome which includes a DNA sequence encoding TREM2*R47H p and the genome does not encode functional IL1RAP and a second mouse is produced which has a genome which includes a DNA sequence encoding human APOE4 or where a first mouse is produced which has a genome which includes a DNA sequence encoding human APOE4 and the genome does not encode functional IL1RAP and a second mouse is produced which has a genome which includes a DNA sequence encoding TREM2*R47H p. The first and second mice are then bred, naturally or by artificial methods, to obtain a genetically modified mouse whose genome includes a DNA sequence encoding APOE4 p, a DNA sequence encoding TREM2*R47H p and does not include a DNA sequence encoding functional IL1RAP, wherein both of APOE4 p, TREM2*R47H p are expressed in the genetically modified mouse and wherein functional IL1RAP is not expressed in the genetically modified mouse.

According to particular examples of the present disclosure, one or more mutations are introduced into the genome of a first mouse which encodes both TREM2*R47H p and human APOE4 using a CRISPR genome editing technique so that the mouse does not encode functional IL1RAP. Alternatively, one or more mutations are introduced into the genome of a first mouse using a CRISPR genome editing technique so that the mouse does not encode functional IL1RAP and a second mouse is provided which encodes both TREM2*R47H p and human APOE4. The first and second mice are then bred, naturally or by artificial methods, to obtain a genetically modified mouse whose genome includes a DNA sequence encoding APOE4 p and a DNA sequence encoding TREM2*R47H p and does not include a DNA sequence encoding functional IL1RAP, wherein all of APOE4 p and TREM2*R47H p are expressed in the genetically modified mouse and wherein functional IL1RAP is not expressed in the genetically modified mouse.

Genomic editing is performed, for example, by methods described herein, and as detailed in J. P. Sundberg and T. Ichiki, Eds., Genetically Engineered Mice Handbook, CRC Press; 2006; M. H. Hofker and J. van Deursen, Eds., Transgenic Mouse Methods and Protocols, Humana Press, 2002; A. L. Joyner, Gene Targeting: A Practical Approach, Oxford University Press, 2000; Manipulating the Mouse Embryo: A Laboratory Manual, 3rd edition, Cold Spring Harbor Laboratory Press; December 15, 2002, ISBN-10: 0879695919; Kursad Turksen (Ed.), Embryonic stem cells: methods and protocols in Methods Mol Biol. 2002;185, Humana Press; Current Protocols in Stem Cell Biology, ISBN: 978047015180; Meyer et al., PNAS USA, 2010, vol. 107 (34), 15022-15026; and Doudna, J. et al. (eds.) CRISPR-Cas: A Laboratory Manual, 2016, CSHP.

"Disruption" of the *Ceacam1* gene and/or *Illrap* gene refers to genetic modification of either or both genes such that expression of functional CEACAM1 and/or functional IL1RAP is absent or reduced to 25% or less, 20% or less, 15% or less, 10% or less, or 5% or less, compared to a mouse of the same background prior in which the corresponding gene has not been disrupted. In some embodiments, the genetically modified mice include an inactivated *Ceacam1* allele or an inactivated *Illrap* allele. An inactivated allele may be a loss-of function allele or a null allele. A loss-of-function mutation, as is known in the art, results in a gene product with little or no function. By comparison, a null mutation results in a gene product with no function.

Any of various methods can be used to disrupt the *Ceacam1* gene and/or *Illrap* gene to produce a genetically modified immunodeficient non-human animal whose genome includes a disruption of the *Ceacam1* gene and/or *Illrap* gene. The *Ceacam1* gene and/or *Illrap* gene is disrupted in the genome of genetically modified animals according to standard methods of genetic engineering such as, but not limited to, nuclease techniques, chemical mutagenesis, irradiation, homologous recombination and transgenic expression of antisense RNA. Such techniques are well-known in the art and further include, but are not limited to, pronuclear microinjection and transformation of embryonic stem cells.

Methods for generating genetically modified animals whose genome includes a disrupted gene that can be used include, but are not limited to, those described in J. P. Sundberg and T. Ichiki, Eds., Genetically Engineered Mice Handbook, CRC Press; 2006; M. H. Hofker and J. van Deursen, Eds., Transgenic Mouse Methods and Protocols, Humana Press, 2002; A. L. Joyner, Gene Targeting: A Practical Approach, Oxford University Press, 2000; Manipulating the Mouse Embryo: A Laboratory Manual, 3rd edition, Cold Spring Harbor Laboratory Press; Dec. 15, 2002, ISBN-10: 0879695919; Kursad Turksen (Ed.), Embryonic stem cells: methods and protocols in Methods Mol Biol. 2002; 185, Humana Press; Current Protocols in Stem Cell Biology, ISBN: 978047015180; Meyer et al. PNAS USA, vol. 107 (34), 15022-15026.

Methods of assessing a genetically modified non-human animal to determine whether the *Ceacam1* gene and/or *Illrap* gene is disrupted such that the non-human animal lacks the capacity to express either or both the *Ceacam1* gene and/or *Illrap* gene are well-known and include standard techniques such as nucleic acid assays, spectrometric assays, immunoassays and functional assays.

Assays for assessment of functional CEACAM1 and/or IL1RAP in an animal having a putative disruption of the *Ceacam1* gene and/or *Il1rap* gene can be performed.

A brief description of several genomic editing techniques is described herein.

### Nuclease Techniques for Genetic Modification of Mice

A genetic modification method, such as but not limited to, a nuclease genetic editing technique, can be used to introduce a desired DNA sequence into the genome at a predetermined target site, such as methods using a homing endonuclease, integrase, meganuclease, transposon, nuclease-mediated process using a zinc finger nuclease (ZFN), a Transcription Activator-Like (TAL), a Clustered Regularly Interspaced Short Palindromic Repeats (CRISPR)-Cas, or Drosophila Recombination-Associated Protein (DRAP). Briefly, a genetic modification method that can be used includes introducing into an ES cell, iPS cell, somatic cell, fertilized oocyte or embryo, RNA molecules encoding a targeted TALEN, ZFN, CRISPR or DRAP and at least one oligonucleotide, then selecting for an ES cell, iPS cell, somatic cell, fertilized oocyte or embryo with the desired genetic modification.

For example, a desired nucleic acid sequence can be introduced into the genome of a mouse at a predetermined target site by a nuclease technique, such as, but not limited to, CRISPR methodology, TAL (transcription activator-like) Effector methodology, Zinc Finger-Mediated Genome Editing or DRAP to produce a genetically modified mouse provided according to embodiments of the present disclosure whose genome includes a nucleic acid encoding APOE4 p operably linked to a promoter, wherein the animal expresses the encoded APOE4 p and whose genome includes a nucleic acid encoding TREM2*R47H p operably linked to a promoter, wherein the animal expresses the encoded TREM2*R47H p.

As used herein, the terms "target site" and "target sequence" in the context of a nuclease genetic editing technique refer to a nucleic acid sequence that defines a portion of a chromosomal sequence to be edited and to which a nuclease is engineered to recognize and bind, provided sufficient conditions for binding exist.

### CRISPR-Cas System

CRISPRs (Clustered Regularly Interspaced Short Palindromic Repeats) are loci containing multiple short direct repeats that are found in the genomes of approximately 40% of sequenced bacteria and 90% of sequenced archaea and confer resistance to foreign DNA elements, see Horvath, 2010, Science, 327: 167-170; Barrangou et al, 2007, Science, 315: 1709-1712; and Makarova et al, 2011, Nature Reviews Microbiology. 9: 467-477.

CRISPRs range in size from 24 to 48 base pairs. They usually show some dyad symmetry, implying the formation of a secondary structure such as a hairpin, but are not truly palindromic. CRISPRs are separated by spacers of similar length.

The CRISPR-associated (cas) genes are often associated with CRISPR repeat-spacer arrays. More than forty different Cas protein families have been described (Haft et al. 2005, PLoS Comput Biol. 1 (6): e60). Particular combinations of cas genes and repeat structures have been used to define 8 CRISPR subtypes, some of which are associated with an additional gene module encoding repeat-associated mysterious proteins (RAMPs).

There are diverse CRISPR systems in different organisms, and one of the simplest is the type II CRISPR system from *Streptococcus pyogenes:* only a single gene encoding the Cas9 protein and two RNAs, a mature CRISPR RNA (crRNA) and a partially complementary trans-acting RNA (tracrRNA), are necessary and sufficient for RNA-guided silencing of foreign DNAs (Gasiunas et al, 2012, PNAS 109: E2579-E2586; Jinek et al, 2012, Science 337: 816-821). Maturation of crRNA requires tracrRNA and RNase III (Deltcheva et al, 2011, Nature 471: 602-607). However, this requirement can be bypassed by using an engineered small guide RNA (sgRNA) containing a designed hairpin that mimics the tracrRNA-crRNA complex (Jinek et al., 2012, Science 337: 816-821). Base pairing between the sgRNA and target DNA causes double-strand breaks (DSBs) due to the endonuclease activity of Cas9. Binding specificity is determined by both sgRNA-DNA base pairing and a short DNA motif (protospacer adjacent motif [PAM] sequence: NGG) juxtaposed to the DNA complementary region (Marraffini & Sontheimer, 2010, Nature Reviews Genetics, 11: 181-190). For example, the CRISPR system requires a minimal set of two molecules, the Cas9 protein and the sgRNA, and therefore can be used as a host-independent gene-targeting platform. The Cas9/CRISPR can be harnessed for site-selective RNA-guided genome editing, such as targeting insertion see for example, Carroll, 2012, Molecular Therapy 20: 1658-1660; Chang et al, 2013, Cell Research 23: 465-472; Cho et al, 2013, Nature Biotechnol 31: 230-232; Cong et al, 2013, Science 339: 819-823; Hwang et al, 2013, Nature Biotechnol 31: 227-229; Jiang et al, 2013, Nature Biotechnol 31: 233-239; Mali et al, 2013, Science 339: 823-826; Qi et al, 2013, Cell 152: 1173-1183; Shen et al, 2013, Cell Research 23: 720-723; and Wang et al, 2013, Cell 153: 910-918). In particular, Wang et al. 2013, Cell 153: 910-918 describe targeted insertion using the CRISPR/Cas9 system combined with oligonucleotides.

### TAL (transcription activator-like) Effectors

Transcription activator-like (TAL) effectors or TALE (transcription activator-like effector) are derived from a plant pathogenic bacteria genus, Xanthomonas, and these proteins mimic plant transcriptional activators and manipulate the plant transcript, see Kay et al., 2007, Science, 318:648-651.

TAL effectors contain a centralized domain of tandem repeats, each repeat containing approximately 34 amino acids, which are key to the DNA binding specificity of these proteins. In addition, they contain a nuclear localization sequence and an acidic transcriptional activation domain, for a review see Schornack et al 2006, J. Plant Physiol., 163(3): 256-272; Scholze and Boch, 2011, Curr Opin Microbiol, 14:47-53.

Specificity of TAL effectors depends on the sequences found in the tandem repeats. The repeated sequence includes approximately 102 bp and the repeats are typically 91-100% homologous with each other (Bonas et al, 1989, Mol Gen Genet 218: 127-136). Polymorphism of the repeats is usually located at positions 12 and 13 and there appears to be a one-to-one correspondence between the identity of the hypervariable diresidues at positions 12 and 13 with the identity of the contiguous nucleotides in the TAL-effector's target sequence, see Moscou and Bogdanove 2009, Science 326: 1501; and Boch et al. 2009, Science 326:1509-1512. The two hypervariable residues are known as repeat variable diresidues (RVDs), whereby one RVD recognizes one nucleotide of DNA sequence and ensures that the DNA binding domain of each TAL-effector can target large recognition sites with high precision (15 - 30nt). Experimentally, the code for DNA recognition of these TAL-effectors has been determined such that an HD sequence at positions 12 and 13 leads to a binding to cytosine (C), NG binds to T, NI to A, C, G or T, NN binds to A or G, and IG binds to T. These DNA binding repeats have been assembled into proteins with new combinations and numbers of repeats, to make artificial transcription factors that are able to interact with new sequences and activate the expression of a reporter gene in plant cells (Boch et al. 2009, Science 326:1509-1512). These DNA binding domains have been shown to have general applicability in the field of targeted genomic editing or targeted gene regulation in all cell types, see Gaj et al., Trends in Biotechnol, 2013, 31(7):397-405. Moreover, engineered TAL effectors have been shown to function in association with exogenous functional protein effector domains such as a nuclease, not naturally found in natural Xanthomonas TAL-effect or proteins in mammalian cells. TAL nucleases (TALNs or TALENs) can be constructed by combining TALs with a nuclease, e.g. FokI nuclease domain at the N-terminus or C-terminus, Kim et al. 1996, PNAS 93:1156-1160; Christian et al. 2010, Genetics 186:757-761; Li et al., 2011, Nucleic Acids Res 39: 6315-6325; and Miller et al., 2011, Nat Biotechnol 29: 143-148. The functionality of TALENs to cause deletions by NHEJ has been shown in rat, mouse, zebrafish, Xenopus, medaka, rat and human cells, Ansai et al., 2013, Genetics, 193: 739-749; Carlson et al., 2012, PNAS, 109: 17382-17387; Hockemeyer et al., 2011, Nature Biotechnol., 29: 731-734; Lei et al, 2012, PNAS, 109: 17484-17489; Moore et al., 2012, PLoS ONE, 7: e37877; Stroud et al., 2013, J. Biol. Chem., 288: 1685-1690; Sung et al, 2013, Nature Biotechnol 31: 23-24; Wefers et al., 2013, PNAS 110: 3782-3787.

For TALEN, methods of making such are further described in the US Pat. Nos. 8,420,782, 8,450,471, 8,450,107, 8,440,432 and 8,440,431, and US patent application publications 2013/0137161 and 2013/0137174.

Other useful endonucleases may include, for example, HhaI, HindIII, NotI, BbvCI, EcoRI, Bg/I, and AlwI. The fact that some endonucleases (e.g., FokI) only function as dimers can be capitalized upon to enhance the target specificity of the TAL effector. For example, in some cases each FokI monomer can be fused to a TAL effector sequence that recognizes a different DNA target sequence, and only when the two recognition sites are in close proximity do the inactive monomers come together to create a functional enzyme. By requiring DNA binding to activate the nuclease, a highly site-specific restriction enzyme can be created.

In some embodiments, the TALEN may further include a nuclear localization signal or sequence (NLS). A NLS is an amino acid sequence that facilitates targeting the TALEN nuclease protein into the nucleus to introduce a double stranded break at the target sequence in the chromosome.

Nuclear localization signals are known in the art, see, for example, Makkerh et al. 1996, Curr Biol. 6:1025-1027. NLS include the sequence PKKKRKV (SEQ ID NO:16) from SV40 Large T-antigen, Kalderon 1984, Cell, 39: 499-509; RPAATKKAGQAKKK (SEQ ID NO:17) from nucleoplasmin, Dingwallet et al., 1988, J Cell Biol., 107, 841-9. Further examples are described in McLane and Corbett 2009, IUBMB Life, 61, 697-70; Dopie et al. 2012, PNAS, 109, E544-E552.

The cleavage domain may be obtained from any endonuclease or exonuclease. Non-limiting examples of endonucleases from which a cleavage domain may be derived include, but are not limited to, restriction endonucleases and homing endonucleases. See, for example, 2002-2003 Catalog, New England Biolabs, Beverly, Mass.; and Belfort et al. (1997) Nucleic Acids Res. 25:3379-3388. Additional enzymes that cleave DNA are known, e.g., SI Nuclease; mung bean nuclease; pancreatic DNase I; micrococcal nuclease; yeast HO endonuclease. See also Linn et al. (eds.) Nucleases, Cold Spring Harbor Laboratory Press, 1993. One or more of these enzymes, or functional fragments thereof, may be used as a source of cleavage domains.

### Zinc Finger-Mediated Genome Editing

The use of zinc finger nucleases (ZFN) for gene editing, such as for targeted insertion via a homology-directed repair process, has been well established. For example, see Carbery et al., 2010, Genetics, 186: 451-459; Cui et al., 2011, Nature Biotechnol., 29: 64-68; Hauschild et al., 2011, PNAS, 108: 12013-12017; Orlando et al., 2010, Nucleic Acids Res., 38: e152-e152; and Porteus & Carroll, 2005, Nature Biotechnology, 23: 967-973.

Components of the ZFN-mediated process include a zinc finger nuclease with a DNA binding domain and a cleavage domain. Such are described for example in Beerli et al. (2002) Nature Biotechnol., 20:135-141; Pabo et al. (2001) Ann. Rev. Biochem., 70:313-340; Isalan et al. (2001) Nature Biotechnol. 19:656-660; Segal et al. (2001) Curr Opin. Biotechnol., 12:632-637; and Choo et al. (2000) Curr Opin. Struct. Biol., 10:411-416; and U.S. Pat. Nos. 6,453,242 and 6,534,261. Methods to design and select a zinc finger binding domain to a target sequence are known in the art, see for example Sera, et al., Biochemistry 2002,41,7074-7081; U.S. Pat. Nos. 6,607,882; 6,534,261 and 6,453,242.

In some embodiments, the zinc finger nuclease may further include a nuclear localization signal or sequence (NLS). A NLS is an amino acid sequence that facilitates targeting the zinc finger nuclease protein into the nucleus to introduce a double stranded break at the target sequence in the chromosome. Nuclear localization signals are known in the art. See, for example, Makkerh et al. (1996) Current Biology 6:1025-1027.

The cleavage domain may be obtained from any endonuclease or exonuclease. Non-limiting examples of endonucleases from which a cleavage domain may be derived include, but are not limited to, restriction endonucleases and homing endonucleases. See, for example, 2002-2003 Catalog, New England Biolabs, Beverly, Mass.; and Belfort et al. (1997) Nucleic Acids Res. 25:3379-3388. Additional enzymes that cleave DNA are known (e.g., SI Nuclease; mung bean nuclease; pancreatic DNase I; micrococcal nuclease; yeast HO endonuclease). See also Linn et al. (eds.) Nucleases, Cold Spring Harbor Laboratory Press, 1993. One or more of these enzymes (or functional fragments thereof) may be used as a source of cleavage domains. A cleavage domain also may be derived from an enzyme or portion thereof, as described above, that requires dimerization for cleavage activity.

Two zinc finger nucleases may be required for cleavage, as each nuclease includes a monomer of the active enzyme dimer. Alternatively, a single zinc finger nuclease may include both monomers to create an active enzyme dimer. Restriction endonucleases (restriction enzymes) are present in many species and are capable of sequence-specific binding to DNA (at a recognition site), and cleaving DNA at or near the site of binding. Certain restriction enzymes (e.g., Type IIS) cleave DNA at sites removed from the recognition site and have separable binding and cleavage domains. For example, the Type IIS enzyme FokI catalyzes double stranded cleavage of DNA, at 9 nucleotides from its recognition site on one strand and 13 nucleotides from its recognition site on the other. See, for example, U.S. Pat. Nos. 5,356,802; 5,436,150 and 5,487,994; as well as Li et al. (1992) PNAS 89:4275-4279; Li et al. (1993) PNAS 90:2764-2768; Kim et al. (1994) PNAS 91:883-887; Kim et al. (1994) J. Biol. Chem. 269:31, 978-31, 982. Thus, a zinc finger nuclease may include the cleavage domain from at least one Type IIS restriction enzyme and one or more zinc finger binding domains, which may or may not be engineered. Exemplary Type IIS restriction enzymes are described for example in International Publication WO 07/014275. Additional restriction enzymes also contain separable binding and cleavage domains, and these also are contemplated by the present disclosure. See, for example, Roberts et al. (2003) Nucleic Acids Res. 31: 418-420. An exemplary Type IIS restriction enzyme, whose cleavage domain is separable from the binding domain, is FokI. This particular enzyme is active as a dimer (Bitinaite et al. 1998, PNAS 95: 10,570-10,575). Accordingly, for the purposes of the present disclosure, the portion of the FokI enzyme used in a zinc finger nuclease is considered a cleavage monomer. Thus, for targeted double stranded cleavage using a FokI cleavage domain, two zinc finger nucleases, each including a FokI cleavage monomer, may be used to reconstitute an active enzyme dimer. Alternatively, a single polypeptide molecule containing a zinc finger binding domain and two FokI cleavage monomers may also be used. In certain embodiments, the cleavage domain may include one or more engineered cleavage monomers that minimize or prevent homodimerization, as described, for example, in U.S. Patent Publication Nos. 2005/0064474, 2006/0188987, and 2008/0131962. By way of non-limiting example, amino acid residues at positions 446, 447, 479, 483, 484, 486, 487, 490, 491, 496, 498, 499, 500, 531, 534, 537 and 538 of FokI are all targets for influencing dimerization of the FokI cleavage half-domains. Exemplary engineered cleavage monomers of FokI that form obligate heterodimers include a pair in which a first cleavage monomer includes mutations at amino acid residue positions 490 and 538 of FokI and a second cleavage monomer that includes mutations at amino-acid residue positions 486 and 499. Thus, in one embodiment, a mutation at amino acid position 490 replaces Glu (E) with Lys (K); a mutation at amino acid residue 538 replaces Ile (I) with Lys (K); a mutation at amino acid residue 486 replaces Gln (Q) with Glu (E); and a mutation at position 499 replaces Ile (I) with Lys (K). Specifically, the engineered cleavage monomers may be prepared by mutating positions 490 from E to K and 538 from I to K in one cleavage monomer to produce an engineered cleavage monomer designated "E490K:I538K" and by mutating positions 486 from Q to E and 499 from I to L in another cleavage monomer to produce an engineered cleavage monomer designated "Q486E:I499L." The above described engineered cleavage monomers are obligate heterodimer mutants in which aberrant cleavage is minimized or abolished. Engineered cleavage monomers may be prepared using a suitable method, for example, by site-directed mutagenesis of wild-type cleavage monomers (FokI) as described in U.S. Patent Publication No. 2005/0064474.

The zinc finger nuclease described above may be engineered to introduce a double stranded break at the targeted site of integration. The double stranded break may be at the targeted site of integration, or it may be up to 1, 2, 3, 4, 5, 10, 15, 20, 25, 30, 35, 40, 45, 50, 100 or 1000 nucleotides away from the site of integration. In some embodiments, the double stranded break may be up to 1, 2, 3, 4, 5, 10, 15, or 20 nucleotides away from the site of integration. In other embodiments, the double stranded break may be up to 10, 15, 20, 25, 30, 35, 40, 45 or 50 nucleotides away from the site of integration. In yet other embodiments, the double stranded break may be up to 50, 100 or 1000 nucleotides away from the site of integration.

The DRAP technology has been described in US Pat. Nos. 6,534,643, 6,858,716 and 6,830,910 and Watt *et al.,* 2006.

Optionally, a nucleic acid sequence encoding a protein can be inserted at a random target site in the genome so that the genome includes a nucleic acid encoding the protein. Typically, for random insertion, the nucleic acid sequence also contains a promoter to drive expression of the inserted nucleic acid.

In a further option, a nucleic acid encoding the desired protein, APOE4 p, TREM2*R47H p or both APOE4 p and TREM2*R47H p, or one or more of: ABCA7*A1541G p, APP* p, PLCG2*M28L p, and MTHFR*A262V p, or a variant of any thereof, is inserted into a predetermined target site in the genome other than the corresponding Apoe4 gene, the Trem2 gene, the Abca7 gene, the App gene, or the PLCG2 gene.

For example, a nucleic acid encoding the desired protein, APOE4 p, TREM2*R47H p or both APOE4 p and TREM2*R47H p, or one or more of: ABCA7*A1541G p, APP* p, PLCG2*M28L p, and MTHFR*A262V p, or a variant of any thereof, is inserted into a predetermined target site in the genome known to result in reliable expression, such as the *Hprt* or the *Rosa26* locus.

According to aspects, for genomic editing at a predetermined target site, a targeting construct is made using recombinant DNA techniques and includes 5' and 3' sequences which are homologous to the targeted endogenous gene in the cell. The targeting construct further includes a selectable marker such as neomycin phosphotransferase, hygromycin or puromycin, a nucleic acid encoding the desired protein, APOE4 p, TREM2*R47H p or both APOE4 p and TREM2*R47H p, or one or more of: ABCA7*A1541G p, APP* p, PLCG2*M28L p, and MTHFR*A262V p, or a variant of any thereof, and optionally a polyadenylation signal. To ensure correct transcription and translation of the nucleic acid encoding the desired protein, the nucleic acid encoding the desired protein is either in frame with the endogenous gene locus, or a splice acceptor site and internal ribosome entry site (IRES) sequences can be included.

Such a targeting construct is transfected into a desired cell type, such as but not limited to, stem cells and the cells are screened to detect the correct genomic editing event using PCR, Southern blot or sequencing analysis. Cells with the correct genomic editing event can be further analyzed for expression of the encoded protein by protein analysis, such as ELISA or Western blot analysis. If desired, the nucleic acid encoding the selectable marker can be configured to be removed by treating the stem cells with a recombinase such as Cre recombinase or Flippase (Flp). After recombinase treatment, the cells are analyzed for the presence of the nucleic acid encoding the desired protein.

Cells with the correct genomic editing event are selected and injected into preimplantation embryos as described above. Chimeric males are selected for breeding. Offspring can be analyzed for transmission of the ES cell genome by coat color and genetic analysis, such as PCR, Southern blot or sequencing and can be tested for expression of the desired protein, such as by protein analysis (Western blot, ELISA) or other functional assays. Offspring expressing the correct proteins are intercrossed to create mice homozygous for the genetic modification(s).

Generation of a genetically modified mouse expressing APOE4 p and TREM2*R47H p, and one or more of: ABCA7*A1541G p, APP* p, PLCG2*M28L p, and MTHFR*A262V p, or a variant of any thereof, and/or which does not express functional CEACAM1 and/or IL1RAP may include injection or transfection of appropriate nucleic acids, such as one or more one or more nucleic acids encoding a desired protein and/or one or more expression constructs, such as an expression construct encoding a protein or RNA (such as cas9 or a guide RNA for use in CRISPR), into a preimplantation embryo or stem cells, such as embryonic stem (ES) cells or induced pluripotent stem (iPS) cells.

The terms "expression construct" and "expression cassette" are used herein to refer to a double-stranded recombinant DNA molecule containing a desired nucleic acid coding sequence and containing one or more regulatory elements necessary or desirable for the expression of the operably linked coding sequence.

The term "regulatory element" as used herein refers to a nucleotide sequence which controls some aspect of the expression of nucleic acid sequences. Exemplary regulatory elements illustratively include an enhancer, an internal ribosome entry site (IRES), an intron; an origin of replication, a polyadenylation signal (pA), a promoter, a transcription termination sequence, and an upstream regulatory domain, which contribute to the replication, transcription, post-transcriptional processing of a nucleic acid sequence. Those of ordinary skill in the art are capable of selecting and using these and other regulatory elements in an expression construct with no more than routine experimentation. Expression constructs can be generated recombinantly or synthetically using well-known methodology.

The term "operably linked" as used herein refers to a nucleic acid in functional relationship with a second nucleic acid.

A regulatory element is included in an expression cassette is a promoter in particular embodiments. The term "promoter" as used herein refers to a DNA sequence operably linked to a nucleic acid sequence to be transcribed such as a nucleic acid sequence encoding a desired molecule. A promoter is generally positioned upstream of a nucleic acid sequence to be transcribed and provides a site for specific binding by RNA polymerase and other transcription factors. In specific embodiments, a promoter is generally positioned upstream of the nucleic acid sequence transcribed to produce the desired molecule, and provides a site for specific binding by RNA polymerase and other transcription factors. An included promoter can be a constitutive promoter or can provide inducible expression; and can provide ubiquitous, tissue-specific or cell-type specific expression.

Ubiquitous promoters that can be included in an expression construct include, but are not limited to, a 3-phosphoglycerate kinase (PGK-1) promoter, a beta-actin promoter, a ROSA26 promoter, a heat shock protein 70 (Hsp70) promoter, an EF-1 alpha gene encoding elongation factor 1 alpha (EF1) promoter, an eukaryotic initiation factor 4A (eIF-4A1) promoter, a chloramphenicol acetyltransferase (CAT) promoter and a CMV (cytomegalovirus) promoter.

These and other promoters are known in the art as exemplified in Abboud, S. L. et al, J. Histochem & Cytochem., 51(7):941-949, 2003; Schorpp et al., Nucl. Acids Res., 24(9):1787-1788, 1996; McBurney, M. W. et al., Devel. Dynamics, 200:278-293, 1994; and Majumder, M. et al., Blood, 87(8):3203-3211, 1996.

In addition to a promoter, one or more enhancer sequences may be included such as, but not limited to, cytomegalovirus (CMV) early enhancer element and an SV40 enhancer element.

Additional included sequences include an intron sequence such as the beta globin intron or a generic intron, a transcription termination sequence, and an mRNA polyadenylation (pA) sequence such as, but not limited to SV40-pA, beta-globin-pA and SCF-pA.

An expression construct may include sequences necessary for amplification in bacterial cells, such as a selection marker (e.g. kanamycin or ampicillin resistance gene) and a replicon.

For methods of DNA injection of an expression construct into a preimplantation embryo, the expression construct is optionally linearized before injection into mouse preimplantation embryos. Preferably the expression construct is injected into fertilized oocytes. Fertilized oocytes are collected from superovulated females the day after mating (0.5 dpc) and injected with the expression construct. The injected oocytes are either cultured overnight or transferred directly into oviducts of 0.5-day p.c. pseudopregnant females.

Methods for superovulation, harvesting of oocytes, expression construct injection and embryo transfer are known in the art and described in Manipulating the Mouse Embryo: A Laboratory Manual, 3rd edition, Cold Spring Harbor Laboratory Press; December 15, 2002, ISBN-10: 0879695919.

Offspring can be tested for the presence of the desired mutation or inserted sequence by DNA analysis, such as PCR, Southern blot or sequencing. Mice which are carrying the desired mutation or inserted sequence can be tested for protein expression such as for example, by ELISA or Western blot analysis.

Alternatively, a nucleic acid or expression construct may be transfected into stem cells (ES cells or iPS cells) using well-known methods, such as electroporation, calcium-phosphate precipitation and lipofection. The cells are screened for the presence of the desired mutation or inserted sequence by DNA analysis, such as PCR, Southern blot or sequencing. Cells with the desired mutation or inserted sequence by can be tested for functional expression by protein analysis, such as for example, by ELISA or Western blot analysis.

Mouse ES cells are grown in media optimized for the particular line. Typically ES media contains 15% fetal bovine serum (FBS) or synthetic or semi-synthetic equivalents, 2 mM glutamine, 1 mM Na pyruvate, 0.1 mM non-essential amino acids, 50 U/ml penicillin and streptomycin, 0.1 mM 2-mercaptoethanol and 1000 U/ml LIF (plus, for some cell lines chemical inhibitors of differentiation) in Dulbecco's Modified Eagle Media (DMEM). A detailed description is known in the art (Tremml et al., 2008, Current Protocols in Stem Cell Biology, Chapter 1:Unit 1C.4. For review of inhibitors of ES cell differentiation, see Buehr, M., et al. (2003). Genesis of embryonic stem cells. Philosophical Transactions of the Royal Society B: Biological Sciences 358, 1397-1402.

Selected cells incorporating the desired mutation or inserted sequence can be injected into preimplantation embryos. For microinjection, ES or iPS cell are rendered to single cells using a mixture of trypsin and EDTA, followed by resuspension in ES media. Groups of single cells are selected using a finely drawn-out glass needle (20-25 micrometer inside diameter) and introduced through the embryo's zona pellucida and into the blastocysts cavity (blastocoel) using an inverted microscope fitted with micromanipulators.

Alternatively, to blastocyst injection, stem cells can be injected into early-stage embryos (e.g. 2-cell, 4-cell, 8-cell, premorula or morula). Injection may be assisted with a laser or piezo pulses drilled opening the zona pellucida. Approximately 9-10 selected stem cells (ES or iPS cells) are injected per blastocysts, or 8-cell stage embryo, 6-9 stem cells per 4-cell stage embryo, and about 6 stem cells per 2-cell stage embryo. Following stem cell introduction, embryos are allowed to recover for a few hours at 37 °C in 5% CO₂, 5% O₂ in nitrogen or cultured overnight before transfer into pseudopregnant recipient females. In a further alternative to stem cell injection, stem cells can be aggregated with morula stage embryos. All these methods are well established and can be used to produce stem cell chimeras. For a more detailed description see Manipulating the Mouse Embryo: A Laboratory Manual, 3rd edition (A. Nagy, M. Gertsenstein, K. Vintersten, R. Behringer, Cold Spring Harbor Laboratory Press; December 15, 2002, ISBN-10: 0879695919, Nagy et al., 1990, Development 110, 815-821; US 7,576,259: Method for making genetic modifications, US7659442, US 7,294,754, and Kraus et al. 2010, Genesis 48, 394-399.

Pseudopregnant embryo recipients are prepared using methods known in the art. Briefly, fertile female mice between 6-8 weeks of age are mated with vasectomized or sterile males to induce a hormonal state conductive to supporting surgically introduced embryos. At 2.5 days post coitum (dpc) up to 15 of the stem cell containing blastocysts are introduced into the uterine horn very near to the uterus-oviduct junction. For early-stage embryos and morula, such embryos are either cultured in vitro into blastocysts or implanted into 0.5 dpc or 1.5 dpc pseudopregnant females according to the embryo stage into the oviduct. Chimeric pups from the implanted embryos are born 16-20 days after the transfer depending on the embryo age at implantation. Chimeric males are selected for breeding. Offspring can be analyzed for transmission of the ES cell genome by coat color and genetic analysis, such as PCR, Southern blot or sequencing. Further the expression of the encoded protein(s) can be analyzed by protein analysis (Western blot, ELISA) or other functional assays.

A genetically modified mouse of the present disclosure may be heterozygous or homozygous for the genetic modifications.

According to examples of the present disclosure, a genetically modified mouse of the present disclosure may be heterozygous or homozygous for the knock-in humanized APOE4 modification wherein the mouse expresses APOE4 p, may be heterozygous or homozygous for the mutated genomic sequence encoding TREM2*R47H p wherein the mouse expresses TREM2*R47H p, and may be heterozygous or homozygous for one or more of: a mutated genomic sequence encoding ABCA7*A1541G p, a mutated genomic sequence encoding APP* p, a mutated genomic sequence encoding PLCG2*M28L p, or a mutated genomic sequence encoding a variant of any thereof.

Homozygous genetically modified mice expressing a first encoded mutated protein can be crossed with homozygous genetically modified mice expressing a second encoded mutated protein to create a congenic strain homozygous for both modifications and expressing both mutated proteins according to embodiments.

Homozygous genetically modified mice expressing a first encoded mutated protein and a second encoded mutated protein can be crossed with homozygous genetically modified mice expressing a third encoded mutated protein to create a congenic strain homozygous for all three modifications and expressing all three mutated proteins according to embodiments.

Similarly, homozygous genetically modified mice expressing a first encoded mutated protein, a second encoded mutated protein and a third encoded mutated protein and/or fourth encoded mutated protein can be crossed with homozygous genetically modified mice expressing a further encoded mutated protein to create a congenic strain homozygous for all of the encoded modifications and expressing all of the mutated proteins according to embodiments.

Homozygous genetically modified mice expressing APOE4 p can be crossed with homozygous genetically modified mice expressing TREM2*R47H p to create a congenic strain homozygous for both modifications and expressing both APOE4 p and TREM2*R47H p according to embodiments.

Homozygous genetically modified mice expressing APOE4 p and TREM2*R47H p can be crossed with homozygous genetically modified mice expressing any one or more of: ABCA7*A1541G p, APP* p, PLCG2*M28L p, MTHFR*A262V p, or a variant of any thereof, or genetically modified such that the *Ceacam1* gene and/or Il1rap gene is disrupted, to create a congenic strain homozygous for the three modifications and expressing APOE4 p, TREM2*R47H p, and ABCA7* A1541G p, APP* p, PLCG2*M28L p, MTHFR*A262V p, or a variant of any thereof, and/or where functional CEACAM1 and/or IL1RAP are not expressed, according to embodiments.

Genetically modified mice of the present disclosure can be any of various strains.

A genetic modification can be introduced into the genome of an isolated mouse embryonic stem (ES) cell, a mouse induced pluripotent stem (iPS) cell, a mouse somatic cell, a fertilized mouse oocyte (zygote) or a mouse embryo in a knock-in strategy to produce a genetically modified mouse of the present disclosure.

Embodiments of the disclosure provide a genetically modified mouse that includes a desired genetic modification in all or substantially all of its cells, as well as a genetically modified mouse that includes a desired genetic modification in some, but not all its cells.

A genetically modified mouse according to aspects of the present disclosure can include one or more additional genetic variants associated with increased risk of late-onset Alzheimer's disease in humans.

### Identification of Treatments and Compounds

Methods for screening for putative treatments for human Alzheimer's disease are provided according to embodiments of the present invention which include: administering a putative treatment for Alzheimer's disease to a genetically modified mouse, wherein the genome of the mouse includes: (1) a DNA sequence encoding human APOE4 protein (APOE4 p) operably linked to a promoter; and (2) a DNA sequence encoding mouse TREM2 protein having a mutation p.R47H (TREM2*R47H p) operably linked to a promoter, wherein the mouse expresses APOE4 p and TREM2*R47H p and a nucleic acid encoding: APP* p operably linked to a promoter, wherein the animal expresses the encoded APP* p, wherein the mouse is characterized by one or more symptoms or signs associated with expression of APOE4 p, TREM2*R47H p and expression of APP* p relevant to non-familial late-onset Alzheimer's disease of the present disclosure; and assessing an effect of the putative treatment on the mouse. Also disclosed herein but not forming part of the present invention are methods for screening for putative treatments for human Alzheimer's disease which include: administering a putative treatment for Alzheimer's disease to a genetically modified mouse, wherein the genome of the mouse includes: (1) a DNA sequence encoding human APOE4 protein (APOE4 p) operably linked to a promoter; and (2) a DNA sequence encoding mouse TREM2 protein having a mutation p.R47H (TREM2*R47H p) operably linked to a promoter, wherein the mouse expresses APOE4 p and TREM2*R47H p and one or more of: (3) a nucleic acid encoding: ABCA7*A1541G p operably linked to a promoter, wherein the animal expresses the encoded ABCA7*A1541G p; (4) a nucleic acid encoding: PLCG2*M28L p operably linked to a promoter, wherein the animal expresses the encoded PLCG2*M28L p; (5) a nucleic acid encoding: MTHFR*A262V p operably linked to a promoter, wherein the animal expresses the encoded MTHFR*A262V p; (6) a disrupted (e.g., inactivated) *Ceacam1* gene such that functional CEACAM1 is not produced in the genetically modified mouse; and (7) a disrupted (e.g., inactivated) Il1rap gene such that functional IL1RAP is not produced in the genetically modified mouse, wherein the mouse is characterized by one or more symptoms or signs associated with expression of APOE4 p, TREM2*R47H p and one or more of: (1) expression of ABCA7*A1541G p; (2) expression of PLCG2*M28L p; (3) expression of MTfWR*A262V; (4) lack of expression of functional CEACAM1; and (5) lack of expression of functional IL1RAP, relevant to non-familial late-onset Alzheimer's disease of the present disclosure; and assessing an effect of the putative treatment on the mouse.

Methods for screening for putative treatments for human Alzheimer's disease are provided according to embodiments of the present invention which include: administering a putative treatment for Alzheimer's disease to a mouse whose genome includes: (1) a DNA sequence encoding human APOE4 protein (APOE4 p) operably linked to a promoter; and (2) a DNA sequence encoding mouse TREM2 protein having a mutation p.R47H (TREM2*R47H p) operably linked to a promoter, wherein the mouse expresses APOE4 p and TREM2*R47H p and a nucleic acid encoding: APP* p operably linked to a promoter, wherein the animal expresses the encoded APP* p; and assessing an effect of the putative treatment on the mouse. Also disclosed herein but not forming part of the claimed invention are methods for screening for putative treatments for human Alzheimer's disease which include: administering a putative treatment for Alzheimer's disease to a mouse whose genome includes: (1) a DNA sequence encoding human APOE4 protein (APOE4 p) operably linked to a promoter; and (2) a DNA sequence encoding mouse TREM2 protein having a mutation p.R47H (TREM2*R47H p) operably linked to a promoter, wherein the mouse expresses APOE4 p and TREM2*R47H p and one or more of: (3) a nucleic acid encoding: ABCA7* A1541G p operably linked to a promoter, wherein the animal expresses the encoded ABCA7* A1541G p; (4) a nucleic acid encoding: PLCG2*M28L p operably linked to a promoter, wherein the animal expresses the encoded PLCG2*M28L p; (5) a nucleic acid encoding: MTHFR*A262V p operably linked to a promoter, wherein the animal expresses the encoded MTHFR*A262V p; (6) a disrupted (e.g., inactivated) *Ceacam1* gene such that functional CEACAM1 is not produced in the genetically modified mouse; and (7) a disrupted (e.g., inactivated) Il1rap gene such that functional IL1RAP is not produced in the genetically modified mouse; and assessing an effect of the putative treatment on the mouse.

Methods for screening for a compound for use in the treatment of Alzheimer's disease, are provided according to aspects of the present invention which include administering a compound to a genetically modified mouse of the present disclosure; and assessing an effect of the compound in the treatment of one or more symptoms or signs associated with expression of human APOE4 p and mouse TREM2*R47H p and APP* p, wherein the one or more symptoms or signs is relevant to non-familial late-onset Alzheimer's disease. Also disclosed herein but not forming part of the claimed invention are methods for screening for a compound for use in the treatment of Alzheimer's disease, which include administering a compound to a genetically modified mouse of the present disclosure; and assessing an effect of the compound in the treatment of one or more symptoms or signs associated with expression of human APOE4 p and mouse TREM2*R47H p and one or more of: ABCA7*A1541G p, PLCG2*M28L p, MTHFR*A262V p, a disrupted *Ceacam1* gene such that functional CEACAM1 is not produced in the genetically modified mouse; and a disrupted Il1rap gene such that functional IL1RAP is not produced in the genetically modified mouse, wherein the one or more symptoms or signs is relevant to non-familial late-onset Alzheimer's disease. Assessing an effect of the compound in the treatment of one or more symptoms or signs associated with expression of human APOE4 p and mouse TREM2*R47H p and one or more of: ABCA7*A1541G p, APP* p, PLCG2*M28L p, MTHFR*A262V p a disrupted *Ceacam1* gene such that functional CEACAM1 is not produced in the genetically modified mouse; and a disrupted Il1rap gene such that functional IL1RAP is not produced in the genetically modified mouse wherein the symptoms or signs are relevant to non-familial late-onset Alzheimer's disease preferably includes comparing the result of the assessment with a suitable control, such as, but not limited to, the effect of the compound on a control, such as an APOE3-expressing mouse or a wild-type mouse, such as a mouse of the same or similar background without one or more of these mutations, e.g., a mouse carrying the *Apoe* gene.

Such signs and symptoms include, but are not limited to, any one or more of: 1) presence of significantly more microglia in the brain of a genetically modified mouse of the present disclosure compared to a relevant control, such as a mouse of the same or similar background expressing one or more of: non-mutant *APOE3, Trem2, Abca7, App, Plcg2, Mthfr, Ceacam1,* or *Illrap;* or a wild-type control mouse of the same or similar background; 2) presence of significantly more amyloid plaques in the brain of a genetically modified mouse of the present disclosure compared to a relevant control, such as a mouse of the same or similar background expressing one or more of: non-mutant *APOE3, Trem2, Abca7, App, Plcg2, Mthfr, Ceacam1,* or *Illrap;* or a wild-type control mouse of the same or similar background; 3) presence of significantly more tau aggregates in the brain of a genetically modified mouse of the present disclosure compared to a relevant control, such as a mouse of the same or similar background expressing one or more of: non-mutant *APOE3, Trem2, Abca7, App, Plcg2, Mthfr, Ceacam1,* or *Illrap;* or a wild-type control mouse of the same or similar background; 4) presence of significantly more inflammation in the brain of a genetically modified mouse of the present disclosure compared to a relevant control, such as a mouse of the same or similar background expressing one or more of: non-mutant APOE3, *Trem2, Abca7, App, Plcg2, Mthfr, Ceacam1,* or *Illrap;* or a wild-type control mouse of the same or similar background; 5) presence of significantly more synaptic and/or neuronal loss in the brain of a genetically modified mouse of the present disclosure compared to a relevant control, such as a mouse of the same or similar background expressing one or more of: non-mutant *APOE3, Trem2, Abca7, App, Plcg2, Mthfr, Ceacam1,* or *Illrap;* or a wild-type control mouse of the same or similar background; 6) presence of significantly more cognitive deficit in the brain of a genetically modified mouse of the present disclosure compared to a relevant control, such as a mouse of the same or similar background expressing one or more of: non-mutant *APOE3, Trem2, Abca7, App, Plcg2, Mthfr, Ceacam1,* or *Illrap;* or a wild-type control mouse of the same or similar background; 7) presence of significantly more indications of frailty an aging genetically modified mouse of the present disclosure compared to a relevant control, such as a mouse of the same or similar background expressing one or more of: non-mutant *APOE3, Trem2, Abca7, App, Plcg2, Mthfr, Ceacam1,* or *Illrap;* or a wild-type control mouse of the same or similar background; 8) presence of significantly more blood flow deficit in the brain of a genetically modified mouse of the present disclosure compared to a relevant control, such as a mouse of the same or similar background expressing one or more of: non-mutant *APOE3, Trem2, Abca7, App, Plcg2, Mthfr, Ceacam1,* or *Illrap;* or a wild-type control mouse of the same or similar background; 9) a significant difference in presence, level, and/or function of one or more biomarkers of non-familial late-onset Alzheimer's disease in blood, serum, or tissue of a genetically modified mouse of the present disclosure compared to a relevant control, such as a mouse of the same or similar background expressing one or more of: non-mutant *APOE3, Trem2, Abca7, App, Plcg2, Mthfr, Ceacam1,* or *Illrap;* or a wild-type control mouse of the same or similar background; 10) cerebrovascular leakage in a genetically modified mouse of the present disclosure compared to a relevant control, such as a mouse of the same or similar background expressing one or more of: non-mutant *APOE3, Trem2, Abca7, App, Plcg2, Mthfr, Ceacam1,* or *Illrap;* or a wild-type control mouse of the same or similar background; and 11) levels of one or more blood lipoproteins, such as high density lipoprotein, and low density lipoprotein, and/or cholesterol, in a genetically modified mouse of the present disclosure compared to a relevant control, such as a mouse of the same or similar background expressing one or more of: non-mutant *APOE3, Trem2, Abca7, App, Plcg2, Mthfr, Ceacam1,* or *Illrap;* or a wild-type control mouse of the same or similar background.

According to aspects of the present invention, methods for screening for putative treatments for human Alzheimer's disease are provided wherein assessing an effect of a compound includes comparing the effect of the compound on the genetically modified mouse, wherein the genome of the mouse includes: (1) a DNA sequence encoding human APOE4 protein (APOE4 p) operably linked to a promoter; and (2) a DNA sequence encoding mouse TREM2 protein having a mutation p.R47H (TREM2*R47H p) operably linked to a promoter, wherein the mouse expresses APOE4 p and TREM2*R47H p, and wherein the mouse includes a nucleic acid encoding: APP* p operably linked to a promoter, wherein the animal expresses the encoded APP* p, and wherein the mouse is characterized by one or more symptoms or signs associated with expression of APOE4 p and TREM2*R47H p and APP, relevant to non-familial late-onset Alzheimer's disease of the present disclosure, with a control. Also disclosed herein but not forming part of the claimed invention are methods for screening for putative treatments for human Alzheimer's disease wherein assessing an effect of a compound includes comparing the effect of the compound on the genetically modified mouse, wherein the genome of the mouse includes: (1) a DNA sequence encoding human APOE4 protein (APOE4 p) operably linked to a promoter; and (2) a DNA sequence encoding mouse TREM2 protein having a mutation p.R47H (TREM2*R47H p) operably linked to a promoter, wherein the mouse expresses APOE4 p and TREM2*R47H p, and wherein the mouse includes one or more of the following: (1) a nucleic acid encoding: ABCA7* A1541G p operably linked to a promoter, wherein the animal expresses the encoded ABCA7*A1541G p; (2) a nucleic acid encoding: PLCG2*M28L p operably linked to a promoter, wherein the animal expresses the encoded PLCG2*M28L p; (3) a nucleic acid encoding: MTHFR*A262V p operably linked to a promoter, wherein the animal expresses the encoded MTHFR*A262V p; (4) a disrupted *Ceacam1* gene such that functional CEACAM1 is not produced in the genetically modified mouse; and (5) a disrupted Il1rap gene such that functional IL1RAP is not produced in the genetically modified mouse; and wherein the mouse is characterized by one or more symptoms or signs associated with expression of APOE4 p and TREM2*R47H p and one or more of the following: ABCA7, PLCG2, MTHFR, CEACAM1, IL1RAP, relevant to non-familial late-onset Alzheimer's disease of the present disclosure, with a control.

A suitable control includes, for example, administering the compound to a control mouse which does not express human APOE4 p and mouse TREM2*R47H p and does not include a relevant one or more of the following: (1) a nucleic acid encoding: ABCA7* A1541G p operably linked to a promoter, wherein the animal expresses the encoded ABCA7* A1541G p; (2) a nucleic acid encoding: APP* p operably linked to a promoter, wherein the animal expresses the encoded APP* p; (3) a nucleic acid encoding: PLCG2*M28L p operably linked to a promoter, wherein the animal expresses the encoded PLCG2*M28L p; (4) a nucleic acid encoding: MTHFR*A262V p operably linked to a promoter, wherein the animal expresses the encoded MTHFR*A262V p; (5) a disrupted *Ceacam1* gene such that functional CEACAM1 is not produced in the genetically modified mouse; and (6) a disrupted Il1rap gene such that functional IL1RAP is not produced in the genetically modified mouse; and assessing an effect of the compound on the control mouse. A suitable control includes, for example, administering the compound to an APOE3-expressing mouse or wild-type control mouse. A wild-type control mouse can be any mouse which does not express human APOE4 p and mouse TREM2*R47H p; and a relevant one or more of the following: (1) a nucleic acid encoding: ABCA7* A1541G p operably linked to a promoter, wherein the animal expresses the encoded ABCA7*A1541G p; (2) a nucleic acid encoding: APP* p operably linked to a promoter, wherein the animal expresses the encoded APP* p; (3) a nucleic acid encoding: PLCG2*M28L p operably linked to a promoter, wherein the animal expresses the encoded PLCG2*M28L p; (4) a nucleic acid encoding: MTHFR*A262V p operably linked to a promoter, wherein the animal expresses the encoded MTHFR*A262V p; (5) a disrupted *Ceacam1* gene such that functional CEACAM1 is not produced in the genetically modified mouse; and (6) a disrupted Il1rap gene such that functional IL1RAP is not produced in the genetically modified mouse. A suitable control includes, for example, administering the compound to a wild-type C57BL/6J mouse; and assessing an effect of the compound on the wild-type C57BL/6J mouse.

Symptoms or signs associated with expression of human APOE4 p and mouse TREM2*R47H p relevant to non-familial late-onset Alzheimer's disease can be assessed by methods well-known in the art including, but not limited to, immunoassay, nucleic acid assay, histochemical staining, cognitive assays, *in vivo* imaging, physical assessment of the animals, cerebrovascular leakage assessment, and morphological assessment of tissues and/or cells.

Immunoassays that can be used are well-known in the art and include, but are not limited to, enzyme-linked immunosorbent assay (ELISA) such as but not limited to, antigen capture ELISA, indirect ELISA, fixed cell ELISA; immunochromatography; antigen capture; flow cytometry; immunoblot; immunoprecipitation; immunodiffusion; competitive immunoassays, immunocytochemistry; radioimmunoassay; and combinations of any of these. Generalized details of immunoassays are described in standard references, illustratively including Wild, D., The Immunoassay Handbook, 3rd Ed., Elsevier Science, 2005; Gosling, J. P., Imunoassays: A Practical Approach, Practical Approach Series, Oxford University Press, 2005; E.Harlow and D. Lane, Antibodies: A Laboratory Manual, Cold Spring Harbor Laboratory Press, 1988; F. Breitling and S. Dübel, Recombinant Antibodies, John Wiley & Sons, New York, 1999; H. Zola, Monoclonal Antibodies: Preparation and Use of Monoclonal Antibodies and Engineered Antibody Derivatives, Basics: From Background to Bench, BIOS Scientific Publishers, 2000; B.K.C. Lo, Antibody Engineering: Methods and Protocols, Methods in Molecular Biology, Humana Press, 2003; F. M. Ausubel et al., Eds., Short Protocols in Molecular Biology, Current Protocols, Wiley, 2002; Ormerod, M. G., Flow Cytometry: a practical approach, Oxford University Press, 2000; and Givan, A. L., Flow Cytometry: first principles, Wiley, New York, 2001.

According to aspects of the present disclosure, nucleic acid assays to detect a nucleic acid analyte relevant to non-familial late-onset Alzheimer's disease in a genetically modified mouse of the present disclosure compared to a wild-type mouse includes, but is not limited to, nucleic acid amplification techniques such as, but not limited to, PCR, RT-PCR ligation-mediated PCR and phi-29 PCR; nucleic acid hybridization techniques such as, but not limited to, Northern blot, Southern blot, RNase protection assay, dot blot, transcriptome analysis, and *in situ* hybridization. Nucleic acid assays for both qualitative and quantitative assay of a nucleic acid in a sample are described in detail in standard references, illustratively including J. Sambrook and D.W. Russell, Molecular Cloning: A Laboratory Manual, Cold Spring Harbor Laboratory Press; 3rd Ed., 2001; F. M. Ausubel et al., Eds., Short Protocols in Molecular Biology, Current Protocols, Wiley, 2002; C.W. Dieffenbach et al., PCR Primer: A Laboratory Manual, Cold Spring Harbor Laboratory Press, 2003; and V. Demidov et al., DNA Amplification: Current Technologies and Applications, Taylor & Francis, 2004.

Biomarker assays that can be used are well-known in the art and include, but are not limited to, assays of Aβ and tau species, neurofilaments, neurogranin, interleukins, TNFα, GM-CSF, and soluble Trem2 in cerebrospinal fluid (CSF), blood, serum, or tissue.

Cognitive assays that can be used are well-known in the art and include, but are not limited to, assays of spatial memory, short-term memory, long-term memory, assays of executive function, attentional tasks such as 3 and 5 choice serial reaction time tests, tests of processing speed, set shifting tests, reversal learning tasks, assays of object memory, assays of pattern recognition, assays of passive avoidance memory, assays of habituation, and assays of novel object recognition, water maze testing, fear conditioning tests, radial arm water maze testing, Y-maze testing, T-maze testing, and open field habituation tests.

Physical assessment methods of the animals that can be used are well-known in the art and include, but are not limited to, assessment of indices of frailty in a genetically modified mouse of the present disclosure compared to a control, such as an APOE3-expressing mouse or a wild-type mouse as a comparison to normal aging.

*In vivo* imaging methods that can be used are well-known in the art and include, but are not limited to, magnetic resonance imaging (MRI), computed tomography (CT) imaging, X-ray, optical imaging, and ultrasound imaging. Such imaging techniques can be used to assess one or more symptoms or signs associated with expression of human APOE4 p and mouse TREM2*R47H p and one or more of the following: (1) a nucleic acid encoding: ABCA7* A1541G p operably linked to a promoter, wherein the animal expresses the encoded ABCA7* A1541G p; (2) a nucleic acid encoding: APP* p operably linked to a promoter, wherein the animal expresses the encoded APP* p; (3) a nucleic acid encoding: PLCG2*M28L p operably linked to a promoter, wherein the animal expresses the encoded PLCG2*M28L p; (4) a nucleic acid encoding: MTHFR*A262V p operably linked to a promoter, wherein the animal expresses the encoded MTHFR*A262V p; (5) a disrupted *Ceacam1* gene such that functional CEACAM1 is not produced in the genetically modified mouse; and (6) a disrupted Il1rap gene such that functional IL1RAP is not produced in the genetically modified mouse, relevant to non-familial late-onset Alzheimer's disease including, but not limited to, amyloid or tau aggregation, abnormal blood flow, pathological neuronal loss, and abnormal glucose metabolism.

Assessment of changes to one or more of: a transcriptomic profile, a proteomic profile, and a metabolic profile of a genetically modified mouse of the present disclosure compared to a wild-type mouse and/or compared to clinical samples can be performed using methods that are well-known in the art.

Assessment of inflammation can be performed by assay of one or more biomarkers of inflammation, such as, but not limited to, IL-8, IL-11, TNF-alpha, granulocyte-macrophage colony stimulating factor (GM-CSF), TGF-beta, VEGF, monocyte chemotactic factor-1, macrophage migratory inhibitory factor, s100B, fibrinogen, and interferon gamma-inducible protein 10. Such assays can be performed on samples obtained from a genetically modified mouse of the present disclosure and a wild-type mouse, such as a sample of brain, spinal cord, blood, plasma, serum, cerebrospinal fluid or other relevant tissue or body fluid.

Morphological assessment of tissues and/or cells can include physical examination of gross anatomy and/or microscopic examination of tissues and/or cells with or without histochemical or cytochemical staining of the tissues and/or cells. Morphological assessment of tissues and/or cells can include assessment of synaptic and neuronal loss.

A method for screening for a compound for use in the treatment of Alzheimer's disease in an individual human subject having or suspected of having Alzheimer's disease is provided according to aspects of the present invention which include administering a compound to a genetically modified mouse of the present disclosure; and assessing an effect of the compound on amyloid and/or tau which are associated with expression of human APOE4 p and mouse TREM2*R47H p and a nucleic acid encoding: APP* p operably linked to a promoter, wherein the animal expresses the encoded APP* p, relevant to non-familial late-onset Alzheimer's disease on the mouse. Also disclosed herein but not forming part of the claimed invention is a method for screening for a compound for use in the treatment of Alzheimer's disease in an individual human subject having or suspected of having Alzheimer's disease which includes administering a compound to a genetically modified mouse of the present disclosure; and assessing an effect of the compound on amyloid and/or tau which are associated with expression of human APOE4 p and mouse TREM2*R47H p and one or more of the following: (1) a nucleic acid encoding: ABCA7* A1541G p operably linked to a promoter, wherein the animal expresses the encoded ABCA7* A1541G p; (2) a nucleic acid encoding: PLCG2*M28L p operably linked to a promoter, wherein the animal expresses the encoded PLCG2*M28L p; (3) a nucleic acid encoding: MTHFR*A262V p operably linked to a promoter, wherein the animal expresses the encoded MTHFR*A262V p; (4) a disrupted *Ceacam1* gene such that functional CEACAM1 is not produced in the genetically modified mouse; and (5) a disrupted Il1rap gene such that functional IL1RAP is not produced in the genetically modified mouse, relevant to non-familial late-onset Alzheimer's disease on the mouse. According to particular embodiments, assessing an effect of the compound on amyloid and/or tau includes assessing the level and/or localization of amyloid and/or tau. According to particular embodiments, assessing an effect of the compound on amyloid and/or tau includes assessing amyloid and/or tau aggregates in a genetically modified mouse of the present disclosure, such as assessment of aggregate size, number, location or a combination of any two or more thereof.

Cerebrovascular leakage is a key aspect of non-familial late-onset Alzheimer's disease and it is a surprising and unexpected finding of the present disclosure that expression of human APOE4 p and mouse TREM2*R47H p results in cerebrovascular leakage.

A method for screening for a compound for use in the treatment of non-familial late-onset Alzheimer's disease in an individual human subject having or suspected of having non-familial late-onset Alzheimer's disease is provided according to aspects of the present disclosure which include administering a compound to a genetically modified mouse of the present disclosure; and assessing an effect of the compound on cerebrovascular leakage which is associated with expression of human APOE4 p and mouse TREM2*R47H p relevant to non-familial late-onset Alzheimer's disease in the mouse. Assessing an effect of the compound on cerebrovascular leakage includes, but is not limited to, assessment of cerebrovascular permeability. The term "cerebrovascular permeability" refers to the capacity of a blood vessel wall to allow for normal movement of small molecules or substances such as water, ions, certain drugs and nutrients across the blood vessel wall while acting as a barrier to movement of larger molecules and substances, such as fibrinogen, and albumin. The term "cerebrovascular leakage" refers to an abnormality of blood vessels which results in leakage of larger molecules and substances, such as fibrinogen, and albumin, through blood vessel walls. Assays for assessing cerebrovascular leakage are well-known in the art, including *in vitro* assays using cells or tissues isolated from a genetically modified mouse of the present disclosure, and *in vivo* assays. A non-limiting example of an *in vivo* assay of cerebrovascular leakage includes intravenous injection of a labeled protein (e.g. Evans Blue-labeled albumin) and assessment of appearance of the dye-labeled protein in brain tissues, see for example, Radu et al., An in vivo Assay to Test Blood Vessel Permeability., J. Vis. Exp. 2013, (73):50062.

The putative treatment can be any treatment modality such as, but not limited to, administration of a compound. The term "compound" as used herein is not limited and encompasses small molecule chemicals and biologicals such as peptides, proteins, glycoproteins, including antibodies, for example.

Embodiments of inventive compositions and methods are illustrated in the following examples. These examples are provided for illustrative purposes and are not considered limitations on the scope of inventive compositions and methods.

### EXAMPLES

### Example 1. Generation of a Genetically Modified Mouse Comprising a Genomic Sequence Encoding Human APOE4 protein

The mouse *Apoe4* gene is located on chromosome 7 at 19,696,109 - 19,699,166. An *Apoe4* gene-targeting construct was made that included 4980 bp (SEQ ID NO:9) of mouse sequence, which defined the mouse 5' homology arm including exon 1 of mouse *ApoE,* 4292 bp (SEQ ID NO:10) of human *APOE4* sequence, including human protein coding exons 2-4 of the human gene as well as an additional 1.5 kb of flanking human sequence after the 3'UTR to include any potential regulatory sequences.

Figure 1 shows a schematic diagram of the humanized APOE4 construct (see "m/hAPOE targeting vector E4"). On Figure 1, (C C) above exon 4' shows the nucleotide (nt) present in exon 4 for arginine at p.R310 and R176, respectively, isoform E4. Isoform E2 with (T T) codes for cysteine at both C130 and C176, while E3 (T C) codes for cysteine at C130R and arginine at R176 sites.

Human *APOE4* exon 4 contains sequence that codes for the *APOE4* isoform of the gene and encodes the nucleotide sequence for arginine at R130 and R176. It is noted that APOE4 includes an 18 amino acid signal peptide at the N-terminus of the protein such that APOE4 including the signal peptide is 317 amino acids and the variant amino acids which differ among APOE2, APOE3 and APOE4 are at positions 130 and 176. In the mature APOE proteins (299 amino acids), the variant amino acids which differ among APOE2, APOE3 and APOE4 are at positions 112 and 158.

A Frt neo Frt selection cassette (FNF cassette, SEQ ID NO:11) was inserted after the human sequence followed by a Nde1 restriction site (for ease of Southern screening). The FNF cassette is followed by 5166 bp of mouse sequence (SEQ ID NO:12), the 3' homology arm. The resulting 14,438 bp synthesized construct was cloned into pBlight vector using recombineering techniques, producing a construct called mApoE_hAPOE4_PGKneo_mAPOE for gene targeting in embryonic stem cells.

The *ApoE4* gene-targeting construct was introduced into cultured embryonic stem (ES) cells of a C57Bl6 mouse strain by electroporation. Homologous recombination produced loci that retained all normal mouse regulatory sequences (plus non-coding exon one) together with the human APOE4 protein-encoding exons 2-4. Transfected ES cells were screened by Southern blot in ensure correct targeting. Three clones were identified that were correctly targeted. ES cells containing the correctly targeted locus were introduced into C57BL/6J embryos, and the resultant chimeric mice were bred with C57BL/6J mice. Offspring carrying the modified locus in the germ-line were interbred to generate the homozygous genetically modified genome. All F1 matings produced normal litter sizes with a Mendelian distribution of the locus.

### Example 2. Generation of a Genetically Modified Mouse Comprising a Genomic Sequence Encoding Mouse TREM2 protein

The *Trem2**R47H knock in (KI) allele was generated by pronuclear injection of Cas9 RNA (100 ng) and a single guide sequence (50 ng) GAAGCACTGGGGGAGACGCA (SEQ ID NO:7) with 183 nucleotide (nt) donor oligonucleotide (oligo) (40 ng) (GCCCTCAACACCACGGTGCTGCAGGGCATGGCCGGCCAGTCCTTGAGGGTGTCA TGTACTTATGACGCCTTGAAGCACTGGGGGAGACACA*Aa*GC*aT*GGTGTCGGCAGC TGGGTGAGGAGGGCCCATGCCAGCGTGTGGTGAGCACACACGGTGTGTGGCTGC TGGCCTTCCTGAAGAAGCGG, SEQ ID NO:8) containing a nucleotide G>A point mutation (underlined uppercase "A" at nt 89 in SEQ ID NO:8) for amino acid sequence change at R47H and 2 silent mutations (lysine AAG>AAA (underlined lower case "a" at nt 93 in SEQ ID NO:8) and alanine GCC>GCA (underlined lower case "a" at nt 96 in SEQ ID NO:8)) into the gene (to prevent re-cutting of the donor sequence in homologous directed repair). The CRISPR strategy resulted in the specific R47H knock in, and silent mutations present in the *Trem2* gene of founder mice.

Offspring carrying the modified allele in the germ-line were interbred to generate the homozygous genetically modified genome. All F1 matings produced normal litter sizes with a Mendelian distribution of the locus. The resulting inbred strain of mouse is designated C57BL/6J-Trem2^{em1Adpmc}/J (common name Trem2 R47H KI (JAX)) and expresses TREM2 protein.

### Example 3. Generation of a Genetically Modified Mouse Comprising a Genomic Sequence Encoding Human APOE4 Protein and a Genomic Sequence Encoding TREM2 Protein

In this example, a genetically modified mouse was generated by crossing an inbred strain of mouse, B6(SJL)-Apoe^{tm1.1(APOE*4)Adpmc}/J (common name APOE*4 KI (JAX)) expressing human APOE4 p with an inbred strain of mouse, C57BL/6J-Trem2^{em1Adpmc}/J (common name Trem2 R47H KI (JAX)) expressing mouse TREM2*R47H p. The resulting genetically modified mice, homozygous for both the humanized *APOE4* allele and the R47H allele of the mouse *Trem2* gene, are designated B6(SJL)-*Apoe^{tm1.1(APOE*4)Adiuj} Trem2^{em1Adiuj}*/J (formerly B6(SJL)-Apoe^{tm1.1(APOE*4)Adpmc} Trem2^{em1Adpmc}/J, abbreviated as APOE4 X Trem2 R47H (JAX) mice), common name: B6J.APOE4/Trem2, which express both human APOE4 and mouse TREM2*R47H p.

### Example 4. Generation of a Abca7*A1541G/ApoE4/Trem2*R47H Mouse Model for Late-Onset Alzheimer's Disease

A mutation corresponding to single nucleotide polymorphism rs3752246 in exon 33 of human *ABCA7*, which encodes a mutation of alanine to glycine at position A1527 of human ABCA7 was introduced into the homologous region of exon 32 of the mouse *Abca7* gene, which is A1541G in mouse ABCA7. This was accomplished by altering the corresponding codon from GCT to GGT. The CRISPR project was done directly into mice on the C57BL/6J background that were homozygous for both the human APOE4 and Trem2*R47H alleles (B6J-APOE4/Trem2*R47H mouse which expresses both human APOE4 and mouse TREM2).

CRISPR Guides used to cut the mouse *Abca7* gene:
Abca7_sgRNA1 (SEQ ID NO:33) : ACTTGACCAAGGAGCAGCTATCTGAA PAM site: CTGAA
Abca7_sgRNA2 (SEQ ID NO:34): GCAGCTATCTGAAGCTACACTGTGAGT PAM site: GTGAGT

Sequence of repair donor (mutation is bolded and underlined; this is the reverse orientation, SEQ ID NO:35):

AAAGGTTCCAATCCAGTCTCCACCACCCCTGAGCAACATGACCACTGATAGTAAATGGAT
GGACTCACAGTGTACCTTCAGATAGCTGCTCCTTGGTCAAGTTCAAAGGATGGTTGAGTGTAGTGA
TCTGTGGGCATGGCGGACTGGAC

SEQ ID NO:21- Wild-type mouse ABCA7 protein sequence (site of mutation is underlined and bolded):

MALGTQLMLLLWKNYTYRRRQPIQLLVELLWPLFLFFILVAVRHSHPPLEHHECHFPNKPLPS
AGTVPWLQGLVCNVNNSCFQHPTPGEKPGVLSNFKDSLISRLLADTRTVLGGHSIQDMLDALGKLIPVL
RAVGGGVRPQESDQPTSQGSVTKLLEKILQRASLDPVLGQAQDSMRKFSDAIRDLAQELLTLPSLMELR
ALLRRPRGSAGSLELVSEALCSTKGPSSPGGLSLNWYEANQLNEFMGPEVAPALPDNSLSPACSEFVGT
LDDHPVSRLLWRRLKPLILGKILFAPDTNFTRKLMAQVNQTFEELALLRDLHELWGVLGPQIFNFMNDS
TNVAMLQRLLDVGGTGQRQQTPRAQKKLEAIKDFLDPSRGGYSWREAHADVGRLAGILGQMMECVS
LDKLEAVPSEEALVSRALELLGERRLWAGIVFLSPEHPLDPSELSSPALSPGHLRFKIRMDIDDVTRTNKI
RDKFWDPGPSADPFMDLRYVWGGFVYLQDLLEQAAVRVLGGGNSRTGLYLQQMPHPCYVDDVFLR
VLSRSLPLFLTLAWIYSVALTVKAVVREKETRLRETMRAMGLSRAVLWLGWFLSCLGPFLVSAALLVL
VLKLGNILPYSHPVVIFLFLAAFAVATVAQSFLLSAFFSRANLAAACGGLAYFALYLPYVLCVAWRERL
HLGGLLAASLLSPVAFGFGCESLALLEEQGDGAQWHNLGTGPAEDVFSLAQVSAFLLLDAVIYGLALW
YLEAVCPGQYGIPEPWNFPFRRSYWCGPGPPKSSVLAPAPQDPKVLVEEPPLGLVPGVSIRGLKKHFRG
CPQPALQGLNLDFYEGHITAFLGHNGAGKTTTLSILSGLFPPSSGSASILGHDVQTNMAAIRPHLGICPQ
YNVLFDMLTVEEHVWFYGRLKGVSAAAMGPERERLIRDVGLTLKRDTQTRHLSGGMQRKLSVAIAFV
GGSRVVIMDEPTAGVDPASRRGIWELLLKYREGRTLILSTHHLDEAELLGDRVAMVAGGSLCCCGSPL
FLRRHLGCGYYLTLVKSSQSLVTHDAKGDSEDPRREKKSDGNGRTSDTAFTRGTSDKSNQAPAPGAVP
ITPSTARILELVQQHVPGAQLVEDLPHELLLVLPYAGALDGSFAMVFQELDQQLELLGLTGYGISDTNL
EEIFLKVVEDAHREGGDSRPQLHLRTCTPQPPTGPEASVLENGELAKLVLDPQAPQGLAPNAAQVQGW
TLTCQQLRALLHKRFLLARRSRRGLFAQVVLPALFVGLALFFSLIVPPFGQYPPLQLSPAMYGPQVSFFS
EDAPGDPNRMKLLEALLGEAGLQEPSMQDKDARGSECTHSLACYFTVPEVPPDVASILASGNWTPESP
SPACQCSQPGARRLLPDCPAGAGGPPPPQAVAGLGEVVQNLTGRNVSDFLVKTYPSLVRRGLKTKKW
VDEVRYGGFSLGGRDPDLPTGHEVVRTLAEIRALLSPQPGNALDRILNNLTQWALGLDARNSLKIWFN
NKGWHAMVAFVNRANNGLLHALLPSGPVRHAHSITTLNHPLNLTKEQLSEATLIASSVDVLVSICVVF
AMSFVPASFTLVLIEERITRAKHLQLVSGLPQTLYWLGNFLWDMCNYLVAVCIVVFIFLAFQQRAYVAP
ENLPALLLLLLLYGWSITPLMYPDSFFFSVPSTAYVVLTCINLFIGINSSMATFVLELLSDQNLQEVSRILK
QVFLIFPHFCLGRGLIDMVRNQAMADAFERLGDKQFQSPLRWDIIGKNLLAMMAQGPLFLLITLLLQHR
NRLLPQSKPRLLPPLGEEDEDVAQERERVTKGATQGDVLVLRDLTKVYRGQRNPAVDRLCLGIPPGEC
FGLLGVNGAGKTSTFRMVTGDTLPSSGEAVLAGHNVAQERSAAHRSMGYCPQSDAIFDLLTGREHLEL
FARLRGVPEAQVAQTALSGLVRLGLPSYADRPAGTYSGGNKRKLATALALVGDPAVVFLDEPTTGMD
PSARRFLWNSLLSVVREGRSVVLTSHSMEECEALCTRLAIMVNGRFRCLGSSQHLKGRFGAGHTLTLR
VPPDQPEPAIAFIRITFPGAELREVHGSRLRFQLPPGGRCTLTRVFRELAAQGRAHGVEDFSVSQTTLEEV
FLYFSKDQGEEEESSRQEAEEEEVSKPGRQHPKRVSRFLEDPSSVETMI

SEQ ID NO:20- Engineered mutant mouse ABCA7 protein sequence (ABCA7*A1541G p) (mutation is underlined and bolded):

MALGTQLMLLLWKNYTYRRRQPIQLLVELLWPLFLFFILVAVRHSHPPLEHHECHFPNKPLPS
AGTVPWLQGLVCNVNNSCFQHPTPGEKPGVLSNFKDSLISRLLADTRTVLGGHSIQDMLDALGKLIPVL
RAVGGGVRPQESDQPTSQGSVTKLLEKILQRASLDPVLGQAQDSMRKFSDAIRDLAQELLTLPSLMELR
ALLRRPRGSAGSLELVSEALCSTKGPSSPGGLSLNWYEANQLNEFMGPEVAPALPDNSLSPACSEFVGT
LDDHPVSRLLWRRLKPLILGKILFAPDTNFTRKLMAQVNQTFEELALLRDLHELWGVLGPQIFNFMNDS
TNVAMLQRLLDVGGTGQRQQTPRAQKKLEAIKDFLDPSRGGYSWREAHADVGRLAGILGQMMECVS
LDKLEAVPSEEALVSRALELLGERRLWAGIVFLSPEHPLDPSELSSPALSPGHLRFKIRMDIDDVTRTNKI
RDKFWDPGPSADPFMDLRYVWGGFVYLQDLLEQAAVRVLGGGNSRTGLYLQQMPHPCYVDDVFLR
VLSRSLPLFLTLAWIYSVALTVKAVVREKETRLRETMRAMGLSRAVLWLGWFLSCLGPFLVSAALLVL
VLKLGNILPYSHPVVIFLFLAAFAVATVAQSFLLSAFFSRANLAAACGGLAYFALYLPYVLCVAWRERL
HLGGLLAASLLSPVAFGFGCESLALLEEQGDGAQWHNLGTGPAEDVFSLAQVSAFLLLDAVIYGLALW
YLEAVCPGQYGIPEPWNFPFRRSYWCGPGPPKSSVLAPAPQDPKVLVEEPPLGLVPGVSIRGLKKHFRG
CPQPALQGLNLDFYEGHITAFLGHNGAGKTTTLSILSGLFPPSSGSASILGHDVQTNMAAIRPHLGICPQ
YNVLFDMLTVEEHVWFYGRLKGVSAAAMGPERERLIRDVGLTLKRDTQTRHLSGGMQRKLSVAIAFV
GGSRVVIMDEPTAGVDPASRRGIWELLLKYREGRTLILSTHHLDEAELLGDRVAMVAGGSLCCCGSPL
FLRRHLGCGYYLTLVKSSQSLVTHDAKGDSEDPRREKKSDGNGRTSDTAFTRGTSDKSNQAPAPGAVP
ITPSTARILELVQQHVPGAQLVEDLPHELLLVLPYAGALDGSFAMVFQELDQQLELLGLTGYGISDTNL
EEIFLKVVEDAHREGGDSRPQLHLRTCTPQPPTGPEASVLENGELAKLVLDPQAPQGLAPNAAQVQGW
TLTCQQLRALLHKRFLLARRSRRGLFAQVVLPALFVGLALFFSLIVPPFGQYPPLQLSPAMYGPQVSFFS
EDAPGDPNRMKLLEALLGEAGLQEPSMQDKDARGSECTHSLACYFTVPEVPPDVASILASGNWTPESP
SPACQCSQPGARRLLPDCPAGAGGPPPPQAVAGLGEVVQNLTGRNVSDFLVKTYPSLVRRGLKTKKW
VDEVRYGGFSLGGRDPDLPTGHEVVRTLAEIRALLSPQPGNALDRILNNLTQWALGLDARNSLKIWFN
NKGWHAMVAFVNRANNGLLHALLPSGPVRHAHSITTLNHPLNLTKEQLSEGTLIASSVDVLVSICVVF
AMSFVPASFTLVLIEERITRAKHLQLVSGLPQTLYWLGNFLWDMCNYLVAVCIVVFIFLAFQQRAYVAP
ENLPALLLLLLLYGWSITPLMYPDSFFFSVPSTAYVVLTCINLFIGINSSMATFVLELLSDQNLQEVSRILK
QVFLIFPHFCLGRGLIDMVRNQAMADAFERLGDKQFQSPLRWDIIGKNLLAMMAQGPLFLLITLLLQHR
NRLLPQSKPRLLPPLGEEDEDVAQERERVTKGATQGDVLVLRDLTKVYRGQRNPAVDRLCLGIPPGEC
FGLLGVNGAGKTSTFRMVTGDTLPSSGEAVLAGHNVAQERSAAHRSMGYCPQSDAIFDLLTGREHLEL
FARLRGVPEAQVAQTALSGLVRLGLPSYADRPAGTYSGGNKRKLATALALVGDPAVVFLDEPTTGMD
PSARRFLWNSLLSVVREGRSVVLTSHSMEECEALCTRLAIMVNGRFRCLGSSQHLKGRFGAGHTLTLR
VPPDQPEPAIAFIRITFPGAELREVHGSRLRFQLPPGGRCTLTRVFRELAAQGRAHGVEDFSVSQTTLEEV
FLYFSKDQGEEEESSRQEAEEEEVSKPGRQHPKRVSRFLEDPSSVETMI

SEQ ID NO:19: An engineered mouse mutant *Abca7* DNA sequence (Abca7*A1541G g) encoding engineered mutant mouse ABCA7 protein having the A1541G point mutation (ABCA7*A1541G p) - the gct codon that was engineered to ggt is underlined and in bold

ATGGCCTTAGGCACACAGCTGATGCTTCTGCTGTGGAAAAATTACACCTATCGACGGAGA
CAACCGATCCAACTACTAGTGGAGTTGCTTTGGCCCCTCTTCCTCTTCTTCATCCTAGTGGCTGTCC
GTCACTCCCACCCCCCTCTGGAGCATCACGAATGCCACTTTCCAAACAAGCCATTACCATCGGCGG
GCACGGTGCCCTGGCTGCAGGGCCTTGTCTGCAACGTAAACAACTCCTGCTTCCAGCACCCAACGC
CTGGCGAGAAGCCTGGGGTCCTGAGTAACTTTAAGGATTCCTTGATCTCGAGGCTCCTCGCTGATA
CCCGCACAGTGCTCGGGGGCCACAGCATCCAGGACATGCTGGATGCCCTGGGGAAACTGATCCCC
GTGCTCAGGGCAGTTGGAGGTGGAGCACGACCACAGGAGAGTGACCAGCCGACCAGTCAAGGGT
CAGTGACTAAGCTTCTGGAGAAGATCCTGCAAAGGGCATCCCTGGATCCTGTGCTGGGTCAAGCCC
AGGATTCTATGAGAAAGTTCTCAGATGCTATCAGGGATCTTGCCCAGGAGCTCCTGACACTGCCCA
GCCTGATGGAGCTCCGAGCTTTGCTGCGGAGGCCCCGAGGGTCAGCTGGTTCTCTGGAGCTGGTTT
CGGAGGCCCTCTGCAGTACCAAGGGACCCAGCAGTCCAGGGGGCCTGTCCCTCAATTGGTACGAA
GCCAACCAGCTTAATGAGTTCATGGGGCCAGAGGTGGCCCCTGCCCTGCCTGACAACAGTCTCAGC
CCTGCCTGCTCTGAGTTTGTGGGGACACTGGATGACCACCCTGTGTCTCGGCTGCTCTGGAGGCGC
CTGAAGCCATTGATCCTCGGGAAAATTCTCTTTGCACCTGACACAAACTTCACTCGGAAGCTCATG
GCTCAGGTGAACCAGACCTTCGAGGAGCTGGCTCTGTTGAGGGACCTACACGAACTCTGGGGGGT
GCTGGGACCCCAGATCTTCAACTTCATGAATGACAGTACCAACGTGGCCATGCTTCAGAGGCTTCT

GGATGTGGGGGGCACAGGGCAGAGGCAGCAGACACCCAGAGCCCAGAAGAAGTTGGAGGCTATC
AAAGACTTTCTGGATCCTAGTAGGGGTGGCTACAGCTGGCGGGAGGCCCACGCAGATATGGGACG
CCTGGCTGGAATCCTAGGACAAATGATGGAGTGTGTGTCCCTGGACAAGCTGGAGGCTGTGCCCTC
AGAGGAAGCTCTTGTGTCCCGTGCCCTGGAGCTGCTGGGTGAGCGCCGCCTCTGGGCAGGCATCGT
GTTCCTGAGCCCAGAGCATCCTCTGGACCCATCCGAACTGTCATCTCCAGCCCTGAGTCCTGGCCA
CCTACGATTCAAGATTCGAATGGATATCGATGATGTCACAAGGACCAATAAGATCAGGGACAAGT
TTTGGGACCCAGGTCCGTCAGCAGATCCTTTCATGGACCTTCGGTATGTGTGGGGAGGCTTCGTGT
ACCTGCAGGACCTGCTGGAGCAGGCAGCTGTGCGAGTGCTCGGTGGCGGGAACTCCCGCACAGGT
CTCTACCTGCAGCAGATGCCACACCCCTGCTACGTGGATGATGTGTTCCTGCGGGTGCTGAGCCGG
TCTCTGCCTCTGTTTCTGACTCTGGCCTGGATTTATTCGGTGGCGCTCACTGTGAAGGCCGTGGTGC
GTGAGAAAGAGACACGGCTGCGAGAAACCATGCGTGCGATGGGGCTGAGCCGCGCGGTGCTCTGG
CTTGGTTGGTTCCTCAGCTGCCTGGGACCCTTCCTGGTCAGCGCTGCGTTGCTGGTATTAGTGCTTA
AGCTAGGGAACATCCTTCCTTACAGCCACCCGGTTGTAATCTTCCTTTTCTTGGCGGCCTTCGCGGT
GGCCACCGTCGCTCAGAGTTTTCTGCTCAGCGCCTTCTTCTCCAGGGCCAACCTGGCAGCAGCCTG
CGGGGGGCTCGCCTATTTTGCGCTCTATCTGCCCTACGTACTGTGTGTGGCCTGGCGCGAGCGCCT
GCACCTGGGCGGACTCTTAGCTGCGAGCCTGCTGTCCCCTGTAGCCTTTGGCTTTGGATGCGAAAG
CCTGGCGCTACTAGAGGAGCAGGGAGACGGGGCTCAGTGGCACAATTTGGGCACAGGCCCCGCGG
AGGACGTCTTCAGCCTGGCCCAGGTGTCTGCCTTCCTGTTGCTTGATGCCGTCATCTACGGCCTTGC
CCTCTGGTACCTAGAGGCTGTGTGCCCAGGCCAGTATGGAATCCCTGAACCATGGAATTTCCCTTT
TCGGAGGAGCTACTGGTGTGGACCTGGGCCTCCCAAGAGTTCTGTCTTGGCCCCTGCCCCACAAGA
TCCCAAGGTTCTGGTGGAAGAGCCACCCCTTGGCCTGGTTCCTGGTGTCTCCATTCGAGGCCTGAA
GAAACATTTTCGTGGCTGTCCGCAGCCAGCCCTGCAAGGACTCAACCTTGACTTCTACGAAGGCCA
CATCACTGCCTTTTTGGGTCACAACGGGGCTGGCAAGACAACCACACTGTCCATTTTGAGTGGTCT
CTTCCCACCCAGTAGTGGCTCGGCCTCCATCCTGGGCCATGATGTACAAACCAACATGGCAGCCAT
CCGGCCCCACCTGGGCATCTGCCCGCAGTACAATGTGCTGTTTGATATGCTGACAGTGGAAGAACA
TGTTTGGTTCTATGGCCGTTTGAAAGGCGTGAGTGCAGCCGCCATGGGCCCCGAGCGGGAACGTCT
GATACGGGATGTGGGGCTTACCCTCAAGCGGGACACACAGACACGCCACCTCTCTGGTGGAATGC
AGAGAAAACTTTCTGTGGCCATTGCCTTTGTGGGTGGCTCTCGTGTGGTCATCATGGACGAGCCCA
CTGCTGGTGTGGACCCCGCTTCCCGCCGTGGCATTTGGGAATTGCTACTTAAGTACAGAGAAGGTC
GGACACTGATTCTCTCCACTCACCACCTGGATGAGGCAGAGCTCTTGGGAGATCGCGTGGCCATGG
TGGCAGGTGGCTCTTTGTGCTGCTGTGGGTCCCCGCTTTTCTTGCGCCGACACTTGGGCTGCGGTTA
CTACCTGACCCTGGTGAAGAGTTCTCAGTCCCTCGTCACCCATGATGCTAAGGGAGACAGTGAGGA
CCCCAGACGGGAAAAGAAGTCAGATGGCAATGGCAGGACGTCAGACACAGCGTTCACACGAGGA
ACCTCAGACAAGAGCAACCAGGCCCCGGCTCCTGGCGCCGTTCCCATCACCCCAAGCACAGCCCG
GATACTAGAGCTAGTGCAGCAGCATGTGCCTGGAGCACAACTCGTGGAGGACCTGCCCCATGAGC
TTCTGCTTGTGCTACCCTATGCGGGGGCCCTGGATGGCAGCTTCGCCATGGTCTTCCAGGAGCTGG
ATCAGCAGCTGGAGCTCCTGGGTCTCACAGGCTACGGGATCTCGGACACCAACCTGGAGGAGATC
TTCCTAAAGGTGGTGGAGGATGCGCACAGAGAAGGTGGGGACTCTAGACCGCAGCTGCACCTTCG
CACATGCACTCCACAGCCCCCCACAGGGCCAGAGGCATCAGTTCTGGAGAATGGGGAGCTGGCCC
CACAGGGCTTGGCACCCAACGCTGCCCAAGTGCAAGGCTGGACACTTACCTGTCAACAGCTCCGG
GCTCTGCTCCACAAGCGTTTTCTGCTTGCTCGCCGCAGCCGCCGGGGCCTGTTTGCACAGGTTGTGT
TGCCTGCCCTCTTTGTGGGCCTGGCCCTGTTCTTCAGCCTCATTGTGCCTCCTTTTGGCCAGTACCC
ACCCCTGCAGCTCAGCCCTGCTATGTATGGCCCTCAGGTCTCGTTCTTCAGTGAGGATGCCCCTGG
GGACCCCAACCGGATGAAGCTGCTGGAGGCTCTGCTAGGGGAGGCTGGGCTGCAGGAACCCAGTA
TGCAGGACAAAGATGCCAGGGGATCTGAGTGTACACACTCCCTAGCTTGCTACTTCACGGTCCCTG
AGGTCCCTCCTGATGTGGCCAGCATCCTGGCCAGTGGCAACTGGACGCCAGAATCTCCATCCCCAG
CTTGCCAATGCAGTCAGCCTGGAGCCCGCCGCCTGTTGCCAGATTGCCCGGCTGGAGCTGGGGGTC
CACCACCCCCCCAGGCTGTGGCTGGCTTGGGGGAGGTGGTCCAGAACCTCACTGGCCGAAATGTG
TCTGACTTTTTGGTGAAGACATACCCCAGCCTGGTGCGCCGAGGCCTAAAGACCAAGAAGTGGGT
GGATGAGGTCAGATATGGGGGCTTCTCCCTGGGAGGCCGAGATCCAGACCTGCCCACAGGGCATG
AGGTGGTCCGCACATTGGCAGAGATTCGGGCACTGCTGAGCCCCCAACCTGGGAATGCGCTAGAC
CGTATCCTGAACAACCTCACTCAGTGGGCCCTTGGCCTTGATGCTCGGAACAGCCTCAAGATCTGG
TTCAACAACAAGGGCTGGCATGCCATGGTGGCCTTTGTGAACCGAGCCAACAATGGACTCCTACAT
GCCCTCCTACCATCTGGTCCAGTCCGCCATGCCCACAGCATCACTACACTCAACCATCCTTTGAACT
TGACCAAGGAGCAGCTATCTGAAGCTACACTGATAGCCTCCTCTGTGGATGTCCTTGTCTCCATCT
GTGTGGTCTTCGCCATGTCATTTGTCCCAGCCAGCTTTACCCTGGTCCTCATAGAGGAACGCATCAC
CAGAGCCAAGCATCTGCAGCTGGTCAGCGGCCTGCCCCAAACCCTCTATTGGCTTGGCAACTTCCT
CTGGGACATGTGTAACTACTTGGTGGCAGTGTGCATAGTGGTGTTCATCTTCCTAGCCTTTCAGCA
GAGAGCCTATGTGGCCCCAGAGAACCTGCCTGCTCTCTTACTCTTGCTTCTGCTGTATGGGTGGTCT
ATCACACCACTCATGTACCCAGCCTCCTTCTTCTTCTCAGTGCCCAGCACGGCCTATGTGGTGCTCA

CCTGCATCAACCTCTTCATTGGCATCAATAGCAGCATGGCCACCTTCGTGCTAGAACTGCTTTCAG
ATCAGAACCTGCAAGAAGTGAGCCGGATCCTGAAACAAGTGTTTCTTATTTTCCCCCACTTTTGCC
TTGGCCGAGGGCTCATTGACATGGTTCGGAACCAGGCCATGGCAGATGCCTTTGAGCGCTTAGGA
GACAAGCAATTTCAGTCACCCCTACGCTGGGACATCATTGGCAAGAACCTCCTGGCCATGATGGCC
CAGGGACCTCTGTTCCTCCTCATCACACTCCTGCTCCAACACCGCAACCGTCTCCTGCCACAATCA
AAACCAAGACTGCTGCCGCCCCTGGGGGAGGAGGATGAGGATGTGGCTCAAGAGCGTGAGCGGG
TGACCAAGGGGGCTACCCAGGGGGATGTGCTAGTCCTCAGGGACTTGACCAAGGTTTACCGTGGG
CAGAGGAACCCAGCTGTGGATCGCCTGTGCTTAGGGATCCCCCCTGGGGAGTGTTTCGGGCTGCTG
GGTGTCAACGGGGCAGGGAAGACATCCACCTTCCGCATGGTGACAGGGGACACACTGCCCAGCAG
TGGTGAAGCAGTACTGGCAGGCCACAACGTGGCCCAGGAGCGGTCTGCCGCACACCGCAGCATGG
GCTACTGTCCCCAGTCTGATGCCATCTTCGACCTGCTGACCGGCCGGGAACATCTGGAACTGTTTG
CTCGCCTGCGCGGGGTGCCCGAGGCCCAAGTTGCCCAGACTGCGCTCTCTGGCCTGGTGCGCCTGG
GCCTTCCTAGCTATGCAGACCGACCCGCGGGTACCTACAGCGGAGGCAACAAACGGAAGCTGGCG
ACAGCCTTAGCTCTGGTTGGTGACCCAGCTGTGGTCTTTCTGGACGAGCCCACCACAGGCATGGAC
CCAAGTGCGCGGCGATTTCTTTGGAACAGCTTGCTGTCCGTGGTGCGCGAGGGCCGCTCCGTAGTG
CTCACGTCGCACAGCATGGAGGAGTGCGAAGCGCTCTGCACGCGCCTGGCCATCATGGTGAACGG
GCGGTTCCGCTGTCTGGGAAGCTCTCAGCATCTCAAAGGCAGGTTCGGGGCTGGCCACACACTGAC
TCTAAGGGTCCCACCGGACCAGCCTGAGCCGGCGATAGCCTTCATCAGGATCACATTCCCTGGGGC
TGAACTCCGGGAGGTGCACGGCAGCCGTCTGCGCTTCCAACTGCCACCGGGGGGCAGATGCACCC
TGACACGAGTGTTCAGGGAGCTGGCTGCCCAGGGCAGGGCCCACGGTGTGGAGGACTTCTCTGTG
AGCCAGACCACTCTGGAGGAGGTGTTCCTATATTTCTCCAAAGACCAAGGGGAAGAGGAAGAGAG
CAGTCGGCAGGAGGCTGAAGAAGAGGAGGTTTCCAAACCTGGCCGGCAGCATCCCAAACGTGTCA
GCCGATTCCTGGAAGACCCCAGCTCTGTGGAGACCATGATCTGAGCATGCCTGCCTTGGGACTGAG
TGGCAAAGCTCAGACAGAGGATCTCTGTACCATACGCTGGCTCCCAGAAAGCCTTGGGCTCTGGG
GGAAATAAAAAGAAACTAGAATGAGAAAAAAAAAAAAAAA

### Example 5. Generation of a Humanized APP/ApoE4/Trem2*R47H Mouse Model

CRISPR technology was used to introduce the G601R, F606Y, and R609H mutations into exon 14 of the mouse *App* gene so that the mouse ABETA42 sequence matches that of human ABETA42. The CRISPR project was done directly into mice on the C57BL/6J background that were homozygous for both the human APOE4 and Trem2*R47H alleles (B6J-APOE4/Trem2*R47H mouse which expresses both human APOE4 and mouse TREM2). Appropriate founders were bred to the same APOE4/Trem2 background to keep these alleles homozygous.

The corresponding nucleotide sequences for the ABETA42 regions are shown below. Bases that differ are in bold and underlined.
Mouse ABETA42 (SEQ ID NO:36): DAEF**G**HDSG**F**EV**R**HQKLVFFAEDVGSNKGAIIGLMVGGVVIA
Human ABETA42 (SEQ ID NO:37): DAEF**R**HDSG**Y**EV**H**HQKLVFFAEDVGSNKGAIIGLMVGGVVIA

The genomic sequence was targeted using CRISPR guides:
Guide 1 (SEQ ID NO:38): TTTGATGGCGGACTTCAAATC
Guide 2 (SEQ ID NO:39): GTGAAGATGGATGCAGAATT

The repair construct used to produce engineered *App* sequence was (reverse sequence is shown; mutated codons are underlined and mutations are in bold, SEQ ID NO:40):

TTCATCTGGCCAGACGCAATCTCGGGGAGAGGCAGTTTATTTTTGCCTACC
AGTTTTTGATGATGGACTTCATATCCTGAATCATGTCGGAATTCTGCATCCATCTT
CACTTCCGAGATCTCTTCCG

The corresponding nucleotide sequences for the *Abeta42* regions are shown below. Codons that differ are underlined, with engineered mutations in bold.

Mouse C57BL/6J wild-type Abeta 42 encoding sequence (SEQ ID NO:22):

GATGCAGAATTCGGACATGATTCAGGATTTGAAGTCCGCCATCAAAAACT
GGTGTTCTTTGCTGAAGATGTGGGTTCGAACAAAGGCGCCATCATCGGACTCATG
GTGGGCGGCGTTGTCATAGCA

Humanized mouse *Abeta42* encoding sequence (SEQ ID NO:23): A mouse mutant *App* DNA sequence (*App* g) encoding mouse APP protein having G601R, F606Y, and R609H point mutations in exon 14 of the mouse *App* gene

GATGCAGAATTCCGACATGATTCAGGATATGAAGTCCATCATCAAAAACT
GGTGTTCTTTGCTGAAGATGTGGGTTCGAACAAAGGCGCCATCATCGGACTCATG
GTGGGCGGCGTTGTCATAGCA

SEQ ID NO:24 A mouse mutant APP protein having G601R, F606Y, and R609H point mutations (APP* p)
DAEF**R**HDSG**Y**EV**H**HQKLVFFAEDVGSNKGAIIGLMVGGVVIA

### Example 6. Generation of the Plcg2*M28L/ApoE4/Trem2*R47H Mouse Model for Late-Onset Alzheimer's Disease

The *Plcg2* locus has been identified by genome-wide association studies to be related to the risk of Alzheimer's disease. Specifically, the PLCG2 M28L point mutation is associated with increased AD risk. The M28L mutation was engineered using CRISPR technology into the human homologous region of the mouse *Plcg2* gene in mice on the C57BL/6J background that were homozygous for both the human APOE4 and Trem2*R47H alleles (B6J-APOE4/Trem2*R47H mouse which express both human APOE4 and mouse TREM2).

The mouse genomic *Plcg2* sequence was targeted using CRISPR guide (SEQ ID NO:41):GAGAGCGCTGGAGTTAGGGA

The sequence was repaired to alter the ATG to TTG to cause the methionine to leucine mutation. In addition, a silent mutation ACC->ACT was introduced into the next codon to prevent the guide from re-cutting.

Mouse C57BL/6J wild-type *Plcg2* nucleotide sequence (SEQ ID NO:25):

ATGACCACCATGGTCAACGTGGACACCCTTCCAGAATATGAGAAGAGTCA
GATCAAGAGAGCGCTGGAGTTAGGGACGGTGATGACCGTGTTCAACGCCCGCAA
ATCCACCCCAGAGCGGAGGACAG…

The repair construct used to produce engineered *Plcg2* sequence was (the amino-acid altering mutation is in bold, SEQ ID NO:28):

ATGACCACCATGGTCAACGTGGACACCCTTCCAGAATATGAGAAGAGTCA
GATCAAGAGAGCGCTGGAGTTAGGGACGGTGTTGACTGTGTTCAACGCCCGCAA
ATCCACCCCAGAGCGGAGGACAG

Humanized mouse *Plcg2* nucleotide sequence (SEQ ID NO:29) -a mouse mutant PLCG2 (fragment) having an M28L point mutation:

ATGACCACCATGGTCAACGTGGACACCCTTCCAGAATATGAGAAGAGTCA
GATCAAGAGAGCGCTGGAGTTAGGGACGGTGTTGACTGTGTTCAACGCCCGCAA
ATCCACCCCAGAGCGGAGGACAG...

Mouse PLGC2 amino acid sequence (fragment) (first 50 amino acids, SEQ ID NO:26)
MTTMVNVDTL PEYEKSQIKR ALELGTV**M**TV FNARKSTPER RTVQMIMETR ...

Engineered mouse PLGC2 amino acid sequence (first 50 amino acids, SEQ ID NO:30)-A mouse mutant PLCG2*M28L p (fragment) having an M28L point mutation.
MTTMVNVDTL PEYEKSQIKR ALELGTVLTV FNARKSTPER RTVQMIMETR ...

Human Plcg2 amino acid sequence (fragment), with non-disease-associated variant first 50 amino acids, SEQ ID NO:27):

MSTTVNVDSL AEYEKSQIKR ALELGTVMTV FSFRKSTPER
RTVQVIMETR ...

Engineered humanized mouse PLGC2 amino acid sequence Plcg2*M28L (complete, SEQ ID NO:32) 1265 amino acids - a mouse mutant PLCG2 having an M28L point mutation.

MTTMVNVDTLPEYEKSQIKRALELGTVLTVFNARKSTPERRTVQMIMETRQVAWSKTADKIE
GFLDIMEIKEIRPGKNSKDFERAKAVRHKAECCFTILYGTQFVLSTLSLATDSKEDAVKWLSGLKILHQE
AMSASTPTMIESWLRKQIYSVDQTRRNSISLRELKTILPLVNFKVSGIKFLKDKLVEIGAQKDELSFEQFH
LFYKKLMFDQQKSILDEFKKDSSVFILGNTDRPDASAVYLQDFQRFLLHEQQELWAQDLNKVRERMT
KFIDDTMRETAEPFLFVDEFLTYLFSRENSIWDEKYDAVDMQDMNNPLSHYWISSSHNTYLTGDQLRS
ESSTEAYIRCLRAGCRCIELDCWDGPDGKPIIYHGWTRTTKIKFDDVVQAIRDHAFVTSSFPVILSIEEHC
SVEQQRHMAKVFKEVLGDMLLTKPTEASADQLPSPSQLREKIIIKHKKLGPKGDVDVNVEDKKDEHKP
QGELYMWDSIDQKWTRHYCAIADAKLSFGDDIEQAVEEEPVQDTPPTELHFGEKWFHKKVESRTSAE

KLLQEYCAETGAKDGTFLVRESETFPNDYTLSFWRSGRVQHCRIRSTMENGVMKYYLTDNLTFNSIYA
LIQHYREAHLRCAEFELRLTDPVPNPNPHESKPWYYDSLSRGEAEDMLMRIPRDGAFLIRKREGTNSYA
ITFRARGKVKHCRINRDGRHFVLGTSAYFESLVELVSYYEKHALYRKMRLRYPVTPELLERYNMERDI
NSLYDVSRMYVDPSEINPSMPQRTVKALYDYKAKRSDELTFCRGALIHNVSKEPGGWWKGDYGTRIQ
QYFPSNYVEDISAGDAEEMEKQIIEDNPLGSLCKGILDLNTYNVVKAPQGKNQKAFVFILEPKKQGDPP
VEFATDRVEELFEWFQSIREITWKMDTKENNMKYWERNQSIAIELSDLVVYCKPTSKTKDHLENPDFR
EIRSFVETKADSIVRQKPVDLLRYNQKGLTRVYPKGQRVDSSNYDPFRLWLCGSQMVALNFQTPDKY
MQMNHALFSLNGRTGYVLQPESMRSEKYDPMPLESQRKILMTLTVKVLGARHLPKLGRSIACPFVEVE
ICGAEYDSNKFKTTVVNDNGLSPVWAPTQEKVTFEIYDPNLAFLRFVVYEEDMFSDPNFLAHATYPIKG
IKSGFRSVPLKNGYSEDIELASLLVFCEMRPVLESEEELYSSCRQLRRRQEELNNQLFLYDTHQNLRGAN
RDALVKEFNVNENQLQLYQEKCNRRLREKRVSNSRFYS

Nucleotide sequence encoding engineered humanized mouse Plcg2*M28L (complete, SEQ ID NO:31)

ATGACCACCATGGTCAACGTGGACACCCTTCCAGAATATGAGAAGAGTCAGATCAAGAGA
GCGCTGGAGTTAGGGACGGTGTTGACCGTGTTCAACGCCCGCAAATCCACCCCAGAGCGGAGGAC
AGTACAGATGATCATGGAGACTCGGCAGGTGGCATGGAGCAAGACCGCAGACAAGATTGAAGGC
TTCCTGGACATCATGGAGATAAAGGAAATCCGTCCGGGGAAGAACTCCAAGGACTTTGAGCGAGC
CAAGGCTGTCCGCCACAAGGCAGAATGTTGCTTCACCATCCTCTACGGCACCCAGTTTGTCCTCAG
CACGCTCAGTTTGGCAACGGACTCAAAGGAGGATGCGGTGAAGTGGCTCTCTGGTTTGAAGATCCT
ACACCAGGAAGCGATGAGTGCATCCACCCCCACAATGATTGAGAGTTGGCTGAGAAAACAGATTT
ATTCAGTAGATCAAACCCGAAGAAACAGCATCAGCCTCCGGGAGCTGAAGACCATCTTGCCCCTG
GTCAACTTCAAAGTGAGCGGCATCAAGTTTCTCAAGGACAAGCTGGTGGAAATCGGCGCACAGAA
AGATGAGCTCAGCTTTGAACAGTTCCATCTCTTCTATAAGAAACTCATGTTTGACCAGCAAAAATC
GATACTTGACGAATTCAAAAAAGACTCCTCCGTGTTCATCCTAGGAAACACAGACCGGCCGGATG
CCTCGGCTGTCTACCTGCAGGACTTCCAAAGGTTTCTTTTACATGAACAGCAGGAGCTATGGGCTC
AGGATCTGAACAAAGTCCGGGAGCGGATGACCAAGTTTATCGACGACACAATGAGAGAGACCGC
AGAGCCCTTCTTATTTGTGGATGAGTTCCTCACGTATCTGTTCTCCCGGGAGAACAGCATCTGGGA
CGAGAAGTACGATGCAGTGGACATGCAGGACATGAACAACCCCTTGTCCCACTACTGGATCTCCTC
TTCCCACAACACGTACCTCACTGGGGACCAGCTGCGTAGTGAGTCCTCCACGGAAGCGTATATCCG
CTGTCTGCGCGCTGGTTGCCGCTGCATTGAGCTGGACTGCTGGGACGGGCCTGATGGGAAGCCCAT
TATCTACCACGGCTGGACACGGACCACCAAGATCAAGTTTGATGATGTTGTTCAGGCCATCCGAGA
CCACGCCTTTGTTACCTCCAGCTTCCCCGTCATCTTGTCTATCGAGGAGCACTGCAGTGTGGAGCA
GCAACGTCACATGGCCAAGGTGTTCAAGGAAGTGTTAGGAGACATGCTGTTGACGAAGCCCACAG
AGGCCAGTGCTGACCAGCTGCCCTCGCCCAGTCAGCTTCGTGAGAAGATCATTATCAAGCATAAG
AAGCTGGGTCCCAAGGGTGATGTCGACGTCAACGTGGAGGACAAGAAAGATGAGCACAAGCCCC
AGGGCGAGCTGTATATGTGGGACTCCATCGACCAGAAATGGACGCGCCACTACTGTGCCATTGCT
GACGCCAAGCTGTCCTTCGGTGATGACATTGAACAGGCTGTGGAGGAGGAGCCGGTCCAGGACAC
TCCCCCCACGGAGCTGCATTTTGGGGAGAAATGGTTCCACAAGAAGGTGGAGAGTAGGACCAGTG
CGGAGAAGCTGCTGCAAGAGTACTGTGCTGAGACCGGGGCCAAGGACGGCACCTTCCTGGTGCGG
GAGAGCGAGACCTTCCCCAATGACTACACACTCTCCTTCTGGCGGTCTGGCCGGGTGCAGCACTGC
CGGATCCGGTCTACTATGGAGAATGGGGTCATGAAGTACTACCTGACCGACAACCTCACGTTCAAC
AGCATCTACGCCCTCATCCAGCACTACCGTGAGGCACACCTGCGCTGCGCGGAGTTCGAGCTGCGG
CTCACAGACCCGGTGCCCAACCCCAACCCACACGAGTCCAAGCCGTGGTACTATGACAGTCTGAG
CAGGGGGGAAGCGGAGGACATGCTGATGCGGATCCCCCGAGATGGAGCCTTCCTCATCCGGAAGA
GGGAGGGGACCAACTCCTACGCCATCACCTTCAGGGCCAGGGGCAAGGTGAAACATTGTCGCATC
AACCGGGACGGACGCCACTTCGTGCTGGGCACCTCTGCCTACTTTGAGAGCCTGGTGGAGCTTGTC
AGCTACTACGAGAAGCACGCGCTCTATCGCAAGATGAGGCTTCGATACCCCGTGACCCCGGAGCT
CCTGGAGCGATATAATATGGAAAGAGACATCAACTCTCTTTATGACGTCAGCAGGATGTATGTGG
ACCCAAGTGAGATCAACCCTTCCATGCCTCAGAGGACCGTGAAAGCGCTCTATGACTACAAAGCC
AAGCGCAGCGACGAGCTGACCTTCTGCCGCGGTGCCCTCATCCACAATGTCTCCAAGGAGCCCGG
GGGATGGTGGAAAGGGGACTACGGGACCCGGATCCAGCAGTACTTCCCGTCCAACTATGTCGAGG
ATATCTCGGCAGGCGATGCTGAGGAGATGGAAAAGCAGATTATTGAAGACAATCCCCTCGGGTCT
CTCTGCAAAGGCATATTGGATCTTAATACCTACAACGTTGTGAAGGCCCCCCAGGGGAAAAACCA
GAAAGCCTTCGTCTTTATCCTGGAGCCTAAGAAACAGGGAGATCCTCCCGTGGAGTTCGCGACCGA
CCGTGTGGAGGAGCTGTTTGAGTGGTTTCAGAGCATACGGGAGATCACTTGGAAGATGGACACCA
AGGAGAACAACATGAAGTACTGGGAGAGGAACCAGTCCATCGCCATTGAGCTCTCGGACTTGGTT
GTATACTGCAAGCCAACTAGCAAAACCAAGGACCATTTGGAAAATCCCGACTTCCGAGAAATCCG

CTCTTTGTGGAGACGAAGGCAGACAGCATTGTCAGGCAAAAACCTGTCGATCTATTGCGATACAA
TCAGAAGGGCCTGACCCGAGTCTACCCCAAGGGACAGAGAGTTGACTCTTCGAACTATGACCCCTT
CCGTCTGTGGCTGTGCGGCTCCCAGATGGTGGCACTCAATTTCCAGACTCCAGACAAATACATGCA
GATGAACCACGCGCTGTTTTCGCTCAATGGGCGAACAGGCTACGTCCTGCAGCCTGAGAGCATGC
GCTCTGAGAAGTATGATCCGATGCCCCTGGAGTCCCAGAGGAAGATCCTGATGACACTCACTGTCA
AGGTTCTTGGTGCACGCCACCTCCCTAAACTAGGGCGGAGTATTGCCTGTCCTTTTGTGGAGGTGG
AAATCTGCGGGGCTGAGTATGACAGCAACAAATTCAAGACGACGGTTGTGAATGACAATGGCCTC
AGCCCTGTCTGGGCTCCAACTCAGGAGAAGGTGACATTTGAGATCTATGATCCAAACCTTGCCTTC
CTACGCTTTGTGGTCTACGAAGAAGACATGTTCAGTGATCCCAACTTCCTGGCTCATGCCACGTAC
CCCATTAAAGGCATCAAATCCGGGTTTAGATCAGTCCCTCTGAAGAATGGGTACAGTGAAGACAT
CGAGCTGGCATCCCTCCTGGTTTTCTGTGAGATGAGGCCAGTCCTGGAGAGTGAAGAGGAACTCTA
TTCCTCCTGTCGCCAACTGCGGAGGCGGCAAGAAGAGCTCAACAACCAGCTCTTCCTTTACGACAC
GCACCAGAACCTGCGCGGAGCCAACCGTGATGCCCTGGTGAAGGAGTTCAATGTTAACGAGAATC
AGCTGCAGCTGTACCAGGAGAAGTGCAACCGGAGGCTGAGAGAGAAGAGAGTAAGCAACAGCAG
GTTCTACTCCTAGAAGCCGTGTGTGCGTGCGCGCATGCGCACGGTGTTCACGAGCGCATCTGGGTA
CGGGAGAGCCTTATAAACAGCCCCCAGGAAGAGATTCATCTGTGACATTCATCGTCCTCTCTCCGA
GCCACTCACCATAGAGATGACAGTTCTGAAGACGCCCCCGTGGGCAGGAGGCGTTGAGACCTTCC
TGCTATTTTTCCACCTTGGACAACTTTCTTAACTTATACTACCCCTGATAGAGGATCCCCCCAACTG
TGTGCCTTTTGAACTTCCCTGTTCTCAGCCTCATTGAATAAAGACAACAGTTTCATGCCGTTAA

### Example 7. Generation of the Ceacam1 KO/ApoE4/Trem2*R47H Mouse Model for Late-Onset Alzheimer's Disease

Expression of the mouse *Ceacam1* gene in the B6J-APOE4/Trem2*R47H mouse (which expresses both human APOE4 and mouse TREM2) was knocked out using CRISPR technology so that no functional CEACAM1 was expressed in the genetically modified mouse.

The *Ceacam1* genomic sequence was targeted using two CRISPR guides on either side of exon 1:
Upstream guide 1 (SEQ ID NO:42): GGAGCTTTGTCTCCTCCCAG
Upstream guide 2 (SEQ ID NO:43): AAGGAGCTTTGTCTCCTCCC
Downstream guide 1 (SEQ ID NO:44): CCTTTAAAGGCGACAGCTAA
Downstream guide 2 (SEQ ID NO:45): GCCGTTTTACACATAGAACT

CRISPR founders were analyzed by PCR across the targeted region using a PCR primer on either side of the targeted region.
Forward primer (SEQ ID NO:46): CCTTGCTGCTCGGAGTATGT
Reverse primer (SEQ ID NO:47): CTGACCCCCTCCTCTAAAGA

Founders exhibiting a deletion were bred to the B6J-APOE4/Trem2*R47H strain, and progeny screened by PCR as above. A single founder line with a 601bp deletion was selected and sequenced across the region. This has continued to be bred to the B6J-APOE4/Trem2*R47H strain and was given the JAX unique ID 30673 and official strain nomenclature of B6(SJL)-Apoe^{tm1.1(APOE*4)Adpmc} *Ceacam1*^{em#1Adpmc} Tream2^{em1Adpmc}/J.

Mouse C57BL/6J wild-type *Ceacam1* sequence, with 601 bp deleted region shown in small letters (SEQ ID NO:48):

TGTCAGACTCACAGGCTCCACCCCCAGCCCACGTGGATCTGGGAGGTGCC
CTCCCCTG/ggaggagacaaagctcctttaagaaaagcagggcagatatcagggcagcctggcttagcagtagtgttggaga
agaagctagcaggcaggcagcagagacatggagctggcctcagcacatctccacaaagggcaggttccctggggaggactactgc
tcacaggtaaggagatattccttcccagtagagagcaggggagctcagagactggctgggctcttctgggagaggaaaggaacctg
agaagggacatctggcttctgcttgaagcttgacggcaacaggaagctctcagtgagtgtgaattggctagtggtgaggagtaactca
gttctttgctattgttaagtttatcgcactggctaggatatcctgaagactgaagtccacaactctgtcagggtgactctccacgtaaaaac
caaactggggcaagtaaacagaattattgcatactacactgagaaatttgcaaatacactaggctctgggacactgatttgcaaacaggt
atatgagcatgttccaggcagcagggattaagccaaaacccacctagccctgcacacaaagccctagttctatgtgtaaaacggcag/
ACCTGCGAGGGCATCTAGGTGGGGTCAGGTTGGCAAGCACTTC

### Example 8. Generation of the Il1rap KO/ApoE4/Trem2*R47H Mouse Model for Late-Onset Alzheimer's Disease

Expression of the mouse *Il1rap* gene was knocked out in a B6J-APOE4/Trem2*R47H mouse (which expresses both human APOE4 and TREM2*R47H p) using CRISPR technology.

The *Il1rap*genomic sequence was targeted using CRISPR guides on either side of exon 2, which is the first coding region:
Guide 1 (SEQ ID NO:20): TTTGATGGCGGACTTCAAATC
Guide 2 (SEQ ID NO:21): GTGAAGATGGATGCAGAATT

Founders exhibiting a deletion were bred to the B6J-APOE4/Trem2*R47H strain, and progeny screened by PCR as above. A single founder line with a 387bp deletion was selected and sequenced across the region. This has continued to be bred to the B6J-APOE4/Trem2*R47H strain and was given the JAX unique ID 30304 and official strain nomenclature of B6(SJL)-Apoe^{tm1.1(APOE*4)Adpmc} Il1rap^{em#1Adpmc} Trem2^{em1Adpmc}/J.

Mouse C57BL/6J wild-type *Il1rap*sequence, with 387 bp deleted region in Il1rap shown in small letters (SEQ ID NO:49):

CAAATTTGTTGTTGGTTCTGACAATAAAGCTTGGGAATATT/ctaaaacccttggaat
ggttcttagtctatagttatcacacattcacggtgattattattagaactcttcagggaagaagtaccctctcttactttttaaatttatgttgaca
ggatgggacttctgtggtatttgatgagtctgtccttctatgggatcctgcagagtcatgcttcgggtgagtatgtggtgctggataaatga
ctgaaattaatttaacatacaggatcctatatagtgaatttaaatgagcataatgttggttttgttccacagaattctgtagttgcatagaatca
agtgtctgaaagaaccatagaattaggggatgagagaaactagacaacttaggaaggggtctgatgagtataaacacagggacatct/
CTTCAACTTCATGCCCCACAGGCTAATTCTCTGTATTCTCATCTCAAGTACTGTGG
AGGAAATGGCAGGGAGATT

### Example 9. Validation of Genetically Modified Mouse Model of Non-Familial Late-Onset Alzheimer's Disease

### Tissue Harvesting, Protein Isolation and Sectioning

Mice were administered a lethal dose of Ketamine/Xylazine by intraperitoneal injection, and transcardially perfused with 1xPBS (phosphate buffered saline). Brains were dissected, and the right hemisphere was snap frozen for protein isolation, while the left hemisphere was fixed in 4% paraformaldehyde overnight at 4°C. The fixed hemispheres were rinsed with 1xPBS, cryoprotected in 10% sucrose, followed by 30% sucrose at 4°C, and finally embedded in OCT (optimal cutting temperature compound). Frozen brains were sectioned at 25 µm and stored at -80°C until required. Protein was extracted with Trizol Reagent (Life Technologies, Cat#15596-018) following manufacturer's guidelines. Protein pellets were resuspended in a solution of 1: 1 8M urea and 1% SDS.

### Immunofluorescence, Thioflavin S Staining, and Image Capture

Cryosections were rinsed with PBT (1xPBS with 1% TritonX-100) for 5 minutes (mins) then incubated with 500 µL of Liberate Antibody Binding Solution (L.A.B. - Polysciences Inc.) solution for 20 minutes at room temperature (RT) for antigen retrieval. Slides were then incubated overnight at 4°C in the following primary antibodies: rabbit polyclonal anti-GFAP (1:200, Dako); rabbit polyclonal anti-IBA1 (1:250, Wako); rabbit polyclonal anti-NeuN (1:100, Cell Signaling Inc); mouse monoclonal anti-non-phosphorylated neurofilament (1:200, Covance) and sheep polyclonal anti-TREM2 (1:200, RD Systems). The sheep polyclonal anti-TREM2 antibody was previously verified using Trem2 deficient mice. All antibodies were diluted in PBTB (1xPBS, 1% TritonX-100 and 1% BSA) containing 10% normal goat or donkey serum. After primary incubation, sections were washed 3 times in PBT and incubated with appropriate secondary antibodies (goat anti-rabbit Alexa Fluor 488/594/633, goat anti-mouse Alexa Fluor 488, donkey anti-sheep Alexa Fluor 594, 1: 1000 dilution, Life Technologies) for 2 hrs at RT.

All sections were then counterstained with DAPI and mounted with Aqua PolyMount (Polysciences). For Thioflavin S staining, sections stained with IBA1 and GFAP were further counterstained with 1% Thioflavin S (diluted in a 1:1 water:ethanol ratio). Slides were incubated for 8 mins at RT in 1% Thioflavin-S, washed in 80% ethanol, then 95% ethanol and finally in dH₂O and mounted. Images were taken using either the Leica SP5 confocal microscope or the Zeiss Axio Imager.Z2. For each antibody, all images were captured using identical parameters for accurate quantification.

Initial observations were performed in sections from both males and females. Quantification of cell numbers was performed on brain sections from at least 4-6 male mice, as there was no overt difference between sexes. For plaque counts, the number of plaques present in the entorhinal cortical region for each mouse was determined. For IBA1+ cells, 5 equally spaced images were captured (using 20× optical lens) of either the cortex, in the region of the entorhinal cortex, or the hippocampus, from a central brain section of each mouse. For NeuN+ cells, 5 equally spaced images were captured (using 20× optical lens). For IBA1+ cells associated with plaques, images of 8+ plaques per brain were imaged (using 20× optical lens).

Images were processed and all cells in the 20× image were counted using the cell counter plugin for ImageJ/FIJI. A single cell was determined as a DAPI stained nucleus associated with a cell specific antibody stain (e.g. IBA1or NEUN). Cell numbers in the 5 images from each mouse were totaled and then averaged across mice. Mouse number and diet were masked to the investigator for all cell counting assays.

Western blot analysis demonstrates expression of human APOE4 in B6(SJL)-*Apoe^{tm1.1(APOE*4)Adiuj} Trem2^{em1Adiuj}*/J (common name: B6J.APOE4/Trem2) mice

Brain extracts (~25 micrograms protein) were diluted in 2x Laemmli sample buffer (Bio-Rad 1610737) and run on a 4-20% gradient (BioRad mini-protean TGX 456-1096) and transferred to a nitrocellulose membrane (InVitrogen IB301001).

Blots were blocked with 5% non-fat milk powder and probed overnight at +4°C with a human APOE4-specific antibody (Novus Biologicals NBP1-49529) at 1:100 dilution. Blots were then probed with secondary antibody (Millipore goat anti-mouse HRP AP191P) at 1:30,000 dilution for 2 hours at room temperature. Blots were detected using ECL chemiluminescent reagents (GE Healthcare RPN 2109) on Amersham Hyperfilm ECL (GE Healthcare 28906838).

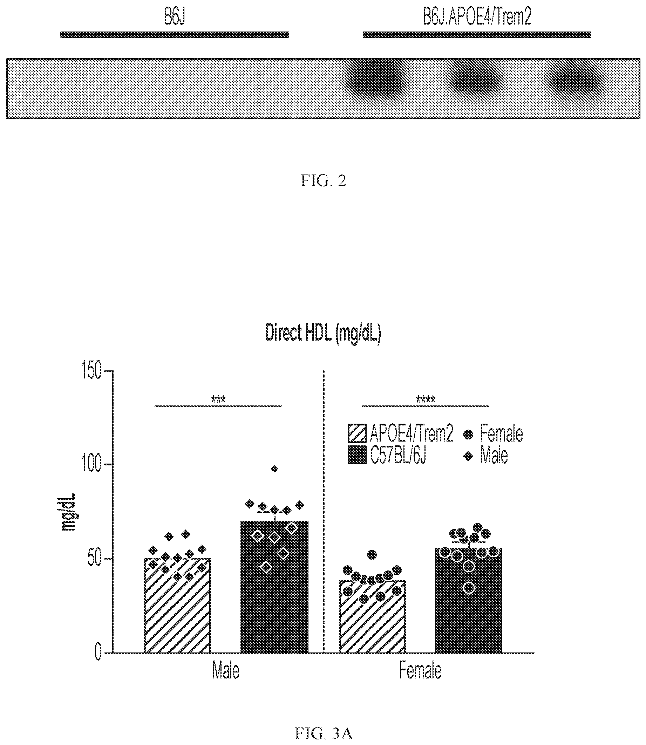

An antibody that is specific for the human APOE4 variant (Novus Biologicals NBP1-49529 at 1:100 dilution) was used to probe Western blots of about 25 micrograms of protein from C57BL/6J (abbreviated B6J) or B6J.APOE4/Trem2 brain tissue. Figure 2 is an image of a Western blot of brain tissue from B6J.APOE4/Trem2 (lanes 4-6) and control (WT (B6J); lanes 1-3) brain tissue. As shown in Figure 2, human APOE4 protein expression was detected only in the protein from B6J.APOE4/Trem2 mice.

### Blood chemistry shows altered metabolism in the B6J.APOE4/Trem2 mice

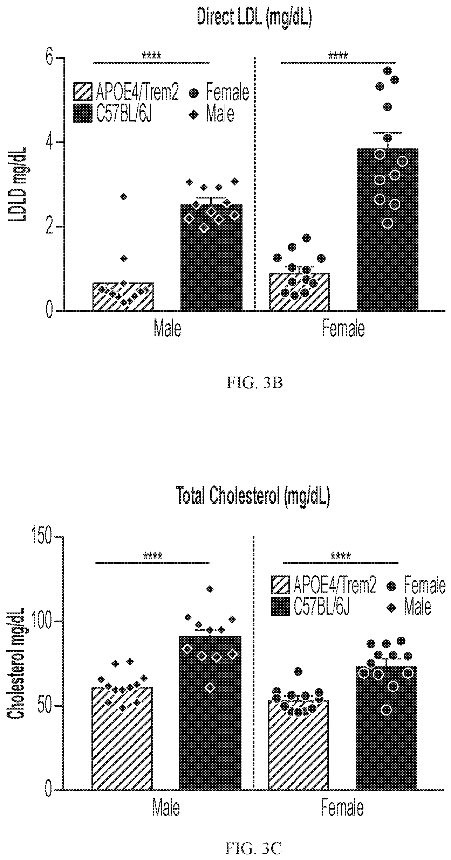

APOE variants have been shown to differentially regulate lipoprotein and cholesterol metabolism. Although the mechanisms by which the APOE4 variant increases the risk for Alzheimer's disease is not known, these effects on altered metabolism are thought to play a role. Blood samples obtained from 12 month old B6J.APOE4/Trem2 (abbreviated APOE4/Trem2 in Figures 3A, 3B and 3C) mice and 12 month old C57BL/6J control mice were assayed for high density lipoprotein (HDL), low density lipoprotein (LDL) and total cholesterol according to standard blood chemistry methods.

Blood, extracted at harvest, was stored in EDTA coated vials on ice and further centrifuged at 5000 RPM for 15 mins. The plasma was removed and aliquoted for long term storage at -80 °C. Plasma aliquots were profiled for the following: Total cholesterol (mg/dL), LDL (mg/dL), HDL (mg/dL)). High density lipoprotein cholesterol (HDL) was assayed using the Wako L-Type HDL-C kit (Wako 99100101) as per manufacturer's instructions. LDL was assayed using Beckman Coulter LDL-Cholesterol kit (Beckman Coulter OSR6196) as per manufacturer's instructions. Total cholesterol was assayed using the Beckman Coulter Cholesterol kit (Beckman Coulter OSR6116).

Results are shown in Figures 3A, 3B, and 3C, respectively. These data show that the B6J.APOE4/Trem2 mouse has APOE4-dependent alterations in cholesterol metabolism, including reduced total cholesterol and reduced levels of both LDL and HDL.

### Immunohistochemistry shows cerebrovascular leakage and inflammation in the B6J.APOE4/Trem2 mice

All mice were bred and housed in a 12/12 hours light/dark cycle. Eight month old mice were injected intraperitoneally with a lethal quantity of ketamine/xylazine according to IACUC approved procedures. Mice were perfused with 1X PBS (phosphate buffered saline) and whole brains were removed. One hemisphere was fixed in 4% paraformaldehyde overnight at +4°C. Following fixation, the tissue was rinsed in 1X PBS, incubated in 10% sucrose overnight at +4°C, then incubated in 30% sucrose overnight at 4°C. Brains were then frozen in optimal cutting temperature (OCT) compound and stored at -80°C until sectioned. Frozen brains were sectioned at 25 microns and mounted on glass slides, and stored at -80°C until required for immunofluorescence staining.

Slides were dried and post fixed in 4% PFA. Sections were subsequently immersed in diH₂O for 3mins at 37°C, then transferred and incubated in 0.5mg/mL pepsin in 0.2N HCl for 15mins at 37°C. Sections were further washed in diH₂O for 3mins, transferred to a humidified chamber, and washed in 1xPBS. Sections were incubated overnight in Rabbit anti-fibrin (1:200) and Goat anti-ColIV (anti-collagen IV) (1:40) at +4°C in 1xPBT. Slides were washed in 1xPBT and incubated in the appropriate secondary antibodies at a concentration of 1:1000 (Donkey anti-rabbit IgG 594, Donkey anti-goat 488) for 2 hours at room temperature. Tissue was counterstained with DAPI and mounted using Aqua-Poly mount (Polyscience Inc). Staining was imaged on the Zeiss AxioImager at a magnification of 20x.

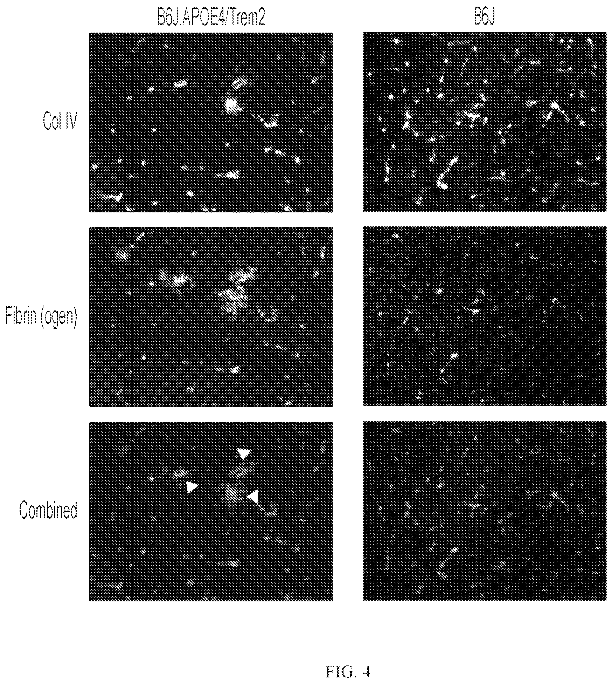

Sagittal brain sections of B6J.APOE4/Trem2 mice were immunostained for fibrinogen (fibrin) to detect cerebrovascular leakage. Representative images of B6J.APOE4/Trem2 tissue at 7-8 months of age are shown in Figure 4 along with representative images from similar sagittal sections of brain from control C57BL/6J (abbreviated B6J) mice. Arrows indicate cerebrovascular leakage as indicated by fibrin immunostaining outside of blood vessels. Blood vessels are shown by immunostaining for collagen IV (Col IV).

Vascular damage may be a key aspect of late-onset Alzheimer's disease pathology. Some existing mouse models of familial Alzheimer's disease have demonstrated cerebral amyloid angiopathy (CAA), but B6J.APOE4/Trem2 mice are the first model that does not over-express familial Alzheimer's disease mutations to show vascular deficits.

### Transcriptional analysis demonstrates AD-relevant changes in gene expression in the B6J.APOE4/Trem2 model

Gene expression analysis was carried out with the NanoString Neuropathology gene panel. This assay measures the expression of 770 neuropathology-related genes. Female mice around eight months of age were assayed for the B6J.APOE4/Trem2 strain and the C57BL/6J (abbreviated B6J) strain, with three biological replicates of each strain.

Total RNA was extracted from brain homogenates. Tissues were lysed and homogenized in TRIzol Reagent (Ambion), then RNA was isolated using the miRNeasy Mini kit (Qiagen), according to manufacturers' protocols, including the optional DNase digest step. Sample concentration and quality were assessed using the Nanodrop 2000 spectrophotometer (Thermo Scientific) and the RNA 6000 Nano LabChip assay (Agilent Technologies).

RNA was hybridized and multiplexed with NanoString probes according to manufacturer's instructions. Counts for target genes were normalized to housekeeping genes to account for variability in RNA content. Data were analyzed using Nanostring nSolver Analysis Software.

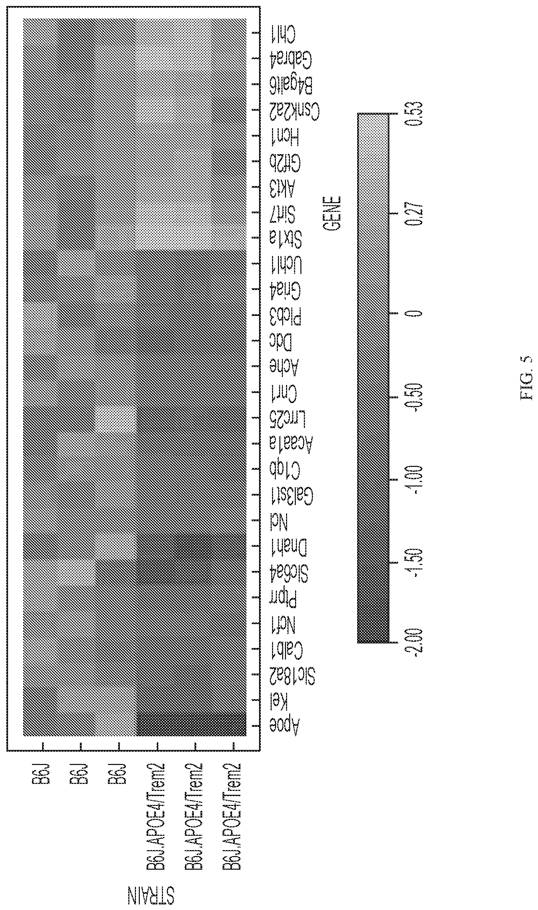

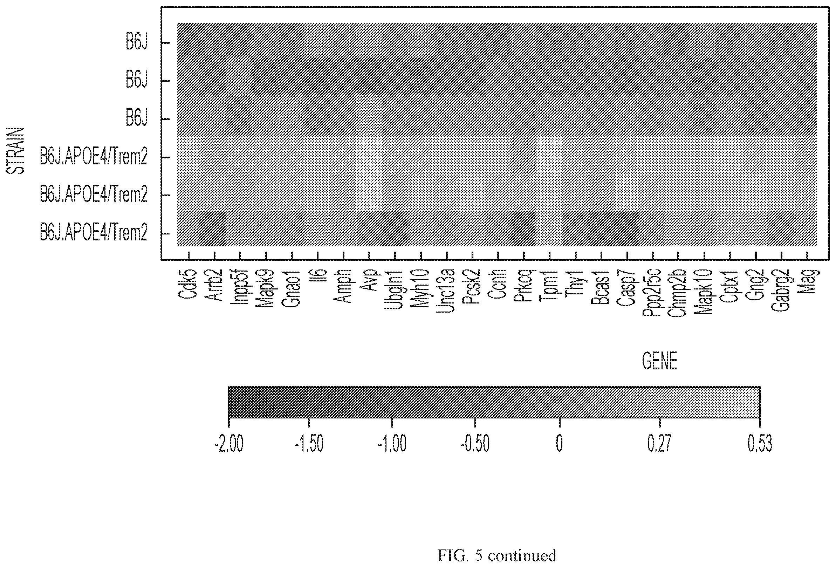

Expression of each gene in the B6J.APOE4/Trem2 strain was compared to the control C57BL/6J strain and genes with significant (p<0.05) expression were identified with a linear regression model as shown in Figure 5. Figure 5 is a gene expression heatmap of differentially-expressed genes in B6J.APOE4/Trem2 compared to C57BL/6J. Expression values are in Log2 fold change relative to the C57BL/6J average of each gene.

The probe used in this panel detects mouse Apoe but not human APOE. Because in this model mouse Apoe has been replaced by the human APOE4 variant, no Apoe expression is detected and this is shown as the most strongly down-regulated transcript.

The list of differentially-expressed genes contains multiple genes related to brain function and neurodegeneration. The Gene Ontology Biological Process and Panther Pathway annotations database was queried to identify processes and pathways represented by these genes. The relevant processes include, but are not limited to, the results in Table I which shows pathways and processes represented by the differentially-expressed genes in the B6J.APOE4/Trem2 strain. Notably, these interlinked processes and pathways include many highly relevant to neurodegeneration.

| **TABLE I** | |
|---|---|
| Process or Pathway | Genes |
| Alzheimer's disease-amyloid secretase pathway | Pcsk2, Mapk10, Mapk9, Prkcq |
| 5HT2 type receptor mediated signaling pathway | Slc18a2, Plcb2, Slc6a4, Gng2, Prkcq |
| Inflammation mediated by chemokine and cytokine signaling pathway | Akt3, Gnao1, Plcb3, Arrb2, Il6, Myh10, Gng2 |
| Modulation of chemical synaptic transmission | Cnr1, Stx1a, Arrb2, Unc13a, Cdk5, Calb1, Slc6a4, Gria4 |
| Neuron development | Cnr1, Thyl, Mapk10, Hcn1, Cdk5, Chl1, Il6, Mapk9, Myh10, Uchl1 |
| Cognition | Cnr1, Amph, Cdk5, Calb1, Chl1, Slc6a4, Chmp2b |
| Apoptosis signaling | Akt3, Mapk10, Mapk9, Casp7, Prkcq |
| Aging | Cnr1, Slc18a2, Gnao1 |

Scientific and technical terms used herein are intended to have the meanings commonly understood by those of ordinary skill in the art. Such terms are found defined and used in context in various standard references illustratively including J. Sambrook and D.W. Russell, Molecular Cloning: A Laboratory Manual, Cold Spring Harbor Laboratory Press; 3rd Ed., 2001; F.M. Ausubel, Ed., Short Protocols in Molecular Biology, Current Protocols; 5th Ed., 2002; B. Alberts et al., Molecular Biology of the Cell, 4th Ed., Garland, 2002; D.L. Nelson and M.M. Cox, Lehninger Principles of Biochemistry, 4th Ed., W.H. Freeman & Company, 2004; Herdewijn, P. (Ed.), Oligonucleotide Synthesis: Methods and Applications, Methods in Molecular Biology, Humana Press, 2004; A. Nagy, M. Gertsenstein, K. Vintersten, R. Behringer (Eds) 2002, Manipulating the Mouse Embryo: A Laboratory Manual, 3rd edition, Cold Spring Harbor Laboratory Press, ISBN-10: 0879695919; and K. Turksen (Ed.), Embryonic stem cells: methods and protocols in Methods in Molecular Biology, 2002; 185, Humana Press; Current Protocols in Stem Cell Biology, ISBN: 9780470151808.

### Example 10. Generation of Mthfr*C677T/APOE4/Trem2*R47H

This triple mutant line was generated by utilizing CRISPR/Cas9 endonuclease mediated genome editing of the Mthfr gene in B6(SJL)-Apoe^{tm1.1(APOE*4)Adiuj} Trem2^{em1Adiuj}/J mice. For the Mthfr^{em1Adiuj} allele: plasmids encoding a single guide RNA designed to introduce a R47H point mutation into the Mthfr gene and the Cas9 nuclease were introduced into the cytoplasm B6(SJL)-Apoe^{tm1.1(APOE*4)Adiuj} Trem2^{em1Adiuj}/J (Stock No. 028709)-derived fertilized eggs with well recognized pronuclei. Correctly targeted embryos were transferred to pseudopregnant females. Correctly targeted pups were identified by sequencing and PCR and further bred to B6(SJL)-Apoe^{tm1.1(APOE*4)Adiuj} Trem2^{em1Adiuj}/J (Stock No. 028709) for 2 generations to develop the colony.

### FURTHER SEQUENCES

The amino acid sequence of APOE4 p is shown along with exemplary nucleic acid sequences encoding APOE4 p.
SEQ ID NO:1: APOE4 p (317 amino acids)
SEQ ID NO:2: Exons 2, 3 and 4 of the human *APOE4* genomic DNA sequence encoding human APOE4 p including the 18 amino acid signal peptide 
ACTGGCCAATCACAGGCAGGAAGATGAAGGTTCTGTGGGCTGCGTTGCTGGTCACATTCC
TGGCAGGTATGGGGGCGGGGCTTGCTCGGTTCCCCCCGCTCCTCCCCCTCTCATCCTCACCTCAACC
TCCTGGCCCCATTCAGGCAGACCCTGGGCCCCCTCTTCTGAGGCTTCTGTGCTGCTTCCTGGCTCTG
AACAGCGATTTGACGCTCTCTGGGCCTCGGTTTCCCCCATCCTTGAGATAGGAGTTAGAAGTTGTT
TTGTTGTTGTTGTTTGTTGTTGTTGTTTTGTTTTTTTGAGATGAAGTCTCGCTCTGTCGCCCAGGCTG
GAGTGCAGTGGCGGGATCTCGGCTCACTGCAAGCTCCGCCTCCCAGGTCCACGCCATTCTCCTGCC
TCAGCCTCCCAAGTAGCTGGGACTACAGGCACATGCCACCACACCCGACTAACTTTTTTGTATTTT
CAGTAGAGACGGGGTTTCACCATGTTGGCCAGGCTGGTCTGGAACTCCTGACCTCAGGTGATCTGC
CCGTTTCGATCTCCCAAAGTGCTGGGATTACAGGCGTGAGCCACCGCACCTGGCTGGGAGTTAGAG
GTTTCTAATGCATTGCAGGCAGATAGTGAATACCAGACACGGGGCAGCTGTGATCTTTATTCTCCA
 
TCACCCCCACACAGCCCTGCCTGGGGCACACAAGGACACTCAATACATGCTTTTCCGCTGGGCGCG
GTGGCTCACCCCTGTAATCCCAGCACTTTGGGAGGCCAAGGTGGGAGGATCACTTGAGCCCAGGA
GTTCAACACCAGCCTGGGCAACATAGTGAGACCCTGTCTCTACTAAAAATACAAAAATTAGCCAG
GCATGGTGCCACACACCTGTGCTCTCAGCTACTCAGGAGGCTGAGGCAGGAGGATCGCTTGAGCC
CAGAAGGTCAAGGTTGCAGTGAACCATGTTCAGGCCGCTGCACTCCAGCCTGGGTGACAGAGCAA
GACCCTGTTTATAAATACATAATGCTTTCCAAGTGATTAAACCGACTCCCCCCTCACCCTGCCCACC
ATGGCTCCAAAGAAGCATTTGTGGAGCACCTTCTGTGTGCCCCTAGGTACTAGATGCCTGGACGGG
GTCAGAAGGACCCTGACCCACCTTGAACTTGTTCCACACAGGATGCCAGGCCAAGGTGGAGCAAG
CGGTGGAGACAGAGCCGGAGCCCGAGCTGCGCCAGCAGACCGAGTGGCAGAGCGGCCAGCGCTG
GGAACTGGCACTGGGTCGCTTTTGGGATTACCTGCGCTGGGTGCAGACACTGTCTGAGCAGGTGCA
GGAGGAGCTGCTCAGCTCCCAGGTCACCCAGGAACTGAGGTGAGTGTCCCCATCCTGGCCCTTGAC
CCTCCTGGTGGGCGGCTATACCTCCCCAGGTCCAGGTTTCATTCTGCCCCTGTCGCTAAGTCTTGGG
GGGCCTGGGTCTCTGCTGGTTCTAGCTTCCTCTTCCCATTTCTGACTCCTGGCTTTAGCTCTCTGGAT
ATCTCTCTCTCAGCTTTGTCTCTCTCTCTTCCCTTCTGACTCAGTCTCTCACACTCGTCCTGGCTCTG
TCTCTGTCCTTCCCTAGCTCTTTTATATAGAGACAGAGAGATGGGGTCTCACTGTGTTGCCCAGGCT
GGTCTTGAACTTCTGGGCTCAAGCGATCCTCCCGCCTCGGCCTCCCAAAGTGCTGGGATTAGAGGC
ATGAGCCACCTTGCCCGGCCTCCTAGCTCCTTCTTCGTCTCTGCCTCTGCCCTCTGCATCTGCTCTCT
GCATCTGTCTCTGTCTCCTTCTCTCGGCCTCTGCCCCGTTCCTTCTCTCCCTCTTGGGTCTCTCTGGC
TCATCCCCATCTCGCCCGCCCCATCCCAGCCCTTCTCCCCGCCTCCCACTGTGCGACACCCTCCCGC
CCTCTCGGCCGCAGGGCGCTGATGGACGAGACCATGAAGGAGTTGAAGGCCTACAAATCGGAACT
GGAGGAACAACTGACCCCGGTGGCGGAGGAGACGCGGGCACGGCTGTCCAAGGAGCTGCAGGCG
GCGCAGGCCCGGCTGGGCGCGGACATGGAGGACGTGCGCGGCCGCCTGGTGCAGTACCGCGGCG
AGGTGCAGGCCATGCTCGGCCAGAGCACCGAGGAGCTGCGGGTGCGCCTCGCCTCCCACCTGCGC
AAGCTGCGTAAGCGGCTCCTCCGCGATGCCGATGACCTGCAGAAGCGCCTGGCAGTGTACCAGGC
CGGGGCCCGCGAGGGCGCCGAGCGCGGCCTCAGCGCCATCCGCGAGCGCCTGGGGCCCCTGGTGG
AACAGGGCCGCGTGCGGGCCGCCACTGTGGGCTCCCTGGCCGGCCAGCCGCTACAGGAGCGGGCC
CAGGCCTGGGGCGAGCGGCTGCGCGCGCGGATGGAGGAGATGGGCAGCCGGACCCGCGACCGCC
TGGACGAGGTGAAGGAGCAGGTGGCGGAGGTGCGCGCCAAGCTGGAGGAGCAGGCCCAGCAGAT
ACGCCTGCAGGCCGAGGCCTTCCAGGCCCGCCTCAAGAGCTGGTTCGAGCCCCTGGTGGAAGACA
TGCAGCGCCAGTGGGCCGGGCTGGTGGAGAAGGTGCAGGCTGCCGTGGGCACCAGCGCCGCCCCT
GTGCCCAGCGACAATCACTGAACGCCGAAGCCTGCAGCCATGCGACCCCACGCCACCCCGTGCCT
CCTGCCTCCGCGCAGCCTGCAGCGGGAGACCCTGTCCCCGCCCCAGCCGTCCTCCTGGGGTGGACC
CTAGTTTAATAAAGATTCACCAAGTTTCACGCA
SEQ ID NO:3: TREM2 (249 amino acids, R47H mutation) 
MGPLHQFLLLLITALSQALNTTVLQGMAGQSLRVSCTYDALKHWGRHKAWCRQLGEEGPCQ
RVVSTHGVWLLAFLKKRNGSTVIADDTLAGTVTITLKNLQAGDAGLYQCQSLRGREAEVLQKVLVEV
LEDPLDDQDAGDLWVPEESSSFEGAQVEHSTSRQVSSCGSPLAYHLPPLSKESRDLLPTHLHSSPPGLRS
PEQVSCSQHPLGCGQGQAEAGNTCGQRAGLWPRCWAPTSDPHWTRRYVREF
SEQ ID NO:4: mutated mouse genomic DNA sequence encoding TREM2 (1056 nucleotides) 
ACTTCAAGGGAAAAGCAAGATCTTGCACAAGGTCCCCTCCGGCTGGCTGCTGGCAAAGGA
AAGGTGCCATGGGACCTCTCCACCAGTTTCTCCTGCTGCTGATCACAGCCCTGTCCCAAGCCCTCA
ACACCACGGTGCTGCAGGGCATGGCCGGCCAGTCCTTGAGGGTGTCATGTACTTATGACGCCTTGA
AGCACTGGGGGAGACACAAaGCaTGGTGTCGGCAGCTGGGTGAGGAGGGCCCATGCCAGCGTGTG
GTGAGCACACACGGTGTGTGGCTGCTGGCCTTCCTGAAGAAGCGGAATGGGAGCACAGTCATCGC
AGATGACACCCTTGCTGGAACCGTCACCATCACTCTGAAGAACCTCCAAGCCGGTGACGCGGGCC
TCTACCAGTGTCAGAGTCTCCGAGGCCGAGAGGCTGAGGTCCTGCAGAAAGTACTGGTGGAGGTG
CTGGAGGACCCTCTAGATGACCAAGATGCTGGAGATCTCTGGGTCCCCGAGGAGTCATCGAGTTTC
GAGGGTGCCCAAGTGGAACACAGCACCTCCAGGCAGGTTTCATCCTGTGGGTCACCTCTAGCCTAC
CACCTTCCTCCTCTTTCCAAGGAATCAAGAGACCTCCTTCCCACCCACCTCCATTCTTCTCCTCCTG
GCCTGCGTTCTCCTGAGCAAGTTTCTTGCAGCCAGCATCCTCTGGGCTGTGGCCAGGGGCAGGCAG
AAGCCGGGAACACCTGTGGTCAGAGGGCTGGACTGTGGCCAAGATGCTGGGCACCAACTTCAGAT
CCTCACTGGACCCGGAGGTACGTGAGAGAATTCTGAGTGGGAGGAGAACTACAGCTTAAGTCCAG
CCAGGAGTCAATCCAGCCTGCATGCTCTCCCCTCCTCCACCAAGACTTCTGTTTCTGCTACTTTTGC
TTCAGAGGCCGCCTCTGCCTCAAGCCCACCTATCCTGGGAGCAGGAATACTGGTGTGTACATCTGT
 
GTTGAGTGGGGAAGACAGCTGGATGGTTGTCTGTCAACTTCTGCACTTTGGACATTAAACATTCTC
CACACACCAA
SEQ ID NO:5: TREM2 (249 amino acids, mouse wild type protein, no R47H mutation) 
MGPLHQFLLLLITALSQALNTTVLQGMAGQSLRVSCTYDALKHWGRRKAWC
RQLGEEGPCQRVVSTHGVWLLAFLKKRNGSTVIADDTLAGTVTITLKNLQAGDAGL
YQCQSLRGREAEVLQKVLVEVLEDPLDDQDAGDLWVPEESSSFEGAQVEHSTSRQV
SSCGSPLAYHLPPLSKESRDLLPTHLHSSPPGLRSPEQVSCSQHPLGCGQGQAEAGNT
CGQRAGLWPRCWAPTSDPHWTRRYVREF
SEQ ID NO:6: mouse wild type genomic DNA sequence encoding *Trem2* mouse wild type protein (1056 nucleotides) 
ACTTCAAGGGAAAAGCAAGATCTTGCACAAGGTCCCCTCCGGCTGGCTGCTGGCAAAGGA
AAGGTGCCATGGGACCTCTCCACCAGTTTCTCCTGCTGCTGATCACAGCCCTGTCCCAAGCCCTCA
ACACCACGGTGCTGCAGGGCATGGCCGGCCAGTCCTTGAGGGTGTCATGTACTTATGACGCCTTGA
AGCACTGGGGGAGACGCAAGGCCTGGTGTCGGCAGCTGGGTGAGGAGGGCCCATGCCAGCGTGTG
GTGAGCACACACGGTGTGTGGCTGCTGGCCTTCCTGAAGAAGCGGAATGGGAGCACAGTCATCGC
AGATGACACCCTTGCTGGAACCGTCACCATCACTCTGAAGAACCTCCAAGCCGGTGACGCGGGCC
TCTACCAGTGTCAGAGTCTCCGAGGCCGAGAGGCTGAGGTCCTGCAGAAAGTACTGGTGGAGGTG
CTGGAGGACCCTCTAGATGACCAAGATGCTGGAGATCTCTGGGTCCCCGAGGAGTCATCGAGTTTC
GAGGGTGCCCAAGTGGAACACAGCACCTCCAGGCAGGTTTCATCCTGTGGGTCACCTCTAGCCTAC
CACCTTCCTCCTCTTTCCAAGGAATCAAGAGACCTCCTTCCCACCCACCTCCATTCTTCTCCTCCTG
GCCTGCGTTCTCCTGAGCAAGTTTCTTGCAGCCAGCATCCTCTGGGCTGTGGCCAGGGGCAGGCAG
AAGCCGGGAACACCTGTGGTCAGAGGGCTGGACTGTGGCCAAGATGCTGGGCACCAACTTCAGAT
CCTCACTGGACCCGGAGGTACGTGAGAGAATTCTGAGTGGGAGGAGAACTACAGCTTAAGTCCAG
CCAGGAGTCAATCCAGCCTGCATGCTCTCCCCTCCTCCACCAAGACTTCTGTTTCTGCTACTTTTGC
TTCAGAGGCCGCCTCTGCCTCAAGCCCACCTATCCTGGGAGCAGGAATACTGGTGTGTACATCTGT
GTTGAGTGGGGAAGACAGCTGGATGGTTGTCTGTCAACTTCTGCACTTTGGACATTAAACATTCTC
CACACACCAA
SEQ ID NO:7: Trem2 CRISPR guide
   GAAGCACTGGGGGAGACGCA
SEQ ID NO:8: Trem2 repair oligo (183 nucleotides) containing a nucleotide G>A point mutation (at nucleotide 89 in this oligo sequence) for amino acid sequence change at R47H and 2 silent mutations (lysine AAG>AAA (at nucleotide 93 in this oligo sequence) and alanine GCC>GCA (at nucleotide 96 in this oligo sequence)) into the gene to prevent re-cutting of the donor sequence in homologous directed repair: 
GCCCTCAACACCACGGTGCTGCAGGGCATGGCCGGCCAGTCCTTGAGGGTGTCATGTACT
TATGACGCCTTGAAGCACTGGGGGAGACACAAaGCaTGGTGTCGGCAGCTGGGTGAGGAGGGCCC
ATGCCAGCGTGTGGTGAGCACACACGGTGTGTGGCTGCTGGCCTTCCTGAAGAAGCGG
SEQ ID NO:9: 5' homology arm (4980 nucleotides: mouse *Apoe4* exon 1 and 757 nucleotides of mouse *Apoe4* intron 2 sequence) included in the "humanized" mouse *ApoE* construct inserted into the mouse genome. 
CCTGTATCTCAGGGTTGAGAGGGAAGAGAGTGGGGTTTCCAGATGCCCACATCCCTTGAT
ACCTAGTCCTCACCCAACTCTGCTCTCCTAGTGAACAACTGGTTGGCTACAGTAACGCTGGGCCAG
GCAGGCATGCATGCGACGTATTACCACAAAGCCAGTGACCAGGTGAGCCGGAGGGAGCGCCTAGC
TGTGGGTCCCAGGCTGGGTTTGCTGGTGCAGTTGGTATGCCCAGGAGTCCTAATGGGCCACTCACA
TGTGTGCCGGGCCCTCTCTCCCCCAGCTGCAGGTGGGTGTGGAGTTTGAGGCCAGCACCAGGATGC
AGGACACCAGTGCCTCCTTTGGGTATCAGCTGGACCTGCCCAAGGCCAACTTCCTTTTTAAAGGTA
AGGAAGGCCTCAGCTTCCCTCCTGGGGAACAGGCCAGATAAGACTCAGCTGTCTGTGGTAGAGTC
CATGGGAAATAGGGAGGTTGAACATGTGCTGGTGGAGTGGGCGGGGACTCTGTGCAGCTCCAAAT
TACTCTGGGTGGCTTGTCAAACAGTGGGGCTCCTACCCTGGCATGTTTAGTACATGTTGGGGGCCT
CGCTTTAAGCCCTCTCTTCCCATTCCCATCTAGCAAGCTCTCTGGCCCTCCCCACTCACTCTTACCTT
ACTTGGAGTTCTTCTATTGTGTGATGAAACACTATGATGGTAAACAGCTTAGGGAGCAAAGAGGTT
ATGTTGGGTTACAACTCTCAGGTGGCATGCCTACCACTGAGGAGAAGATGGGGCAGGACTCAGCA
GAAAGCTGAAGGCAAGCTGATGCAGAAGGCCTTGTGGGAGTGCTGCTTGTTGGCTTGCTCCTTATG
GCTTCCTCAGCCTGCTCTTATAGCCTCCGGACCATTAGCCCAGGGTGCCCCACTGAAAATAATCTG
GGTACTCCCACATCAATCACTTAGAAAGTGCCCTACAGGCTTGCCTGCTGCCCTAGCTAATGGAGA
CATATGTATTGAGGTTCCCTCAGCTGGATCTCTTGAGTTCAAGACCAGCCTGGTCTACAGAGCAAG
TTCTGGGACAGCCAGGGCTACCCAGAGAAACCCTGTCTCAGAAAAGAAAAAAAAGAAAAAGGTG
GGGGTGGGGTGACTAGCTTGTATAAAACTAGCCAGCACAGCAGGGAAGCTGTGTGAATAGTGACA
GCCAGGTTCAGAACCTCATTCTGCGCAGTGACATGGGTTTGTCAAGCCTGGGCGGTGTTGAGCCAC
TTAAAGGGTTGCTGTGATGGTCTGGAGTGGCCTTGGTTGTGACTTAGGACATACGGGTCCGTTGTG
GCTACCATAGAGATGGGTGGCCAGGCACATCAAGCCACCCAGTCTCTGCCCCGCTCTCGGCCCACA
GGCTCTGTGAACAGTAACTGGATCGTGGGCGCCACGCTGGAGAAGAAGCTTCCGCCCTTGCCCCTG
ACACTGTCCCTCTGCGCCTTCCTGAACCACCGCAAGAATAAGTTCCTGTGTGGCTTCGGCCTCACC
ATCGGCTGAGCCCTCCTGTCCTTCCTCTGCAGACCATCGCTGGGCCGGCTGCCCTCCCCTCCTCTCC
CTCTCTCTTGGGGTTGGGGCAGTGGGAAGGAGGGGACCTCCCATGCCCAAGGATCCCCAGCGCCA
GGGGACAGTGCCCAGGGGGCCTGGGGTCCCGGAGGGAGTCCTGGGATCTGAAGGGCATTCGATTG
TGAGCGCCCAGGCAGAGGCGCAGAGGCGGCTGTACACAGGCTCAGAAAGGAAAGACTTGATGTC
CTCCTGAGGGCAGCAGAGGAGCGCCGAGCCGCCTGTCACTTCCCCCTCCACCCCTCCATAGAAATC
ATGTTTATAAGTTATGGAAAACCGGGACATTTTACAGAAAAAAAAAAACTTAAAAAACAAAAAAT
ATACGTGGAAAAAAAAATAGGATCTGGGAGGCCTCGGTTTTCTCCAGTTGTTGACATGGTTGCCAG
CAGGCGGCGCCGAGGGTTCAGAAAGCACAGCAGCACCAAGCAGTTTAGAGAAAGCTTGGCCAGG
CATGGTCACACCCTCTGCTCTTGGGGACTTACACTGCCGGAACATCAGAGGCCTGGCCTCACCAGG
AGGGTGGCTCCAGGTCACTCGCCTCTGTGCAGTCTGGCCCCGAGAGCTGGCAGAGGACTTTGTCTC
TGTAAACAGGGTGGGGGCAGGGAGACGGGGCTCAGGAGCCTCCCTGTCCCAAAACGGGCTGAGGT
GGTAGCTTGTGCTGACTTTCTCCCAGTGGGAAGGTCAGAGGTCTCAGAAACTTCAGGAAGAAACG
GAGTTCCTGGAAGTTCAGCACAATAAGGAAAGTTACTCTGGGAGGAACGGGCCCTAGCACCTTCC
CAGGCTGAAACAGGAAATGTCAAGTTGTTTTGTTTTTTAAATTTTGTTGAAGATGTAGCTCTGGGC
CTCACGTGTCAGGCAGGTGCTGCTGGGTTCCCTATAGTGCTTTTCCGTGAGGCCTGCTCTGGGGTGT
GCGATAAGGGCCTCAGATTGGGCTCTGCATCTCACTGCTGCACCTCATGGCATCCCAAGGGAAGCA
AAGACTCACGATGAACTGGTCAGTGTCCTAGACCGCAGCACTAAGACCCTCTCTACTGCCTGGGCT
GGAGATGGGAGCTGGCCCTTAGCAACCCATGAAAATCATCTCACCACACCCAGTCCTTGCCAGTGT
TTTCTGAAGCCAAAGCTAACAGGCCTGGGCCTGGCCAGGCACCCTGTACGTACCCTTGGAGCCAG
GTGTTCCGCCTCTGCCCATCCTGCAGAATCATGTTTTGCCGTGTCTGGTGCCAAACACTGCTATGTG
GCTTCTCTCCTGCCATCAACAGCTGGGAACAGGGAACCTTGTGCAGGCAGTGCTTCTAGCAAGCTT
GCTGTGGTCTCTGAGCCCCTTGTCCTACCTGACTTCCCAGGTACAATGGCTTTCCCACTTTTTGGGG
GTTTTGTTGTTGTTGGGTTTTTTTTTGTTTTTCAAGACAGGGTTTCTCTGTGTAGTCCTGTCGGTCCT
GGAACTCACTTTGTAGACCAGGCTGGCCTCCAACTCAGAAATTCACCTGCCTCTGCCTCCAAGTGC
TGGGATTATGGGATTAAAGGCATGTGCCACCACGCCCAGCTGGCTTTTCCACTTTTTAGCCAGGAC
TTCATTCTATTACCTGAGCTCGGGATCTTCCTGCCTCAGCTTTGCATATGGCTAGCACTATAGACCC
ATGTTCCAGTGAATGACTTATGGCTTGTCTTTTTTTTTTTTTTTTTTTTTTTTTATGTGCATTAGTGTT
TTGCCTGCATGTATGCCTTCGTGAGGGTAGCAGATCTTGGTGTTACAGTTGTGAGCTGCTGTGTGG
GTGCTGCGATTTTGAACCTAGGTCCTGTGAAATGCAGTCAGTGCTCCTAACCTCTGAGTCATCTCTC
CAGCTCCTGCTCTTCTGCTTTTATGAGGAAAAAGAAAAGAGAAGTGGCTTGAGAGTGGAAAATGC
ACATGCAGGGGTGCACACCTGCAGTCCCAGCATGCTACAGCAGAGGCAGAAGGACCTTTGTGGGT
TAGAGGGCAGCCTGAGAATCTTATCTCAAAACAACTTTTTAAAATGTGCTCTGTAGGGGTAGCTCT
 
TCCCTCCCAAGGTGACACATCTGGCAATCGCCAGAAACAGATCAGGAGCATCAACGCTTGGTTTCC
CAGGGCTTGGCTTAATGTATGGCTTCAAACCCATCGGGAGCCACCACTGAACAGCTCCTGAAGGA
ACTGGAGCACGTCCCAGCCTTGGAATGGAAAGAGTTCACCTGTGGTGGAGGAATCAACAACGAGG
GATCCCAGAACAACGATCTTCACCCCAGAAGCTGAGCCTCTTAGCCCCCACCCACCCATTTCCATT
TAGGCTGCCAGCTCTTTTCTTTACAATGCACCAGACCCCGCGGGGAAAGGGAAGGAGCGGTTCTCA
GTGCCCCAGTACCAAGGCCTGGATTATTCAATGAGGTGTCCGCTCCCTTTGTTGGCGGGGGAGGGG
AGCGGGGGGTCACAAGGCATCCAAACTCCACCTCTTTCCTCTGCCCTGCTGTGAAGGGGGAGAGA
ACAACCCGCCTCGTGACAGGGGGCTGGCACAGCCCGCCCTAGCCCTGAGGAGGGGGCGGGACAG
GGGGAGTCCTATAATTGGACCGGTCTGGGATCCGATCCCCTGCTCAGACCCTGGAGGCTAAGGACT
TGTTTCGGAAGGAGCTGGTAAGACAAGCTGGGCTGGGGATTCACCCAGGGACCTTGGTAGGATGT
GGGCTGGGAACCTTGAGATCCCCCGGAGTCCAGGAAACAGGCACAAGAATTGGAAAAGCAGGCA
GCACGATAGAAGTCTTGGGGGACAAACTAAGGACTCGAGGTAACTAGCCTTTGCCAGAGTCAGAG
CAGGTGGAGGGGTTACCTCCAGGAAGGAGTACGGGACTGTCGGTGCACGGCGTACCGGCTCAACT
AGGAACCATCCTATGGCGAAAAAACTCGGGATGAGCCTTAGGCTGCTTTTATATAAATACCTACTG
ATTTCCATCACAGTCCCCAAGTAACCCGGACTGGTTTCAAACTGTGGCTCCTCATGGCTGAGCTCC
CTAAGTTCTGTAGTTGTGGGAGGGTACCACTTCGCAGGGATGGAGGACGATTAAAAATCGTGTTA
AATTAACACAAAATGGAAAGCAGGACTTAGCCGGGAAGAAAGAGGAATGTAAGCTGGACCACCC
GCTGGCCCTCTGTGAAGTGGAATTTGAACCCTAGGAGAGGGAGCTGGAATTTTTGGCAGCGGATC
CACCCCGGGGTGCCGAGATAGCGAACTCGGCAAGGGGAGGTAAACAGACCTTTGGGAAGAGCGG
GTGCTCTGTTTTGGAGATGTTTGTGATGGCTCACAGATCTGAGAAGGGAAGATGGGGTTCTCTGGG
TGGCCGGAGTCCCTCCACCCCCGCCCCCTGGTGTTCAAAGACAATTTTTCCCTCCGCAG
SEQ ID NO:10: DNA sequence including exons 2, 3 and 4 of the human *APOE4* gene and 1500 nucleotides 3'UTR of the human *APOE4* gene after exon 4 (4292 nucleotides) included in the "humanized" mouse *ApoE* construct inserted into the mouse genome. 
ACTGGCCAATCACAGGCAGGAAGATGAAGGTTCTGTGGGCTGCGTTGCTGGTCACATTCC
TGGCAGGTATGGGGGCGGGGCTTGCTCGGTTCCCCCCGCTCCTCCCCCTCTCATCCTCACCTCAACC
TCCTGGCCCCATTCAGGCAGACCCTGGGCCCCCTCTTCTGAGGCTTCTGTGCTGCTTCCTGGCTCTG
AACAGCGATTTGACGCTCTCTGGGCCTCGGTTTCCCCCATCCTTGAGATAGGAGTTAGAAGTTGTT
TTGTTGTTGTTGTTTGTTGTTGTTGTTTTGTTTTTTTGAGATGAAGTCTCGCTCTGTCGCCCAGGCTG
GAGTGCAGTGGCGGGATCTCGGCTCACTGCAAGCTCCGCCTCCCAGGTCCACGCCATTCTCCTGCC
TCAGCCTCCCAAGTAGCTGGGACTACAGGCACATGCCACCACACCCGACTAACTTTTTTGTATTTT
CAGTAGAGACGGGGTTTCACCATGTTGGCCAGGCTGGTCTGGAACTCCTGACCTCAGGTGATCTGC
CCGTTTCGATCTCCCAAAGTGCTGGGATTACAGGCGTGAGCCACCGCACCTGGCTGGGAGTTAGAG
GTTTCTAATGCATTGCAGGCAGATAGTGAATACCAGACACGGGGCAGCTGTGATCTTTATTCTCCA
TCACCCCCACACAGCCCTGCCTGGGGCACACAAGGACACTCAATACATGCTTTTCCGCTGGGCGCG
GTGGCTCACCCCTGTAATCCCAGCACTTTGGGAGGCCAAGGTGGGAGGATCACTTGAGCCCAGGA
GTTCAACACCAGCCTGGGCAACATAGTGAGACCCTGTCTCTACTAAAAATACAAAAATTAGCCAG
GCATGGTGCCACACACCTGTGCTCTCAGCTACTCAGGAGGCTGAGGCAGGAGGATCGCTTGAGCC
CAGAAGGTCAAGGTTGCAGTGAACCATGTTCAGGCCGCTGCACTCCAGCCTGGGTGACAGAGCAA
GACCCTGTTTATAAATACATAATGCTTTCCAAGTGATTAAACCGACTCCCCCCTCACCCTGCCCACC
ATGGCTCCAAAGAAGCATTTGTGGAGCACCTTCTGTGTGCCCCTAGGTACTAGATGCCTGGACGGG
GTCAGAAGGACCCTGACCCACCTTGAACTTGTTCCACACAGGATGCCAGGCCAAGGTGGAGCAAG
CGGTGGAGACAGAGCCGGAGCCCGAGCTGCGCCAGCAGACCGAGTGGCAGAGCGGCCAGCGCTG
GGAACTGGCACTGGGTCGCTTTTGGGATTACCTGCGCTGGGTGCAGACACTGTCTGAGCAGGTGCA
GGAGGAGCTGCTCAGCTCCCAGGTCACCCAGGAACTGAGGTGAGTGTCCCCATCCTGGCCCTTGAC
CCTCCTGGTGGGCGGCTATACCTCCCCAGGTCCAGGTTTCATTCTGCCCCTGTCGCTAAGTCTTGGG
GGGCCTGGGTCTCTGCTGGTTCTAGCTTCCTCTTCCCATTTCTGACTCCTGGCTTTAGCTCTCTGGAT
ATCTCTCTCTCAGCTTTGTCTCTCTCTCTTCCCTTCTGACTCAGTCTCTCACACTCGTCCTGGCTCTG
TCTCTGTCCTTCCCTAGCTCTTTTATATAGAGACAGAGAGATGGGGTCTCACTGTGTTGCCCAGGCT
GGTCTTGAACTTCTGGGCTCAAGCGATCCTCCCGCCTCGGCCTCCCAAAGTGCTGGGATTAGAGGC
ATGAGCCACCTTGCCCGGCCTCCTAGCTCCTTCTTCGTCTCTGCCTCTGCCCTCTGCATCTGCTCTCT
GCATCTGTCTCTGTCTCCTTCTCTCGGCCTCTGCCCCGTTCCTTCTCTCCCTCTTGGGTCTCTCTGGC
TCATCCCCATCTCGCCCGCCCCATCCCAGCCCTTCTCCCCGCCTCCCACTGTGCGACACCCTCCCGC
CCTCTCGGCCGCAGGGCGCTGATGGACGAGACCATGAAGGAGTTGAAGGCCTACAAATCGGAACT
GGAGGAACAACTGACCCCGGTGGCGGAGGAGACGCGGGCACGGCTGTCCAAGGAGCTGCAGGCG
GCGCAGGCCCGGCTGGGCGCGGACATGGAGGACGTGCGCGGCCGCCTGGTGCAGTACCGCGGCG
 
AGGTGCAGGCCATGCTCGGCCAGAGCACCGAGGAGCTGCGGGTGCGCCTCGCCTCCCACCTGCGC
AAGCTGCGTAAGCGGCTCCTCCGCGATGCCGATGACCTGCAGAAGCGCCTGGCAGTGTACCAGGC
CGGGGCCCGCGAGGGCGCCGAGCGCGGCCTCAGCGCCATCCGCGAGCGCCTGGGGCCCCTGGTGG
AACAGGGCCGCGTGCGGGCCGCCACTGTGGGCTCCCTGGCCGGCCAGCCGCTACAGGAGCGGGCC
CAGGCCTGGGGCGAGCGGCTGCGCGCGCGGATGGAGGAGATGGGCAGCCGGACCCGCGACCGCC
TGGACGAGGTGAAGGAGCAGGTGGCGGAGGTGCGCGCCAAGCTGGAGGAGCAGGCCCAGCAGAT
ACGCCTGCAGGCCGAGGCCTTCCAGGCCCGCCTCAAGAGCTGGTTCGAGCCCCTGGTGGAAGACA
TGCAGCGCCAGTGGGCCGGGCTGGTGGAGAAGGTGCAGGCTGCCGTGGGCACCAGCGCCGCCCCT
GTGCCCAGCGACAATCACTGAACGCCGAAGCCTGCAGCCATGCGACCCCACGCCACCCCGTGCCT
CCTGCCTCCGCGCAGCCTGCAGCGGGAGACCCTGTCCCCGCCCCAGCCGTCCTCCTGGGGTGGACC
CTAGTTTAATAAAGATTCACCAAGTTTCACGCATCTGCTGGCCTCCCCCTGTGATTTCCTCTAAGCC
CCAGCCTCAGTTTCTCTTTCTGCCCACATACTGGCCACACAATTCTCAGCCCCCTCCTCTCCATCTG
TGTCTGTGTGTATCTTTCTCTCTGCCCTTTTTTTTTTTTTTAGACGGAGTCTGGCTCTGTCACCCAGG
CTAGAGTGCAGTGGCACGATCTTGGCTCACTGCAACCTCTGCCTCTTGGGTTCAAGCGATTCTGCT
GCCTCAGTAGCTGGGATTACAGGCTCACACCACCACACCCGGCTAATTTTTGTATTTTTAGTAGAG
ACGAGCTTTCACCATGTTGGCCAGGCAGGTCTCAAACTCCTGACCAAGTGATCCACCCGCCGGCCT
CCCAAAGTGCTGAGATTACAGGCCTGAGCCACCATGCCCGGCCTCTGCCCCTCTTTCTTTTTTAGGG
GGCAGGGAAAGGTCTCACCCTGTCACCCGCCATCACAGCTCACTGCAGCCTCCACCTCCTGGACTC
AAGTGATAAGTGATCCTCCCGCCTCAGCCTTTCCAGTAGCTGAGACTACAGGCGCATACCACTAGG
ATTAATTTGGGGGGGGGGGTGGTGTGTGTGGAGATGGGGTCTGGCTTTGTTGGCCAGGCTGATGTG
GAATTCCTGGGCTCAAGCGATACTCCCACCTTGGCCTCCTGAGTAGCTGAGACTACTGGCTAGCAC
CACCACACCCAGCTTTTTATTATTATTTGTAGAGACAAGGTCTCAATATGTTGCCCAGGCTAGTCTC
AAACCCCTGGGCTCAAGAGATCCTCCGCCATCGGCCTCCCAAAGTGCTGGGATTCCAGGCATGGG
GCTCCGAGCCCGGCCTGCCCAACTTAATAATACTTGTTCCTCAGAGTTGCAACTCCAAATGACCTG
AGATTGGTGCCTTTATTCTAAGCTATTTTCATTTTTTTTCTGCTGTCATTATTCTCCCCCTTCTCTCCT
CCAGTCTTATCTGATATCTGCCTCCTTCCCACCCACCCTGCACCCCATCCCACCCCTCTGTCTCTCCC
TGTTCTCCTCAGGAGACTCTGGCTTCCTGTTTTCCTCCACTTCTATCTTTTATCTCTCCCTCCTACGG
TTTCTTTTCTTTCTCCCCGGCCTGCTTGTTTCTCCCCCAACCCCCTTCATCTGGATTTCTTCTTCTGCC
ATTCAGTTTGGTTTGAGCTCTCTGCTTCTCCGGTTCCCTCTGAGCTAGCTGTCCCTTCACCCACTGTG
AACTGGGTTTCCCTGCCCAACCCTCATTCTCTTTCTTTCTTTCTTTTTTTTTTTTTTTTTTTTTTTTTT
TTTTTGAGACAGAGTCTTGCTCTGTTGCCCAGCCTGGAGTGCAGTGGTGCAATCTTGGTTCACTGCA
ACCTCCACTTCCCAGATTCAAGCAATTCTCCTGCCTCAGCCTCCAGAGTAGCTGGGATTACAGGCG
TGTCCCACCACACCCGACTAATTTTTGTATTTTTGGTAGAGACAAGGCTTCGGCATTGTT
SEQ ID NO:11: a Frt PGKneo Frt cassette followed by an Nde1 restriction enzyme site (CATATG) (1834 nucleotides) inserted in the "humanized" mouse *ApoE* construct between nucleotides 9272 and 9273 to allow for selection prior to recombinase-mediated removal of this cassette. 
GAAGTTCCTATTCTCTAGAAAGTATAGGAACTTCAGGTCTGAAGAGGAGTTTACGTCCAG
CCAAGCTAGCTTGGCTGCAGGTCGTCGAAATTCTACCGGGTAGGGGAGGCGCTTTTCCCAAGGCA
GTCTGGAGCATGCGCTTTAGCAGCCCCGCTGGGCACTTGGCGCTACACAAGTGGCCTCTGGCCTCG
CACACATTCCACATCCACCGGTAGGCGCCAACCGGCTCCGTTCTTTGGTGGCCCCTTCGCGCCACC
TTCTACTCCTCCCCTAGTCAGGAAGTTCCCCCCCGCCCCGCAGCTCGCGTCGTGCAGGACGTGACA
AATGGAAGTAGCACGTCTCACTAGTCTCGTGCAGATGGACAGCACCGCTGAGCAATGGAAGCGGG
TAGGCCTTTGGGGCAGCGGCCAATAGCAGCTTTGCTCCTTCGCTTTCTGGGCTCAGAGGCTGGGAA
GGGGTGGGTCCGGGGGCGGGCTCAGGGGCGGGCTCAGGGGCGGGGCGGGCGCCCGAAGGTCCTC
CGGAGGCCCGGCATTCTGCACGCTTCAAAAGCGCACGTCTGCCGCGCTGTTCTCCTCTTCCTCATCT
CCGGGCCTTTCGACCTGCAGCCTGTTGACAATTAATCATCGGCATAGTATATCGGCATAGTATAAT
ACGACAAGGTGAGGAACTAAACCATGGGATCGGCCATTGAACAAGATGGATTGCACGCAGGTTCT
CCGGCCGCTTGGGTGGAGAGGCTATTCGGCTATGACTGGGCACAACAGACAATCGGCTGCTCTGA
TGCCGCCGTGTTCCGGCTGTCAGCGCAGGGGCGCCCGGTTCTTTTTGTCAAGACCGACCTGTCCGG
TGCCCTGAATGAACTGCAGGACGAGGCAGCGCGGCTATCGTGGCTGGCCACGACGGGCGTTCCTT
GCGCAGCTGTGCTCGACGTTGTCACTGAAGCGGGAAGGGACTGGCTGCTATTGGGCGAAGTGCCG
GGGCAGGATCTCCTGTCATCTCACCTTGCTCCTGCCGAGAAAGTATCCATCATGGCTGATGCAATG
CGGCGGCTGCATACGCTTGATCCGGCTACCTGCCCATTCGACCACCAAGCGAAACATCGCATCGAG
CGAGCACGTACTCGGATGGAAGCCGGTCTTGTCGATCAGGATGATCTGGACGAAGAGCATCAGGG
 
GCTCGCGCCAGCCGAACTGTTCGCCAGGCTCAAGGCGCGCATGCCCGACGGCGATGATCTCGTCGT
GACCCATGGCGATGCCTGCTTGCCGAATATCATGGTGGAAAATGGCCGCTTTTCTGGATTCATCGA
CTGTGGCCGGCTGGGTGTGGCGGACCGCTATCAGGACATAGCGTTGGCTACCCGTGATATTGCTGA
AGAGCTTGGCGGCGAATGGGCTGACCGCTTCCTCGTGCTTTACGGTATCGCCGCTCCCGATTCGCA
GCGCATCGCCTTCTATCGCCTTCTTGACGAGTTCTTCTGAGGGGATCAATTCTCTAGAGCTCGCTGA
TCAGCCTCGACTGTGCCTTCTAGTTGCCAGCCATCTGTTGTTTGCCCCTCCCCCGTGCCTTCCTTGA
CCCTGGAAGGTGCCACTCCCACTGTCCTTTCCTAATAAAATGAGGAAATTGCATCGCATTGTCTGA
GTAGGTGTCATTCTATTCTGGGGGGTGGGGTGGGGCAGGACAGCAAGGGGGAGGATTGGGAAGAC
AATAGCAGGCATGCTGGGGATGCGGTGGGCTCTATGGCTTCTGAGGCGGAAAGAACCAGCTGGGG
CTCGACTAGAGCTTGCGGAACCCTTCGAAGTTCCTATTCTCTAGAAAGTATAGGAACTTCCATATG
SEQ ID NO:12: 3' homology arm (5166 nucleotides) included in the "humanized" mouse *ApoE* construct inserted into the mouse genome. 
AAACCTGATGGAGAAGATACAGGCCTCTGTGGCTACCAACCCCATCATCACCCCAGTGGC
CCAGGAGAATCAATGAGTATCCTTCTCCTGTCCTGCAACAACATCCATATCCAGCCAGGTGGCCCT
GTCTCAAGCACCTCTCTGGCCCTCTGGTGGCCCTTGCTTAATAAAGATTCTCCGAGCACATTCTGAG
TCTCTGTGAGTGATTCCAATCAGCTTCAGCCTCAGTTTATTGTTTTTTGCCTTACCTAGCACACATTC
CATGGCCCTGTCACTATCTGTAGAGGGAGGTGGTTTTGCAGCAATAGAAATGAAGCCTAGGACCT
AGCAACATAAAAGAACAAGTGATCTACCACTGAGCCACGCCCACAGCCCCTCACTGGGGGATTCT
AGGCAGGGGCTCTACCACTGAGCCACCCGCAGCCCCTCACTGGGGAATCATATCTACCACTGAGTC
ACGCCCCTCCAGCCCCTCACTACGGGAATTCTAGTCAGTAGCTCTACCACTGAGCCACACCCACAG
CCTCTGGGGCTCTTCACCGCCCCCTACCCCTGGATTCTAGGCATGGGCTCATTTTATTTATTTATTT
ATTTAAGATTTGTTTATCTTATGTATAAGGTACACTGCAGCTGTCTTCAGGCGTCAGGTCCCATTAC
AGATGGTTGTGAGCCACCATGTGGTTGCTGGGAATTGAACTCAGGACTTATAGAAGAGTAGTCAG
TGCTCTTAACTGCTGAGCCATCTCTCCAGCACCCAGTACAGGCTCTTCTATTTAGCTATATCCACCC
TTCTTTTTAGTCTGAAATAGGATCTCAACTGATTTTCCTTGCACTCCCTCTAGCCTAGTTTGGTCTTG
AATATTTGAATCTTTGTTTTCAAATCAATCTCTACAGGAACTGAGAAAGGCATGTACCACTTCATG
TGGGTCAGTTGGGCTACTTTTCCCAACTTCCCAAGCACCCACTGCACAGCTATGCCTTGAATCAAT
CAACATGTAAGAGACCAGGGTCGCCAGGCGGTGTTTACTTTTCTGGTTGTCTTATCTCTCCTCCTCC
GCTCTAGTCTTATCTGACACCCTCTCCTTGCCTTGTCTCTCCTCTTTTTCCCTTCTAGGCTTCCTTTTC
TGGCTTCCTGTTTTCCTGATCCTCTGTTATCTCACCCTCCCGCGGTTTCTTTTGCTCTGGGCCTTTGG
TTGGCGGTTTCTACGGTTTCTACGTGGCTTTTGGAACCTCAGCCTTTCTCCCTTGCTCTGAAGTTAG
CTGGATCTTTGCTCCCTCTGGGTCATGGGGCCTTAGCCCCATTCTTTCTCCCCTGCCTGTGCTGCAT
GCCTTTTGACTTTCCCAGCAAGTGTATGGAGAGTGAGTTCAGGCTGGGGACACAAAACCATTCTCC
CCATGTCCTGGTACTCAAAGGGGTCATGGTGGAAGCTGGAGAGCCCCTGGCTGGCTTCTCCTCCCA
CCCCCTCAGTTCTCAGGGACTCAGCAGGGCTCCCACTAACAGGGGCAGGCTAGGGCTTGAGCTGT
GTCTTGGGTCGGGCCAAGGCTTCAGAACTCAACTGCCTCTGGCACACCCCGAGCCTGCAGCTTTTC
CTGGCATCAAACTCAGCATTATCTGGAGGCAGGCACCACTCCCACACATGACTCATTAGGCCCAAT
GAGAAGATGGGTCGGTGGGCTAGTGACAGGGCCCTAGACAGCCACACTTCATGCCCAGGGGCTAG
GAGCACACCCCCAGAGCCTCACTACTCTCAAATATCAAGATGAGGGGAGAGTATAGAGGGAATGT
CTCCCTCTGAAGACCTGAATTATTGTGATGTTATTTGAGCCACGCTGGCCTTGAACTCTGAACCCTC
CTGCCTCATCTTCAAAGGCTGGAATTACAGGTTCGCGCCACTAGGCCTATCTGAGAACTTAGTTAA
TTCTACAGAAGAGAGTTTGTTTTCACTGGTTTGAAACGAGGCCTCGTGTACCCAGAGCTACTCTTTT
GCCAACTCACTGTAGAGCCAAGGATGACCTTGAACTCTGGTCTTCCTGGCTCCGAGTCCTCGGTAC
TGGGTAGACAATGTGTGATCCTAACCCCCAGTTTTATGTGGTGCTATACAGATGGCATCCAGGGTT
TCCTGCATGCTGGACAGGTCATCAACCAACCGAGCCACATCCTCCTCATCATCATCACTTTGTTGTT
GGTTTTCAATCCAGAGCTCATGGAGCCCAAGCTGGCCTCAATCTATATAACCAAGACTGTGCCTGG
TCTTCCTGTCTCTACCTAACAAATGATGGTGGGATGCAGGGATGTACAACCAGGAGCAGAGCCTTA
CTTCTAAACGAAGAAGGAACCCCACCTCCCTGTGGGCAGACCTGGAGGTGGGGCAAGGACCACAG
AAAGACATTACAGAACTAGGATCGTGAAGGAAGTCATGGAGGCCAGCCTGTTGTACAGAGTGAGT
TCCAGGACAGCCAGGGCTACACAGAGGAAAACAAGAAGGAAAAAAAAAAAAAAAAAAAGAACT
GAATGACTAGCAGTTTTAGAACCTTAGCTGTGTGCTTCCCGTACCCTACCTGTGGCACAGGAAGCC
CTTGTTGCTAAGAGTAGGATGACACTGGAAGCACCAAGCACTGGGCAGAGTAAGGGGGAAATCTG
TTACATTATTCAGATGGGCGCTTGAGGACTGAGCAGGACTCCTTTCAAGAAGCAAAAGGGTCAGG
CCTGGTGCCCACAGGGACTGGAAGCTAGCTAGCGCCGAGTTGGCCCCAGAGATGTCGCAACCCAG
CCAGGGTCGAGAATCTGTTCCACCCCTTTCACAACCATCCCCCTGTTGTCGTCGTCCCCACCCCCAC
CCCCGCCCCAACTGACACGTGGGTTGCAGGGGCACCAGGCCAGCCAACCTAGAGTCTGGGCCCCT
TAGCCACCAGCTGCCAGGGGGTCACTGTCGGTCAATGACAGCTCTGGGGGAGGGGGGGAGAAGG
 
GCCTTGGACTCTAGCCTGAGAGAAAGGATGTTGTGGAAGGAGGGGGCAGGGAGGCAAGTTTAGG
GCCTGCAGGGGCCTAGGAGGCCCCACAAGACCTAAAAACGGGGGAAGGGGGGTTGTGCCAGTTA
GGGGACACTATGGAGCTCTGCAAGCTAAGAGGCTCTTAGCTTCTTTGCAGTTTTGACTAGCTGAAG
AGGCAACTTCTAAGGAAGGGAGATGAGGGGATGCCAAGATCCCAGAGAGCATCCGAGGAGGTCT
GAGGGTGTGCAGATGCAAAGGCATTGGAGGTGAAGGGAGCCAGGGTGCAGCATCCGGGACAGAA
TGTGAGCTGAGGCTCCTGGTCAAGGAGAAGGTAGAGAAGAGCTAACCTGAGGATGCAGTGTGAGC
TAGGGGTGAGATGGGGGTGAGGGGAGTTGTTTGTAAGACCCCCCCCCTTAAGGTGGGACAGCCTC
AGAGAGAGAGTGAGTAGGCAGAGCAGCCAAGGCTGGGTGGAGATCAATGAGATTTGGAAGTGAG
GATGCTGACCAGTGACGAGCCTTGGGCCTAAATAAAAAGCCTGGATGTGGGATCTGCCTCCCAGT
ATCGACCAGAATGACAGAAGAGAGTGGGCTGGTTGTGTTGGTGTTGGTGCATGCCTGTAATCCCA
GCAACTCACTAGAGAGGCAGAGGCAGGAGCATCACAAATTCCAGGCCAGCCAAAAAGCCTATGTA
AAAAAAAAAAAAAGAGAAAAAAGAAAAAAGAAAAAGTAGGTGGACACAGAGACAAGGGGGAGG
CTCAGGGGGAGGGCGACCAGCTTGCTCCGCCCCCTCCCCAACCGGTTAAACCTCTGTGCAGGATCC
TCCCACCGCCATGGGCCTCCTGAGAGATCCTTAGATCCAGGTTAGTGCATAGGAAAGTGTCCCCCC
ACTACCTACAGCTAAGGGATTGGGGTGGTGGGATCATGGTGGAGGGCGGTGGTGAATACTAGCGA
TGTCCCCCGCTACCCGTGCGTCTGCCTCCAGGGTGCCCCTCCAACCAGGATGAGGCTCTTCATCGC
TCTTCCTGTCCTGATTGTGGTCGTAGCCATGACCTTGGAAGGTAAGAAAGAGCCTTGGAAGGTAAG
AAAGAGGCTTGGAAGTGTGAAGTTGGCCTTGTGCCTGCGGCCCAGGCTTAGAAGACCCTCGAGGA
GGGCTCTGAGGTCCCTTTCTGTGTCATCATTCCACTACCGCCCTCCCATCGTCCCCCATCCCACCTG
CCAGGTGCCTTATTTTTGTGTCAAAGTGGGTGCTGAAGGAGGCAACTCTGTCCAGAAAAGACGCA
GTAACCAATGACCTAGGATACCACCCTTTGGAATTGGCTAATCTTCCTAGAAGGGGCGGAGCGTA
AAAACAAGGAGGTGAGAGGTGCAGTAAAATCAAGTGTCCAATACCCTCCCCCATGCTAATGAGTT
TGCTCGCAACCCTCTCGCGGCAGGCCCAGCCCCCGCCCAGGCGGCCCCGGATTTGTCCGGAACATT
GGAGAGCATACCGGATAAACTGAAGGAGTTTGGGAACACTTTGGAAGACAAGGCCCGGGCAGCC
ATTGAACATATCAAACAGAAGGAAATTTTGACCAAGACCCGGTTAGGACCTTTCAGGGCACGGGC
GGGTGGTGTGTGTGTGTGTGCGTGCGCGCGCGCCCGTGTGTAAAAGCCCTAGCAGACAGTTCCACA
CTGACACAATGGGGAAACTGCACCAGAGTGTTTGACACTTTCCCTGAAGTCATAGAACTGTATCTG
AAGTCATTGAACTGGATGTCAAAGTGCTCGTAGTGTGGAGACAGGCGGTGTAACTCCCAGCCAAC
TGTTAGAGATGTTTCCAAGTCCTAGTGAAGGGCCAAGCTAGGCGGCTGACTGGTTAGGACAGACC
CTGACCCCTCCCTGTGTACTCTTGAGACAGGTCATGACTTGAGTCTCCGAGACAGAGAAAGAATGT
AGGAGAGTTGGGGCAGGGGCACAGCAGAAGCTGTACAGGCCTGGGCTCTGCAGTTTACACTGGCC
AAAGAGAGATTAGGGCATGCTGGGTTAAGAACTAGCCAGGCAGTGGTGGCGCACACCTTTGATCC
CAGTATTCATGAGGCAGAGGCAGGCAGATCTCTTTGAGGCCAGCCTGATTGGAGCTAGTTCTAGG
AAAGCCAGGGCTACACAGAGAAACCCTGTCTCAACACCACCACCACCCCCC
SEQ ID NO:13: wild-type amino acid sequence-mouse APOE 
MKALWAVLLVTLLTGCLAEGEPEVTDQLEWQSNQPWEQALNRFWDYLRWVQTLSDQVQEE
LQSSQVTQELTALMEDTMTEVKAYKKELEEQLGPVAEETRARLGKEVQAAQARLGADMEDLRNRLG
QYRNEVHTMLGQSTEEIRARLSTHLRKMRKRLMRDAEDLQKRLAVYKAGAREGAERGVSAIRERLGP
LVEQGRQRTANLGAGAAQPLRDRAQAFGDRIRGRLEEVGNQARDRLEEVREHMEEVRSKMEEQTQQI
RLQAEIFQARLKGWFEPIVEDMHRQWANLMEKIQASVATNPIITPVAQENQ
SEQ ID NO:14: amino acid sequence-human APOE3
SEQ ID NO:15: APOE4 p (299 amino acids) 
KVEQAVETEPEPELRQQTEWQSGQRWELALGRFWDYLRWVQTLSEQVQEELLSSQVTQELR
ALMDETMKELKAYKSELEEQLTPVAEETRARLSKELQAAQARLGADMEDVRGRLVQYRGEVQAMLG
QSTEELRVRLASHLRKLRKRLLRDADDLQKRLAVYQAGAREGAERGLSAIRERLGPLVEQGRVRAAT
VGSLAGQPLQERAQAWGERLRARMEEMGSRTRDRLDEVKEQVAEVRAKLEEQAQQIRLQAEAFQAR
LKSWFEPLVEDMQRQWAGLVEKVQAAVGTSAAPVPSDNH
SEQ ID NO: 49: *Mthfr*A262V* g 
ATGAACCATCAAAAAGCCAAGGTCCTCCCAGCTGGGCACTGTTATCCATCCCTGGGGATG
TGGGCCTCAGAGGCTGGCTGCGTGAGGCTTTCTGTGCCACCCTCTATCAGCAGGAATCCAGCCATG
GTGAACGAGGCCAGAGGAAGTGGCAGTCCCAACCCGCGATCTGAGGGCAGCAGCAGTGGCAGCG
AGAGTTCCAAGGACAGTTCAAGATGTTCCACCCCCAGCCTGGACCCAGAGCGGCACGAGAGACTC
CGGGAGAAGATGAGGCGCAGAATGGACTCTGGTGACAAGTGGTTCTCCCTGGAGTTCTTCCCCCCT
CGAACTGCTGAGGGAGCTGTTAACCTCATCTCCAGGTTTGACCGGATGGCAGCAGGGGGCCCCCTC
TTCGTAGATGTTACCTGGCACCCAGCTGGAGACCCTGGCTCAGACAAGGAGACCTCCTCCATGATG
ATCGCCAGCACAGCAGTAAACTACTGCGGCTTGGAAACCATCCTGCATATGACCTGCTGCCAGCA
GCGCCCGGAGGAGATCACAGGCCATCTGCACAGAGCCAAGCAGCTGGGCCTGAAGAACATAATG
GCGCTGAGGGGAGACCCTGTAGGTGACCACTGGGAAGCAGAGGAAGGAGGCTTCAGCTATGCCAC
AGACCTGGTGAAGCACATCCGGACCGAGTTTGCTGACTATTTTGACATCTGTGTGGCAGGTTACCC
CAGAGGCCACCCCGATGCAGAGAGCTTCGAGGATGACCTGAAGCATTTGAAGGAGAAGGTATCTG
CAGGCGTCGACTTCATTATCACTCAGCTCTTCTTTGAGGCCAGCACCTTCTTCAGCTTTGTGAAGGC
CTGCACAGAGATAGGCATCTCTTGCCCTATCCTGCCTGGGATCTTCCCTATTCAGGGCTACACTTCC
CTTCGGCAGCTTGTAAAACTGTCCAAGCTGGAGGTGCCACAGAAGATCAAGGATGTAATTGAGCC
CATCAAAGACAACGATGCTGCCATCCGCAACTACGGCATTGAGCTGGCTGTAAGCCTGTGCCGGG
AGCTGCTGGACAGTGGCTTGGTGCCAGGCCTCCACTTCTATACCCTCAACCGCGAGGTGGCCACCA
TGGAGGTGCTAAAGCAACTGGGCATGTGGACCGAGGACCCCAGGCGTCCCTTGCCCTGGGCTCTC
AGTGCGCATCCCAAGCGCCGGGAGGAAGATGTCCGTCCCATCTTCTGGGCCTCCAGACCAAAGAG
CTACATCTACCGCACACAGGACTGGGATGAGTTTCCTAACGGCCGCTGGGGTAATTCTTCCTCACC
AGCCTTTGGGGAGCTGAAAGACTACTACCTCTTCTACCTGAAAAGCAAGTCCCCCAGGGAGGAGC
TGCTGAAGATGTGGGGCGAGGAGCTCACCAGCGAAGAGAGTGTCTTTGAAGTCTTTGAACACTAC
CTCTCTGGAGAGCCGAATCGCCATGGCTACAGAGTAACCTGCCTGCCCTGGAACGATGAACCCCTG
GCAGCGGAAACCAGCCTGATGAAGGAAGAGCTGCTCCGCGTGAACAGGCTGGGCATCCTCACCAT
CAACTCTCAGCCCAACATCAACGCAAAACCATCCTCAGACCCTGTTGTGGGCTGGGGCCCCAGTGG
GGGTTATGTCTTCCAGAAGGCCTACCTCGAATTCTTCACCTCCCGTGAAACTGTGGAGGCGCTTCT
GCAGGTGCTGAAGACATACGAGCTGCGGGTCAACTACCACATCGTGGACGTGAAGGGAGAGAAC
ATCACTAATGCCCCTGAGCTGCAGCCCAATGCCGTGACGTGGGGCATCTTCCCGGGTCGAGAGATC
ATCCAGCCTACTGTGGTGGACCCCATCAGCTTCATGTTCTGGAAGGATGAGGCCTTTGCCCTGTGG
ATCGAGCAGTGGGGCAAGCTATACGAGGAGGAGTCGCCATCCCGCATGATCATCCAATACATCCA
TGACAACTATTTCCTGGTCAACCTGGTGGACAACGAGTTCCCGCTGGACAGCTGCCTGTGGCAGGT
GGTGGAGGACACGTTTGAGCTGCTCAACAGGCATCCCACGGAGAGAGAGACACAGGCTCCATGA
SEQ ID NO: 50: Mthfr*A262V p

MNHQKAKVLPAGHCYPSLGMWASEAGCVRLSVPPSISRNPAMVNEARGSGSPNPRSEGSSSG
SESSKDSSRCSTPSLDPERHERLREKMRRRMDSGDKWFSLEFFPPRTAEGAVNLISRFDRMAAGGPLFV
DVTWHPAGDPGSDKETSSMMIASTAVNYCGLETILHMTCCQQRPEEITGHLHRAKQLGLKNIMALRG
DPVGDHWEAEEGGFSYATDLVKHIRTEFADYFDICVAGYPRGHPDAESFEDDLKHLKEKVSAGVDFIIT
QLFFEASTFFSFVKACTEIGISCPILPGIFPIQGYTSLRQLVKLSKLEVPQKIKDVIEPIKDNDAAIRNYGIEL
AVSLCRELLDSGLVPGLHFYTLNREVATMEVLKQLGMWTEDPRRPLPWALSAHPKRREEDVRPIFWA
SRPKSYIYRTQDWDEFPNGRWGNSSSPAFGELKDYYLFYLKSKSPREELLKMWGEELTSEESVFEVFEH
YLSGEPNRHGYRVTCLPWNDEPLAAETSLMKEELLRVNRLGILTINSQPNINAKPSSDPVVGWGPSGGY
VFQKAYLEFFTSRETVEALLQVLKTYELRVNYHIVDVKGENITNAPELQPNAVTWGIFPGREIIQPTVVD
PISFMFWKDEAFALWIEQWGKLYEEESPSRMIIQYIHDNYFLVNLVDNEFPLDSCLWQVVEDTFELLNR
HPTERETQAP

The genetically modified mice and methods of use described herein are presently representative of preferred embodiments, exemplary, and not intended as limitations on the scope of the disclosure. Changes therein and other uses will occur to those skilled in the art.

The indefinite articles "a" and "an," as used herein in the specification and in the claims, unless clearly indicated to the contrary, should be understood to mean "at least one."

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03.

The terms "about" and "substantially" preceding a numerical value mean ±10% of the recited numerical value.

Where a range of values is provided, each value between the upper and lower ends of the range are specifically contemplated and described herein.

## Claims

1. A genetically modified mouse model of late-onset Alzheimer's disease comprising
(a) a genomic nucleic acid encoding human APOE4,
(b) a genomic nucleic acid encoding mouse TREM2 modified to include a R47H substitution, and
(c) a genomic nucleic acid encoding mouse APP modified to include G601R, F606Y, and R609H substitutions.

2. The genetically modified mouse of claim 1, wherein the genomic nucleic acid encoding human APOE4 comprises a nucleotide sequence having at least 95% identity to the nucleotide sequence of SEQ ID NO:2, and/or the human APOE4 comprises an amino acid sequence having at least 95% identity to the amino acid sequence of SEQ ID NO:1,
optionally wherein the genomic nucleic acid encoding human APOE4 comprises the nucleotide sequence of SEQ ID NO:2, and/or the human APOE4 comprises the amino acid sequence of SEQ ID NO:1.

3. The genetically modified mouse of claims 1 or 2, wherein the genetically modified mouse expresses the human APOE4 and/or does not express a detectable level of mouse APOE4.

4. The genetically modified mouse of any one of claims 1-3, wherein the genomic nucleic acid encoding mouse TREM2 modified to include a R47H substitution comprises a nucleotide sequence having at least 95% identity to the nucleotide sequence of SEQ ID NO:4, and/or the mouse TREM2 modified to include a R47H substitution comprises an amino acid sequence having at least 95% identity to the amino acid sequence of SEQ ID NO:3, optionally wherein the genomic nucleic acid encoding mouse TREM2 modified to include a R47H substitution comprises the nucleotide sequence of SEQ ID NO:4, and/or the mouse TREM2 modified to include a R47H substitution comprises the amino acid sequence of SEQ ID NO:3.

5. The genetically modified mouse of any one of claims 1-4, wherein the genetically modified mouse expresses the mouse TREM2 modified to include a R47H substitution.

6. The genetically modified mouse of any one of claims 1-5, wherein the genomic nucleic acid encoding mouse APP modified to include G601R, F606Y, and R609H substitutions comprises a nucleotide sequence that shares at least 95% identity with the nucleotide sequence of SEQ ID NO:23, and/or the mouse APP modified to include G601R, F606Y, and R609H substitutions comprises an amino acid sequence that shares at least 95% identity with the amino acid sequence of SEQ ID NO:24.

7. The genetically modified mouse of any one of claims 1-6, wherein the genomic nucleic acid encoding mouse APP modified to include G601R, F606Y, and R609H substitutions comprises the nucleotide sequence of SEQ ID NO:23, and/or the mouse APP modified to include G601R, F606Y, and R609H substitutions comprises the amino acid sequence of SEQ ID NO:24.

8. The genetically modified mouse of any one of claims 1-7, wherein the genetically modified mouse expresses the mouse APP modified to include G601R, F606Y, and R609H substitutions.

9. The genetically modified mouse of any one of claim 1-8, wherein total cholesterol level, low density lipoprotein (LDL) level, and/or high density lipoprotein (HDL) level is reduced in the genetically modified mouse, relative to a control mouse,
wherein the control mouse is a wild-type C57BL/6J mouse,
optionally wherein the total cholesterol level in the genetically modified mouse is
reduced by at least 10% or at least 20%, relative to the control mouse; and/or
wherein the LDL level in the genetically modified mouse is reduced by at least 30%, at least 40%, or at least 50%, relative to the control mouse; and/or
wherein the HDL level in the genetically modified mouse is reduced by at least 10%, relative to the control mouse.

10. The genetically modified mouse of any one of claims 1-9, wherein the genetically modified mouse exhibits signs of cerebrovascular leakage.

11. The genetically modified mouse of any one of claims 1-10, wherein at least one of the following genes is differentially expressed in the genetically modified mouse: Pcsk2, Mapk10, Mapk9, Prkcq, Slc18a2, Plcb2, Slc6a4, Gng2, Akt3, Gnao1, Plcb3, Arrb2, Il6, Myh10, Cnr1, Stx1a, Unc13a, Cdk5, Calb1, Gria4, Thy1, Hcn1, Chl1, Uchl1, Amph, Chmp2b, and Casp7.

12. A method of producing a genetically modified mouse model of late-onset Alzheimer's disease, the method comprising:
(a) (i) providing a genetically modified mouse comprising a genomic nucleic acid encoding human APOE4 and a genomic nucleic acid encoding mouse TREM2 modified to include a R47H substitution; or (ii) introducing into a mouse a genomic nucleic acid encoding human APOE4 and a genomic nucleic acid encoding mouse TREM2 modified to include a R47H substitution; and
(b) introducing into the genetically modified mouse a genomic nucleic acid encoding mouse APP modified to include G601R, F606Y, and R609H substitutions.

13. A method of screening for a compound for use in the treatment of late-onset Alzheimer's disease, comprising:
(i) administering a compound to a genetically modified mouse of any one of claims 1 to 10; and
(ii) assessing the mouse for an effect of the compound on a symptom associated with Alzheimer's disease.

## Patentansprüche

1. Genetisch modifiziertes Mausmodell für spät auftretende Alzheimer-Krankheit, umfassend
(a) eine genomische Nukleinsäure, die menschliches APOE4 kodiert,
(b) eine genomische Nukleinsäure, die das Maus-TREM2 kodiert, die so modifiziert ist, dass sie die Substitution R47H einschließt und
(c) eine genomische Nukleinsäure, die das Maus-APP kodiert, die so modifiziert ist, dass sie die Substitutionen G601R, F606Y, und R609H einschließt.

2. Genetisch modifizierte Maus nach Anspruch 1, wobei die genomische Nukleinsäure, die menschliches APOE4 kodiert, eine Nukleotidsequenz umfasst, die mindestens 95 % Identität mit der Nukleotidsequenz von SEQ-ID-NR:2 aufweist, und/oder das menschliche APOE4 eine Aminosäuresequenz umfasst, die mindestens 95 % Identität mit der Aminosäuresequenz von SEQ-ID-NR: 1 aufweist, wobei optional die genomische Nukleinsäure, die menschliches APOE4 kodiert, die Nukleotidsequenz von SEQ-ID-NR:2 umfasst und/oder das menschliche APOE4 die Aminosäuresequenz von SEQ-ID-NR:1 umfasst.

3. Genetisch modifizierte Maus nach Anspruch 1 oder 2, wobei die genetisch modifizierte Maus das menschliche APOE4 ausdrückt und/oder kein nachweisbares Niveau an Maus-APOE4 ausdrückt.

4. Genetisch modifizierte Maus nach einem der Ansprüche 1-3, wobei die genomische Nukleinsäure, die das Maus-TREM2 kodiert, die so modifiziert ist, dass sie eine Substitution R47H einschließt, und die Nukleotidsequenz umfasst, die mindestens 95 % Identität mit der Nukleotidsequenz von SEQ-ID-NR:4 aufweist, und/oder das zur Aufnahme einer Substitution R47H modifizierte Maus-TREM2 eine Aminosäuresequenz einschließt, die mindestens 95 % Identität mit der Aminosäuresequenz von SEQ-ID-NR:3 aufweist, wobei optional die genomische Nukleinsäure, die das Maus-TREM2 kodiert, das so modifiziert ist, dass es eine Substitution R47H einschließt, die Nukleotidsequenz von SEQ-ID-NR:4 einschließt, und/oder das Maus-TREM2, das so modifiziert ist, dass es eine Substitution R47H einschließt, die Aminosäuresequenz von SEQ-ID-NR:3 einschließt.

5. Genetisch modifizierte Maus nach einem der Ansprüche 1-4, wobei die genetisch modifizierte Maus das Maus-TREM2 ausdrückt, das eine Substitution R47H einschließt.

6. Genetisch modifizierte Maus nach einem der Ansprüche 1-5, wobei die genomische Nukleinsäure, die das Maus-APP kodiert, die so modifiziert ist, dass sie die Substitutionen G601R, F606Y und R609H einschließt, eine Nukleotidsequenz umfasst, die mindestens 95 % Identität mit der Nukleotidsequenz von SEQ-ID-NR:23 aufweist, und/oder das Maus-APP, das so modifiziert ist, dass sie die Substitutionen G601R, F606Y und R609H eine Aminosäuresequenz umfasst, die mindestens 95 % Identität mit der Aminosäuresequenz von SEQ-ID-NR:24 aufweist.

7. Genetisch modifizierte Maus nach einem der Ansprüche 1-6, wobei die genomische Nukleinsäure, die das Maus-APP kodiert, die so modifiziert ist, dass sie die Substitutionen G601R, F606Y und R609H die Sequenz von SEQ-ID-NR:23 umfasst, und/oder das Maus-APP, das so modifiziert ist, dass es die Substitutionen G601R, F606Y und R609H die Aminosäuresequenz von SEQ-ID-NR:24 einschließt.

8. Genetisch modifizierte Maus nach einem der Ansprüche 1-7, wobei die genetisch modifizierte Maus das Maus-APP ausdrückt, das die Substitutionen G601R, F606Y und R609H einschließt.

9. Genetisch modifizierte Maus nach einem der Ansprüche 1-8, wobei der Gesamtcholesterinspiegel, der Low-Density-Lipoprotein (LDL)-Spiegel und/oder der High-Density-Lipoprotein (HDL)-Spiegel in der genetisch modifizierten Maus im Vergleich zu einer Kontrollmaus reduziert ist,
wobei die Kontrollmaus eine Wildtyp-C57BL/6J-Maus ist, wobei optional der Gesamtcholesterinspiegel in der genetisch modifizierten Maus im Vergleich zur Kontrollmaus um mindestens 10 % oder mindestens 20 % reduziert ist; und/oder
wobei der LDL-Spiegel in der genetisch modifizierten Maus im Vergleich zur Kontrollmaus um mindestens 30 %, mindestens 40 % oder mindestens 50 % reduziert ist; und/oder
wobei der HDL-Spiegel in der genetisch modifizierten Maus im Vergleich zur Kontrollmaus um mindestens 10 % reduziert ist.

10. Genetisch modifizierte Maus nach einem der Ansprüche 1-9, wobei die genetisch modifizierte Maus Anzeichen einer zerebrovaskulären Leckage aufweist.

11. Genetisch modifizierte Maus nach einem der Ansprüche 1-10, wobei mindestens eines der folgenden Gene in der genetisch modifizierten Maus unterschiedlich ausgedrückt wird: Pcsk2, Mapkl0, Mapk9, Prkcq, Slcl8a2, Plcb2, Slc6a4, Gng2, Akt3, Gnaol, Plcb3, Arrb2,I16, Myhl0, Cnrl, Stxla, Uncl3a, Cdk5, Calbl, Gria4, Thyl, Hcnl, Chll, Uchll, Amph, Chmp2b, and Casp7.

12. Verfahren zur Herstellung eines genetisch modifizierten Mausmodells für spät auftretende Alzheimer-Krankheit, das Verfahren umfassend:
(a) (i) Bereitstellen einer genetisch modifizierten Maus, umfassend eine genomische Nukleinsäure, die menschliches APOE4 kodiert, und eine genomische Nukleinsäure, die das Maus-TREM2 kodiert, das so modifiziert ist, dass es eine R47H-Substitution einschließt; oder (ii) Einführen einer genomischen Nukleinsäure, die menschliches APOE4 kodiert, und einer genomischen Nukleinsäure, die das Maus-TREM2 kodiert, das so modifiziert ist, dass es eine Substitution R47H einschließt, in eine Maus; und
(b) Einführen einer genomischen Nukleinsäure, die das Maus-APP kodiert, das so modifiziert ist, dass es die Substitutionen G601R, F606Y und R609H einschließt, in die genetisch modifizierte Maus.

13. Verfahren zum Screening einer Verbindung, um sie bei der Behandlung von spät auftretender Alzheimer-Krankheit zu verwenden, umfassend:
(i) Verabreichen einer Verbindung an eine genetisch modifizierte Maus nach einem der Ansprüche 1-10; und
(ii) Untersuchen der Wirkung der Verbindung auf ein mit der Alzheimer-Krankheit verbundenes Symptom bei der Maus.

## Revendications

1. Modèle de souris génétiquement modifiée de la maladie d'Alzheimer à apparition tardive comprenant
(a) un acide nucléique génomique codant pour l'APOE4 humaine,
(b) un acide nucléique génomique codant pour la TREM2 de souris modifiée pour inclure une substitution R47H, et
(c) un acide nucléique génomique codant pour l'APP de souris modifiée pour inclure les substitutions G601R, F606Y, et R609H.

2. Souris génétiquement modifiée selon la revendication 1, dans laquelle l'acide nucléique génomique codant pour l'APOE4 humaine comprend une séquence nucléotidique ayant au moins 95 % d'identité avec la séquence nucléotidique de SEQ ID NO:2, et/ou l'APOE4 humaine comprend une séquence d'acides aminés ayant au moins 95 % d'identité avec la séquence d'acides aminés de SEQ ID NO:1,
éventuellement dans laquelle l'acide nucléique génomique codant pour l'APOE4 humaine comprend la séquence nucléotidique de SEQ ID NO:2, et/ou l'APOE4 humaine comprend la séquence d'acides aminés de SEQ ID NO:1.

3. Souris génétiquement modifiée selon les revendications 1 ou 2, dans laquelle la souris génétiquement modifiée exprime l'APOE4 humaine et/ou n'exprime pas un niveau détectable d'APOE4 de souris.

4. Souris génétiquement modifiée selon l'une quelconque des revendications 1 à 3, dans laquelle l'acide nucléique génomique codant pour la TREM2 de souris modifiée pour inclure une substitution R47H comprend une séquence nucléotidique ayant au moins 95 % d'identité avec la séquence nucléotidique de SEQ ID NO:4, et/ou la TREM2 de souris modifiée pour inclure une substitution R47H comprend une séquence d'acides aminés ayant au moins 95 % d'identité avec la séquence d'acides aminés de SEQ ID NO:3, éventuellement dans laquelle l'acide nucléique génomique codant pour la TREM2 de souris modifiée pour inclure une substitution R47H comprend la séquence nucléotidique de SEQ ID NO:4, et/ou la TREM2 de souris modifiée pour inclure une substitution R47H comprend la séquence d'acides aminés de SEQ ID NO:3.

5. Souris génétiquement modifiée selon l'une quelconque des revendications 1 à 4, dans laquelle la souris génétiquement modifiée exprime la TREM2 de souris modifiée pour inclure une substitution R47H.

6. Souris génétiquement modifiée selon l'une quelconque des revendications 1 à 5, dans laquelle l'acide nucléique génomique codant pour l'APP de souris modifiée pour inclure les substitutions G601R, F606Y, et R609H comprend une séquence nucléotidique qui partage au moins 95 % d'identité avec la séquence nucléotidique de SEQ ID NO:23, et/ou l'APP de souris modifiée pour inclure les substitutions G601R, F606Y, et R609H comprend une séquence d'acides aminés qui partage au moins 95 % d'identité avec la séquence d'acides aminés de SEQ ID NO:24.

7. Souris génétiquement modifiée selon l'une quelconque des revendications 1 à 6, dans laquelle l'acide nucléique génomique codant pour l'APP de souris modifiée pour inclure les substitutions G601R, F606Y, et R609H comprend la séquence nucléotidique de SEQ ID NO:23, et/ou l'APP de souris modifiée pour inclure les substitutions G601R, F606Y, et R609H comprend la séquence d'acides aminés de SEQ ID NO:24.

8. Souris génétiquement modifiée selon l'une quelconque des revendications 1 à 7, dans laquelle la souris génétiquement modifiée exprime l'APP de souris modifiée pour inclure les substitutions G601R, F606Y, et R609H.

9. Souris génétiquement modifiée selon l'une quelconque des revendications 1 à 8, dans laquelle le taux de cholestérol total, le taux de lipoprotéines de basse densité (LDL) et/ou le taux de lipoprotéines de haute densité (HDL) sont réduits chez la souris génétiquement modifiée, par rapport à une souris témoin, dans laquelle la souris témoin est une souris C57BL/6J de type sauvage,
éventuellement dans laquelle le taux de cholestérol total chez la souris génétiquement modifiée est réduit d'au moins 10 % ou d'au moins 20 % par rapport à la souris témoin ; et/ou
dans laquelle le taux de LDL chez la souris génétiquement modifiée est réduit d'au moins 30 %, d'au moins 40 % ou d'au moins 50 % par rapport à la souris témoin ; et/ou dans laquelle le taux de HDL chez la souris génétiquement modifiée est réduit d'au moins 10 % par rapport à la souris témoin.

10. Souris génétiquement modifiée selon l'une quelconque des revendications 1 à 9, dans laquelle la souris génétiquement modifiée présente des signes de fuite cérébrovasculaire.

11. Souris génétiquement modifiée selon l'une quelconque des revendications 1 à 10, dans laquelle au moins l'un des gènes suivants est exprimé de manière différentielle chez la souris génétiquement modifiée : Pcsk2, Mapk10, Mapk9, Prkcq, Slc18a2, Plcb2, Slc6a4, Gng2, Akt3, Gnao1, Plcb3, Arrb2, Il6, Myh10, Cnr1, Stx1a, Unc13a, Cdk5, Calb1, Gria4, Thy1, Hcn1, Chl1, Uch1l, Amph, Chmp2b, et Casp7.

12. Procédé de production d'un modèle de souris génétiquement modifiée de la maladie d'Alzheimer à apparition tardive, le procédé comprenant :
(a) (i) la fourniture d'une souris génétiquement modifiée comprenant un acide nucléique génomique codant pour l'APOE4 humaine et un acide nucléique génomique codant pour la TREM2 de souris modifiée pour inclure une substitution R47H ; ou (ii) l'introduction dans une souris d'un acide nucléique génomique codant pour l'APOE4 humaine et d'un acide nucléique génomique codant pour la TREM2 de souris modifiée pour inclure une substitution R47H ; et
(b) l'introduction dans la souris génétiquement modifiée d'un acide nucléique génomique codant pour l'APP de souris modifiée pour inclure les substitutions G601R, F606Y, et R609H.

13. Procédé de criblage d'un composé destiné à être utilisé dans le traitement de la maladie d'Alzheimer à apparition tardive, comprenant :
(i) l'administration d'un composé à une souris génétiquement modifiée selon l'une quelconque des revendications 1 à 10 ; et
(ii) l'évaluation chez la souris de l'effet du composé sur un symptôme associé à la maladie d'Alzheimer.
